# EUROPEAN PATENT APPLICATION

(11) **EP 4 488 268 A1**
(43) Date of publication of application: **08.01.2025**
(21) Application number: 23762972.0
(22) Date of filing: 02.03.2023
(51) Int. Cl.: C07D 401/12, C07D 401/14, C07D 237/20, C07D 471/04, A61K 31/444, A61K 31/501, A61K 31/50, A61K 31/407, A61P 29/00, A61P 37/00

(54) **COMPOUND USED AS TYK2 INHIBITOR, PREPARATION METHOD THEREFOR, AND PHARMACEUTICAL USE THEREOF**

(30) Priority: 04.03.2022 CN 202210210857; 06.01.2023 CN 202310020684
(71) Applicant: Shanghai Zhigen Pharmaceutical & Technology Co. Ltd., Shanghai 201318 (CN)
(72) Inventor: ZHANG, Nan, Shanghai 201318 (CN); CHEN, Yilang, Shanghai 201318 (CN)
(74) Representative: Mewburn Ellis LLP
(86) International application number: PCT/CN2023/079387
(87) International publication number: WO 2023/165574

(57) **Abstract**

Disclosed are a compound used as a TyK2 inhibitor, a preparation method therefor, and pharmaceutical use thereof. Specifically, the compound has a structure represented by formula (I), wherein the definitions of the groups and substituents are as described in the specification. In addition, also disclosed are a preparation method for the compound and use thereof in preventing and/or treating a related disease mediated by TYK2.

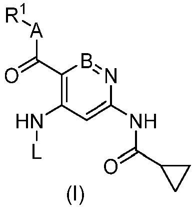

## Description

### THCHNICAL FIFLD

The present invention relates to the field of small molecule pharmaceuticals and specifically to compounds used as TYK2 inhibitors, preparation method thereof, and their application in medicine.

### BACKGROUND ART

Autoimmune diseases are an inflammatory disease in which the autoimmune system of body attacks normal cells causing a decrease in normal immunity, an accentuation of abnormal immunity, and ultimately tissue damage or organ dysfunction. Currently, more than 80 autoimmune diseases have been reported, affecting approximately 5-8% of population in the world. Among the more prevalent ones are psoriasis (PSO), rheumatoid arthritis (RA), psoriatic arthritis (PsA), ulcerative colitis (UC), Crohn's disease (CD), multiple sclerosis (MS), type 1 diabetes mellitus, spondyloarthritis (SpA), chronic graft-versus-host disease (cGVHD), atopic dermatitis, alopecia areata, asthma, systemic lupus erythematosus (SLE). The sites of these diseases cover almost all body organs. They can be systemic, such as SLE, which can affect the skin, joints, kidneys, and central nervous system; or organ-specific, such as type 1 diabetes (pancreas), UC (colon) [Cell 2020 181(1):63-80]. The pathogenesis of autoimmune diseases is complex, the disease disability rate is high, there is no radical cure, and most patients suffer great pain for life. Therefore, autoimmune diseases have been called the "immortal cancer".

Since 2000, the main small molecule drugs clinically approved for the treatment of autoimmune diseases have been Janus kinase (JAK) inhibitors. This family includes JAK1, JAK2, JAK3, and TYK2. Members of the JAK family regulate immunomodulatory cytokine-initiated signaling by phosphorylating their receptors, which in turn leads to recruitment of signal transducers, phosphorylation, and activation of transcriptional (STAT) proteins, thereby affecting STAT-dependent transcriptional and functional responses. These signaling pathways of cytokine play a key role in the pathogenesis of autoimmune and inflammatory diseases. To date, the clinically approved JAK inhibitors Tofacitinib, Baricitinib, and Upadacitinib act on the kinase domains of JAK family (JH1 domain) and act by competing binding with ATP in vivo. The above mentioned JAK inhibitors exhibit a series of serious clinical side effects such as anemia, infections, increased levels of creatinine, hepatic transaminases, creatine phosphokinase, LDL cholesterol, HDL cholesterol, and decreased NK cell, lymphocyte, neutrophil, and platelet counts due to the poor selectivity of the kinase isoforms. As a result, the three marketed drugs mentioned above carry black box warnings about the risk of causing serious infections, malignancies, and blood clots, and their clinical use is very limited.

Recent studies have shown that TYK2, which belongs to the JAK family, is an optimal target for balancing pharmacological efficacy and safety in autoimmune diseases [J. Med. Chem. 2019, 62, 20, 8953-8972]. TYK2, in association with JAK2, induces/stabilizes the expression of IL-17 and other pro-inflammatory factors by mediating signaling through IL-12 and IL-23 (cytokine-containing p40 subunit); and in association with JAK1, mediates signaling of type 1 interferons (IFN-α, etc.) TYK2-mediated cytokines mentioned above have been identified as the most important inflammatory factors of the autoimmune system [Nature Reviews Drug Discovery 2021, 20, 39-63]. Blockade of the above inflammatory cytokine signaling pathways has demonstrated excellent pharmacodynamic activity in animal models of rheumatoid arthritis, multiple sclerosis, psoriasis, systemic lupus erythematosus, inflammatory bowel disease and spondyloarthritis [J. Med. Chem. 2019, 62, 20, 8973-8995; Science Translational Medicine 2019, 502(11), : eaaw1736; J Clin Invest. 2020, 130(4), 1863-1878]. And monoclonal antibody drugs developed against the above inflammatory factors IL-12, IL-23, IL-17 and IFN-α have shown excellent results in the clinical treatment of several autoimmune system diseases.

The above evidence suggests that TYK2, as a key kinase mediating the IL-12, IL-23, IL-17 and IFN-α inflammatory signaling cascade, is a good drug-forming target for inflammatory and autoimmune diseases. Clearly, highly subtype-selective (JAK1, JAK2, JAK3) TYK2 inhibitors have the potential to avoid the serious side effects of pre-existing JAK inhibition. BMS-986165 (Deucravacitinib), a highly subtype-selective TYK2 inhibitor developed by Bristol-Myers Squibb, has shown excellent efficacy and safety in two Phase III clinical studies of moderate to severe psoriasis. Meanwhile, BMS-986165 is in Phase II clinical evaluation in the indications of psoriatic arthritis, systemic lupus erythematosus, ulcerative colitis, and Crohn's disease, demonstrating strong therapeutic potential for autoimmune diseases. Representative patents for the development of highly kinase (isoform) selective TYK2 inhibitors are WO2014074660, WO2014074661, and WO2019103952.

The present invention provides a class of TYK2 inhibitors with enhanced kinase inhibitory activity, high subtype selectivity, excellent metabolic properties, enhanced in vivo potency, and novel structure. The TYK2 inhibitors disclosed herein can be used for the prevention and/or treatment of TYK2-mediated related diseases, in particular autoimmune diseases associated with IL-12, IL-23, IL-17, and type 1 interferon (IFN-α, etc.).

### SUMMARY OF THE INVENTION

The purpose of the present invention is to provide a compound shown in formula (I) and its preparation method and its use in the prevention and/or treatment of diseases mediation by TYK2.

In the first aspect of the present invention, it provides a compound shown in formula (I), or a tautomer, a mesomer, a racemate, an enantiomer, a diastereoisomer, or a mixture thereof, or a pharmaceutically acceptable salt thereof, 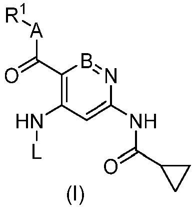 wherein,
R¹ is selected from the group consisting of H, substituted or unsubstituted C1-C6 alkyl, substituted or unsubstituted C3-C6 cycloalkyl, the substitution refers to be substituted by one or more substituents selected from the group consisting of deuterium, halogen;
A is NH or CH₂;
B is CH or N;
L is selected from the group consisting of 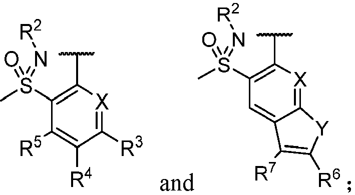
R² is selected from the group consisting of H, -CD₃, C1-C6 alkyl, C3-C6 cycloalkyl;
X is CH or N;
Y is NH, O or S;
R³, R⁴, R⁵ are each independently selected from the group consisting of H, -NH₂, -OH, C1-C6 alkoxy, C1-C6 alkyl, halogenated C1-C6 alkyl, C3-C6 cycloalkyl, - CONH₂, -CN, halogen, -O(CH₂)ₙR⁹, C2-C6 alkenyl, C2-C6 alkynyl, C3-C6 cycloalkyl-substituted C2-C6 alkynyl and -NR⁸(CH₂)ₙR⁹;
n is 1, 2, 3, 4 or 5;
R⁹ is selected from the group consisting of -OH, C1-C6 alkoxy, C4-C6 cycloalkoxy, 4-8-membered heterocyclic group containing 1, 2 or 3 heteroatoms selected from N, O or S and -CN;
R⁸ is H or C1-C6 alkyl;
R⁶, R⁷ are each independently selected from the group consisting of H, C1-C6 alkyl, halogen;.

In another preferred embodiment, R¹ is selected from the group consisting of H, substituted or unsubstituted C1-C6 alkyl, substituted or unsubstituted C3-C6 cycloalkyl, the substitution refers to be substituted by one or more substituents selected from the group consisting of deuterium, halogen;
A is NH or CH₂;
B is CH or N;
L is selected from the group consisting of 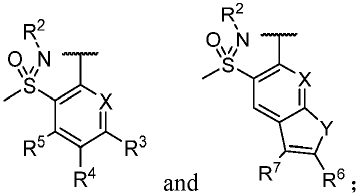
R² is selected from the group consisting of H, -CD₃, C1-C6 alkyl, C3-C6 cycloalkyl;
X is CH or N;
Y is NH, O or S;
R³, R⁴, R⁵ are each independently selected from the group consisting of H, -NH₂, -OH, C1-C6 alkoxy, C1-C6 alkyl, halogenated C1-C6 alkyl, C3-C6 cycloalkyl, - CONH₂, -CN, halogen, -O(CH₂)ₙR⁹ and -NR'(CH₂)ₙR⁹;
n is 1, 2, 3, 4 or 5;
R⁹ is selected from the group consisting of -OH, C1-C6 alkoxy, C4-C6 cycloalkoxy, 4-8-membered heterocyclic group containing 1, 2 or 3 heteroatoms selected from N, O or S and -CN;
R⁸ is H or C1-C6 alkyl;
R⁶, R⁷ are each independently selected from the group consisting of H, C1-C6 alkyl, halogen.

In another preferred embodiment, the additional condition is that when R² is H, R³ is -NH₂ or not both R⁴ and R⁵ are H.

In another preferred embodiment, R¹ is deuterated or unsubstituted C1-C6 alkyl.

In another preferred embodiment, R¹ is a fully deuterated C1-C6 alkyl.

In another preferred embodiment, the fully deuterated refers to all H of the group is substituted with D.

In another preferred embodiment, R¹ is C1-C6 alkyl.

In another preferred embodiment, R¹ is fully deuterated C1-C6 alkyl;
A is NH;
B is CH or N.

In another preferred embodiment, R¹ is selected from the group consisting of -CH₃ and -CD₃;
A is NH;
B is CH or N.

In another preferred embodiment, L is 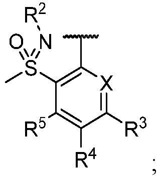
R² is selected from the group consisting of H, -CD₃, C1-C6 alkyl;
X is N;
R³, R⁴, R⁵ are each independently selected from the group consisting of H, -NH₂, -OH, C1-C6 alkoxy, C1-C6 alkyl, C3-C6 cycloalkyl, -CONH₂, -CN, halogen, - O(CH₂)ₙR⁹, C2-C6 alkenyl, C2-C6 alkynyl, C3-C6 cycloalkyl-substituted C2-C6 alkynyl and -NR⁸(CH₂)ₙR⁹;
n is 1, 2, 3, 4 or 5;
R⁹ is selected from the group consisting of C1-C6 alkoxy, C4-C6 cycloalkoxy, 4-8-membered heterocyclic group containing 1, 2 or 3 heteroatoms selected from N, O or S and -CN;
R⁸ is H or C1-C6 alkyl.

In another preferred embodiment, L is 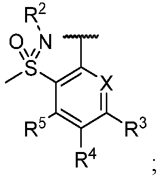
R² is selected from the group consisting of H, -CD₃, C1-C6 alkyl;
X is N;
R³, R⁴, R⁵ are each independently selected from the group consisting of H, -NH₂, -OH, C1-C6 alkoxy, C1-C6 alkyl, C3-C6 cycloalkyl, -CONH₂, -CN, halogen, - O(CH₂)ₙR⁹ and -NR⁸(CH₂)ₙR⁹;
n is 1, 2, 3, 4 or 5;
R⁹ is selected from the group consisting of C1-C6 alkoxy, C4-C6 cycloalkoxy, 4-8-membered heterocyclic group containing 1, 2 or 3 heteroatoms selected from N, O or S and -CN;
R⁸ is H or C1-C6 alkyl.

In another preferred embodiment, R² is H, -CD₃ and -CH₃.

In another preferred embodiment, R⁹ is selected from the group consisting of - OCH3, -CN,

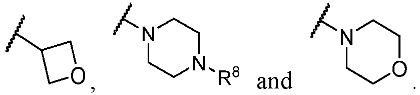

In another preferred embodiment, R³, R⁴ are each independently selected from the group consisting of -NH₂, -OH, C1-C6 alkoxy, C1-C6 alkyl, C3-C6 cycloalkyl, - CONH₂, -CN, halogen, -O(CH₂)ₙR⁹, C2-C6 alkenyl, C2-C6 alkynyl, C3-C6 cycloalkyl-substituted C2-C6 alkynyl, and -NR⁸(CH₂)ₙR⁹.

In another preferred embodiment, R³, R⁴ are each independently selected from the group consisting of -NH₂, C1-C6 alkyl, halogen, C3-C6 cycloalkyl, C2-C6 alkenyl, C2-C6 alkynyl, C3-C6 cycloalkyl-substituted C2-C6 alkynyl.

In another preferred embodiment, R³ is -NH₂.

In another preferred embodiment, R⁴ is C1-C6 alkyl, halogen, C3-C6 cycloalkyl, C2-C6 alkenyl, C2-C6 alkynyl, C3-C6 cycloalkyl-substituted C2-C6 alkynyl.

In another preferred embodiment, R³ is -NH₂; R⁴ is halogen.

In another preferred embodiment, R³ is -NH₂; R⁴ is Cl or Br.

In another preferred embodiment, R¹ is a fully deuterated C1-C6 alkyl;
A is NH;
B is N;
L is 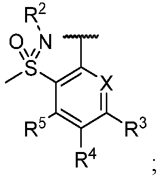
R² is selected from the group consisting of H, -CD₃, C1-C6 alkyl;
X is N;
R³ is -NH₂;
R⁴ is selected from the group consisting of halogen, C3-C6 cycloalkyl, C2-C6 alkenyl, C2-C6 alkynyl, C3-C6 cycloalkyl-substituted C2-C6 alkynyl;
R⁵ is H.

In another preferred embodiment, R¹ is a fully deuterated C1-C6 alkyl;
A is NH;
B is N;
L is 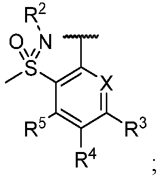
R² is selected from the group consisting of H, C1-C6 alkyl;
X is N;
R³ is -NH₂;
R⁴ is halogen;
R⁵ is H.

In another preferred embodiment, R¹ is a fully deuterated C1-C6 alkyl;
A is NH;
B is N;
L is 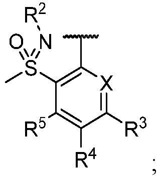
R² is -CD₃;
X is N;
R³ is -NH₂;
R⁴ is selected from the group consisting of halogen, C3-C6 cycloalkyl, C2-C6 alkenyl, C2-C6 alkynyl, C3-C6 cycloalkyl-substituted C2-C6 alkynyl;
R⁵ is H.

In another preferred embodiment, the compound is selected from the group consisting of:

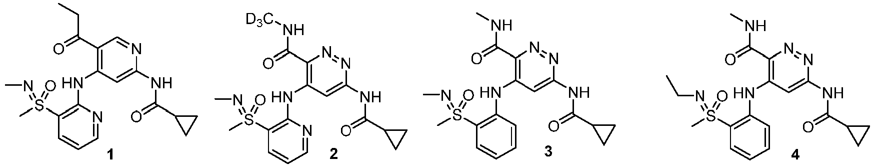

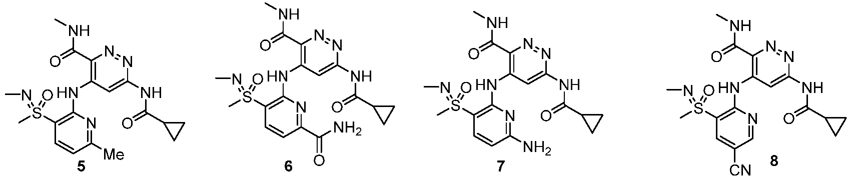

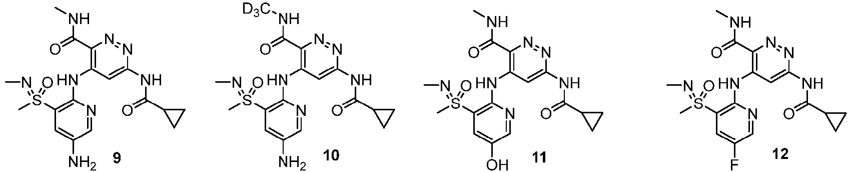

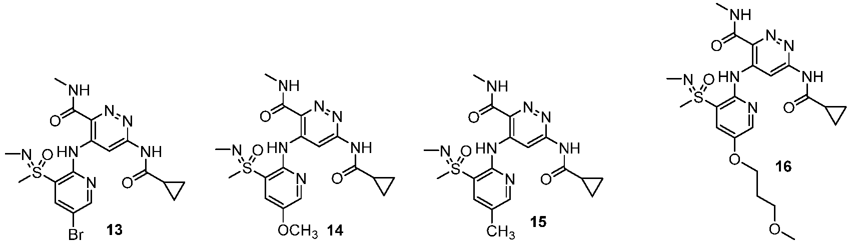

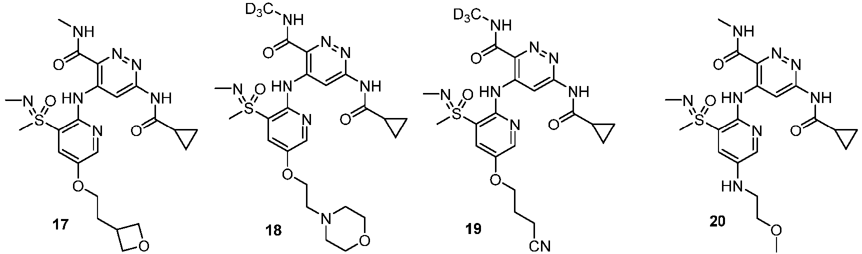

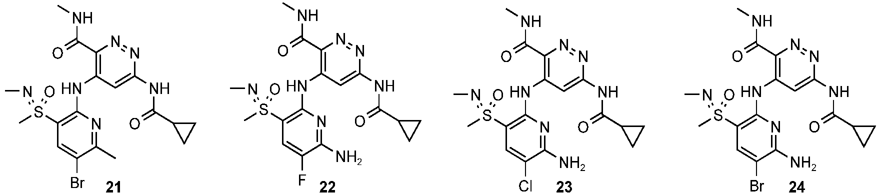

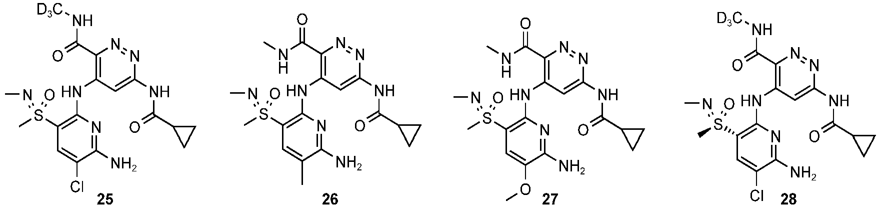

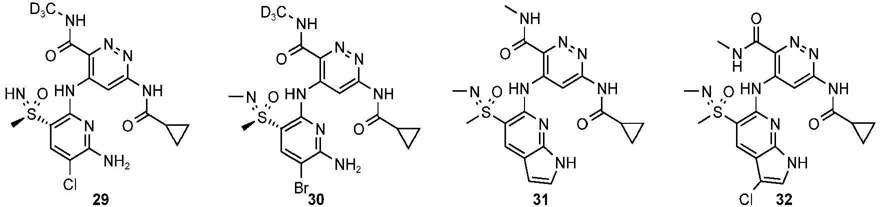

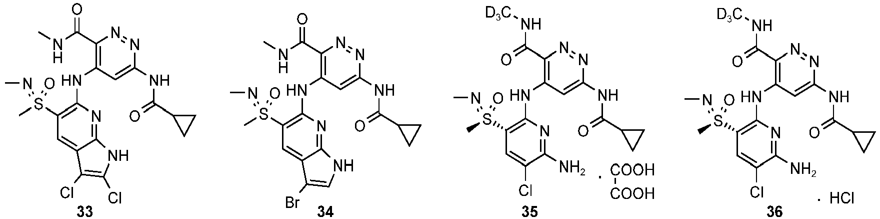

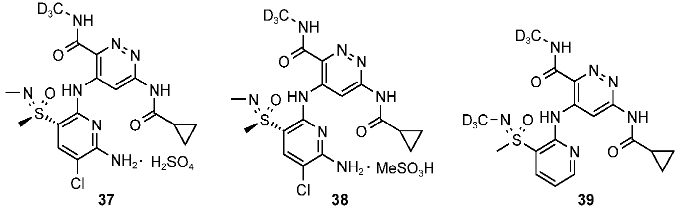

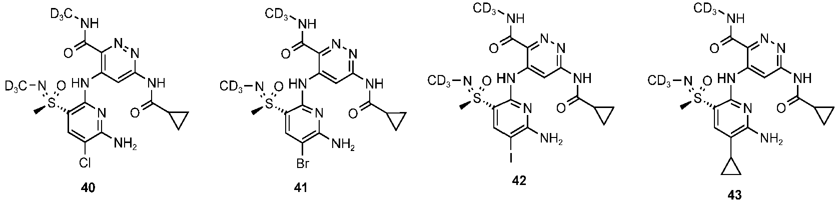

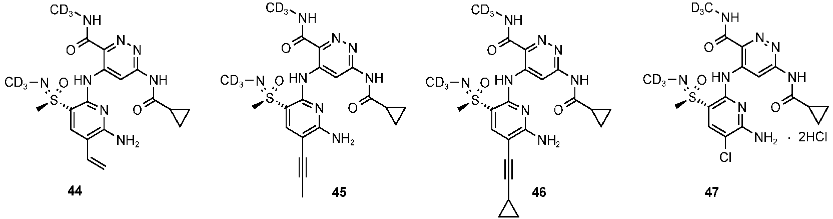

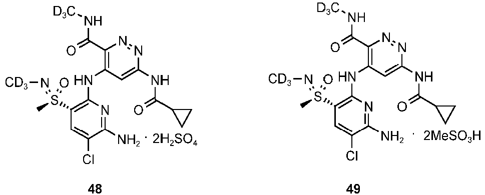

In the second aspect of the present invention, it provides a preparation method of the compound or the tautomer, the mesomer, the racemate, the enantiomer, the diastereoisomer, or the mixture thereof, or the pharmaceutically acceptable salt thereof of the first aspect of the present invention, comprising the following steps: 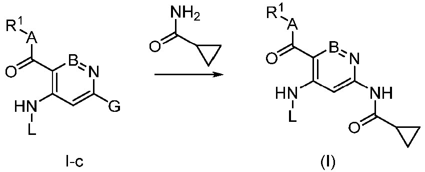
reacting formula I-c with cyclopropyl amide to obtain the compound of formula (I);
wherein,
R¹, A, B, L are as defined in the first aspect of the present invention;
G is halogen.

In the third aspect of the present invention, it provides a pharmaceutical composition comprising a pharmaceutically acceptable carrier and one or more safe and effective amounts of the compound or the tautomer, the mesomer, the racemate, the enantiomer, the diastereoisomer, or the mixture thereof, or the pharmaceutically acceptable salt thereof of the first aspect of the present invention.

In another preferred embodiment, the pharmaceutical composition is an oral formulation.

In the fourth aspect of the present invention, it provides a use of the compound or the tautomer, the mesomer, the racemate, the enantiomer, the diastereoisomer, or the mixture thereof, or the pharmaceutically acceptable salt thereof of the first aspect of the present invention for the preparation of a medicament, the medicament is used for the prevention and/or treatment of a disease mediated by TYK2.

In another preferred embodiment, the disease mediated by TYK2 is an inflammatory or autoimmune disease in mammals.

In another preferred embodiment, the inflammatory or autoimmune disease is selected from the group consisting of rheumatoid arthritis, multiple sclerosis, systemic lupus erythematosus, lupus nephritis, cutaneous lupus, inflammatory bowel disease, psoriasis, Crohn's disease, psoriatic arthritis, Sjogren's Syndrome(ss), systemic scleroderma, ulcerative colitis, Gray's disease, discoid lupus erythematosus, adult Still's disease, Systemic juvenile idiopathic arthritis, gout, gouty arthritis, type 1 diabetes mellitus, insulin-dependent diabetes mellitus, sepsis, septic shock, shigellosis, pancreatitis, glomerulonephritis, autoimmune gastritis, autoimmune hemolytic anemia, autoimmune neutropenia, thrombocytopenia, atopic dermatitis, myasthenia gravis, pancreatitis, ankylosing spondylitis, pemphigus vulgaris, antiphospholipid syndrome, idiopathic thrombocytopenia, ANCA-associated vasculitis, Kawasaki disease, chronic inflammatory demyelinating polyneuropathy, dermatomyositis, polymyositis, uveitis, Gerbaud's syndrome, autoimmune pneumonitis, autoimmune thyroiditis, autoimmune inflammatory ophthalmopathy, and chronic demyelinating polyneuropathy.

It should be understood that in the present invention, any of the technical features specifically described above and below (such as in the Examples) can be combined with each other, so as to constitute new or preferred technical solutions. Due to space limitations, it will not redundantly be described one by one herein.

### DESCRIPTION OF FIGURES

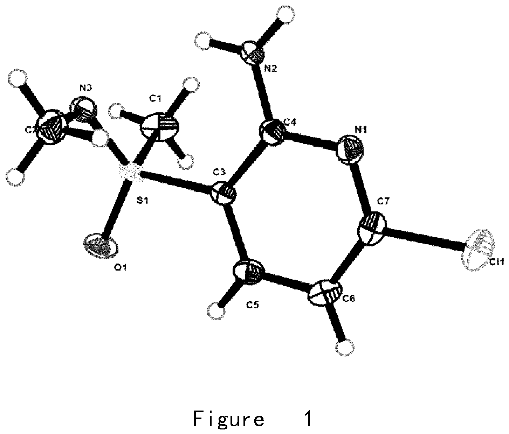

Figure 1 shows single crystal diffraction data for compound 28e of the present invention.

### DETAILED DESCRIPTION OF THE INVENTION

After long-term and in-depth research, through structural optimization, the present inventors have obtained a compound shown in formula (I) with high inhibitory activity and high selectivity of TYK2, excellent metabolic properties and drug-forming properties, and a preparation method thereof and its use in the treatment and/or prevention of TYK2-mediated related diseases. On above basis, the inventors have completed the present invention.

### TERMS

In the present invention, unless otherwise indicated, the terms used have the ordinary meaning known to the skilled person in the art.

In the present invention, the term "halogen" refers to F, Cl, Br or I.

In the present invention, the term "C1-C6 alkyl" refers to straight-chain or branched alkyl groups comprising 1-6 carbon atoms, for example methyl, ethyl, propyl, iso-propyl, butyl, iso-butyl, *tert*-butyl, neo-pentyl, ter-pentyl, or the like.

In the present invention, the term "C2-C6 alkenyl" refers to a straight or branched alkenyl group with 2-6 carbon atoms containing a double bond, and non-limitingly includes vinyl, propenyl, butenyl, iso-butenyl, pentenyl, hexenyl and the like.

In the present invention, the term "C2-C6 alkynyl" refers to a straight or branched alkynyl group with 2-6 carbon atoms containing a triple bond, and non-limitingly includes ethynyl, propynyl, butynyl, iso-butynyl, pentynyl and hexynyl, etc.

In the present invention, the term "C3-C8 cycloalkyl" refers to a cyclic alkyl group with 3-8 carbon atoms on the ring, and non-limitingly includes cyclopropyl, cyclobutyl, cyclopentyl, cyclohexyl, cycloheptyl, cyclooctyl, and the like. The term "C3-C6 cycloalkyl" has a similar meaning.

In the present invention, the term "C1-C6 alkoxy" refers to straight-chain or branched alkoxy groups with 1-6 carbon atoms, and non-limitingly includes methoxy, ethoxy, propoxy, iso-propoxy and butoxy. Preferably C1-C4 alkoxy.

In the present invention, the term "heterocyclic group" refers to a heterocyclic group with 4-8 ring atoms containing 1, 2 or 3 heteroatoms selected from N, O, S, including but not limited to the following groups:

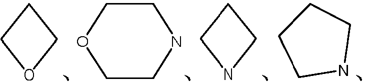

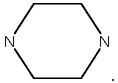

**In** the present invention, the terms "aryl ring" or "aryl" have the same meaning, preferably "C6-C10 aryl". The term "C6-C10 aryl" refers to an aromatic ring with 6-10 carbon atoms without heteroatoms on the ring, such as phenyl, naphthyl, etc.

**In** the present invention, the term "aromatic heterocycle" or "heteroaryl" has the same meaning and refers to a heteroaromatic group containing one to more heteroatoms. For example, "C3-C10 heteroaryl" refers to an aromatic heterocyclic ring with 3 to 10 carbon atoms and 1 to 4 heteroatoms selected from oxygen, sulfur, and nitrogen. Nonlimiting examples are furanyl, thienyl, pyridinyl, pyrazolyl, pyrrolyl, N-alkylpyrrolyl, pyrimidinyl, pyrazinyl, imidazolyl, tetrazolyl, etc. The heteroaryl ring may be fused on an aryl, heterocyclyl or cycloalkyl ring, wherein the ring attached to the parent structure is a heteroaryl ring. The heteroaryl group may be optionally substituted or unsubstituted.

**In** the present invention, the term "halogenated" means substituted with a halogen.

**In** the present invention, the term "deuterated" means substituted with deuterium.

**In** the present invention, the term "substitution" refers to one or more hydrogen atoms on a particular group are substituted by a particular substituent. The particular substituent is a substituent described above accordingly, or a substituent in the embodiments. Unless indicated specifically, a substituted group may have a substituent group selected from a particular group at any substitutable site of the group, and the substituent group can be identical or different at each site. It should be understood by the person skilled in the art that the combinations of substituents expected in the present invention are those that are stable or chemically realizable. For example (but not limited to), substituents are halogen, hydroxyl, carboxy (-COOH), C1-C6 alkyl, C2-C6 alkenyl, C2-C6 alkynyl, C3-C8 cycloalkyl, 3-12 membered heterocyclic group, aryl, heteroaryl, C1-C8 aldehyde, C2-C10 acyl, C2-C10 ester, amino, C1-C6 alkoxy, C1-C10 sulfonyl, etc.

In the present invention, the terms 1-6 refer to 1, 2, 3, 4, 5 or 6. Other similar terms each independently have a similar meaning. The term "more" refers to 2-6, such as 2, 3, 4, 5 or 6.

It should be understood that when a certain group is present at a number of different sites in a compound, its definition at each site is independent and may be the same or different. That is, the term "selected from the group consisting of:" has the same meaning as the term "each independently selected from the group consisting of:".

### Compounds

For the first aspect of the present invention, the compounds of formula (I) may contain one or more chiral centers, which exist as enantiomers and diastereomers. The compounds of formula (I) of the present invention also contain many geometrical isomers such as alkenes, C=N double bonds, etc. Unless indicated otherwise, all chiral (enantiomer and diastereoisomer), racemic, cis geometric isomers, trans geometric isomers, and mixtures of cis- and trans- geometric isomers described above are included in the present invention. For enantiomers, two enantiomers can be obtained using general chiral splitting methods or asymmetric synthesis methods. For diastereoisomers, they can be separated by methods such as stepwise recrystallization or chromatographic separation.

In another preferred embodiment, any one of R¹, R², R³, R⁴, R⁵*,* R⁶, R⁷, R⁸, R⁹, A, B, L, X, Y, n is independently a corresponding group in the specific compound described in the present invention.

As used herein, the term "pharmaceutically acceptable salt" refers to a salt of a compound of the invention formed with an acid or base suitable for use as a drug. Pharmaceutically acceptable salts include inorganic and organic salts. A preferably class of salts are salts formed by the compounds of the present invention with acids. Acids suitable for forming salts include, but are not limited to: inorganic acids such as hydrochloric acid, hydrobromic acid, hydrofluoric acid, sulfuric acid, nitric acid, phosphoric acid, and the like; organic acids such as formic acid, acetic acid, trifluoroacetic acid, propionic acid, oxalic acid, malonic acid, succinic acid, fumaric acid, maleic acid, lactic acid, malic acid, tartaric acid, citric acid, picric acid, benzoic acid, methanesulfonic acid, ethylsulfonic acid, p-toluenesulfonic acid, benzene sulfonic acid, naphthalenesulfonic acid, and the likes; and amino acids such as proline, phenylalanine, aspartic acid and glutamic acid, etc.

Another preferably class of salts are formed by the compounds of the present invention with bases, such as alkali metal salts (e.g., sodium or potassium salts), alkaline earth metal salts (e.g., magnesium or calcium salts), ammonium salts (e.g., lower alkanolammonium salts, and other pharmaceutically acceptable amine salts), for example, methylamine salts, ethylamine salts, propylamine salts, dimethylamine salts, trimethylamine salts, diethylamine salts, triethylamine salts, tert-butylamine salts, ethylene diamine salts, hydroxyethylamine salts, dihydroxyethylamine salts, trihydroxyethylamine salt and amine salts formed from morpholine, piperazine, trihydroxyethylamine salts, hydroxyethylamine salts, dihydroxyethylamine salts, trihydroxyethylamine salts, and amine salts formed from morpholine, piperazine, and lysine, respectively.

### Preparation Method

The preparation method of the compound of Formula I of the present invention are described more specifically below, but these specific methods do not constitute any limitation of the present invention. The compounds of the present invention may also be conveniently prepared by optionally combining various synthetic methods described in this specification or known in the art, and such combinations may be easily carried out by one skilled in the art to the invention.

Typically, the process flow for the preparation of the compounds of the present invention is as follows, wherein the raw materials and reagents used are commercially available if not indicated otherwise.

Representatively, compounds are prepared as follows: 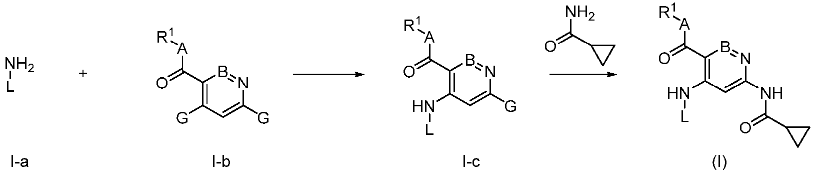 the amino compound of formula I-a is reacted with formula I-b to provide formula I-c, and formula I-c is reacted with cyclopropyl amide to provide formula (I); wherein G is halogen; and L, A, B, R¹ are as described in the first aspect of the present invention.

In another preferred embodiment, when L of formula I-a is 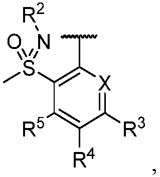 it provides a preparation method for the compound shown in formula I-a-1 or a tautomer, a mesomer, a racemate, an enantiomer, a diastereoisomer, or a mixture thereof, or a pharmaceutically acceptable salt thereof, the method includes the following steps: 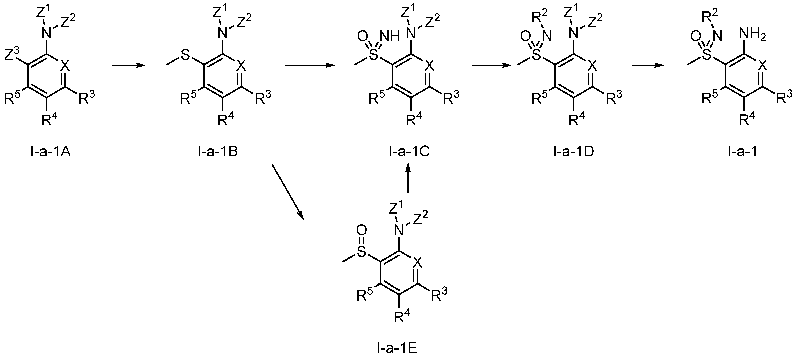 a methylthio group is introduced into Formula I-a-1A to obtain formula I-a-1B, formula I-a-1B is oxidized to obtain the sulfoxide compound formula I-a-1E, the sulfoxide compound formula I-a-1E is oxidized again to obtain the sulfoximide compound formula I-a-1C, formula I-a-1B can also be oxidized in one step to the sulfoximide compound formula I-a-1C, a substituent is introduced into the sulfoximide compound formula I-a-1C to obtain formula I-a-1D, and formula I-a-1D is deprotected to obtain formula I-a-1; wherein Z¹ and Z² are the same or different, and each independently selected from the group consisting of H, -COtBu, -Boc, -Ts, -Ns, -Bn, - PMB, -DMB; Z³ is H or halogen; and X, R², R³, R⁴, R⁵ are as described in the first aspect of the present invention.

In another preferred embodiment, it provides a preparation method for a compound of a single chiral configuration shown in Formula I-a-2 or a pharmaceutically acceptable salt thereof, the method also includes the following steps: 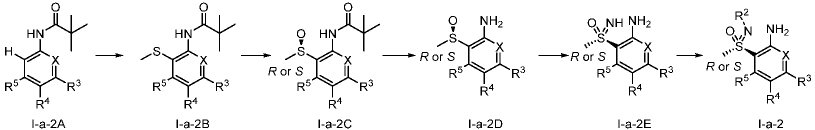 a methylthio group is introduced into formula I-a-2A after the strong base dehydrogenation to provide formula I-a-2B, formula I-a-2B is oxidized by a suitable chiral oxidation method to provide the sulfoxide compound I-a-2C in a single chiral configuration, the tertiary butyryl group is removed from the formula I-a-2C to provide formula I-a-2D, and the formula I-a-2D is oxidized again to provide the sulfoximide compound formula I-a-2E in a single chiral configuration, formula I-a-2E is subjected to a substitution reaction to give formula I-a-2 in a single chiral configuration; X, R², R³, R⁴, R⁵ are as described in the first aspect of the present invention.

In another preferred embodiment, it provides a preparation method for a compound of formula I-a-3 in a single chiral configuration or a pharmaceutically acceptable salt thereof, the method also includes the following steps: 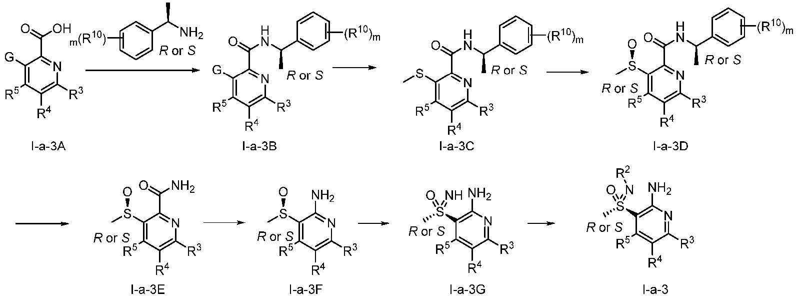 carboxylic acid compound formula I-a-3A is condensed with chiral benzylamine to provide amide compound I-a-3B, formula I-a-3B undergoes methylthio substitution to obtain formula I-a-3C, I-a-2B is oxidized by suitable chiral oxidation method to obtain sulfoxide compound formula I-a-3D, benzyl fragment is removed from formula I-a-3D to obtain amide compound formula I-a-3E, formula I-a-3E undergoes a rearrangement reaction to obtain the amide compound I-a-3F, formula I-a-3F is oxidized again to obtain the sulfoximide compound I-a-3G in a single chiral configuration, and formula I-a-3G undergoes a substitution reaction to obtain formula I-a-3 in a single chiral configuration; wherein G is selected from halogen; and R², R³, R⁴, R⁵, R¹⁰, and m are as described in the first aspect of the present invention.

In another preferred embodiment, the method also includes the following step:
further reacting the resulting product with a first substance to obtain the compound shown in formula (I); the first substance is selected from the group consisting of HCl, H₂SO₄, methanesulfonic acid, benzenesulfonic acid, and p-toluenesulfonic acid.

To ensure that each synthesis step proceeds smoothly, a person skilled in the art of the present invention may in advance perform protection and deprotection operations on the hydroxyl group in the compounds involved in the reaction, using common protecting groups such as TBS, TMS, TBDPS, Bn, THP, Tf, etc., and perform protection and deprotection operations on the NH or NH₂ in the compounds involved in the reaction, using common protecting groups such as Boc, Cbz, Bn, Ts, THP, etc.

The compounds indicated in each formula therein may contain one or more chiral centers, which exist as enantiomers and diastereomers, and may also contain many geometric isomers such as olefins, C=N double bonds, etc. Unless otherwise indicated, all chiral isomers (enantiomers and diastereomers), racemates, cis-geometric isomers, trans-geometric isomers, and mixtures of cis- and trans-geometric isomers described above are included in the present invention. For enantiomers, two enantiomers can be obtained using general chiral splitting methods or asymmetric synthesis methods. For diastereoisomers, they can be separated by methods such as stepwise recrystallization or chromatographic separation.

### Pharmaceutical Compositions and Administration Methods

The pharmaceutical composition of the present invention comprises a safe and effective amount of a compound of the present invention or a pharmacologically acceptable salt thereof, and a pharmacologically acceptable excipient or carrier. In which, "safe and effective amount" is meant that the amount of the compound is sufficient to significantly improve the condition without causing serious side effects. Generally, the pharmaceutical composition contains 1-2000 mg of the compound of the present invention/dose, more preferably, 10-1000 mg of the compound of the present invention/dose. Preferably, the "dose" is a capsule or tablet.

"Pharmaceutically acceptable carrier" means one or more compatible solid or liquid fillers or gelatinous materials which are suitable for human use and should be of sufficient purity and sufficiently low toxicity. "Compatibility" means that each component in the composition can be admixed with the compounds of the present invention and with each other without significantly reducing the efficacy of the compounds. Some examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

The pharmaceutical composition is an injection, a capsule, a tablet, a pill, a powder, or a granule.

The administration mode of the compound or pharmaceutical composition of the present invention is not particularly limited, and representative administration modes include, but are not limited to, oral, intratumoral, rectal, parenteral (intravenous, intramuscular or subcutaneous) and topical administration.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, such as starch, lactose, sucrose, glucose, mannitol and silicic acid; (b) binders, such as hydroxymethyl cellulose, alginate, gelatin, polyvinylpyrrolidone, sucrose and arabic gum; (c) humectants, such as glycerol; (d) disintegrating agents such as agar, calcium carbonate, potato starch or tapioca starch, alginic acid, certain composite silicates, and sodium carbonate; (e) retarding solvents, such as wax, (f) absorption accelerators, such as quaternary ammonium compound; (g) wetting agents, such as cetyl alcohol and glyceryl monostearate; (h) adsorbents, such as kaolin; and (i) lubricants, such as talc, calcium stearate, magnesium stearate, solid polyethylene glycol, sodium dodecyl sulfate or mixture thereof. In capsules, tablets and pills, the dosage forms may also contain buffering agents.

Solid dosage forms such as tablets, dragees, capsules, pills and granules can be prepared with coatings and shells such as enteric coatings and other materials known in the art. They may contain opacifying agents and the release of the active compound or compound in such compositions may be released in a portion of the digestive tract in a delayed manner. Examples of embedding components that can be employed are polymeric materials and waxy materials. If necessary, the active compound may also be in microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the compositions may contain adjuvants such as wetting agents, emulsifying and suspending agents, sweetening agents, flavoring agents and spices.

In addition to the active compound, the suspension may contain suspending agent, such as ethoxylated isooctadecanol, polyoxyethylene sorbitol and dehydrated sorbitan ester, microcrystalline cellulose, aluminum methoxide and agar, or the mixture thereof etc..

The compositions for parenteral injection may comprise physiologically acceptable sterile aqueous or anhydrous solutions, dispersions, suspensions or emulsions, and sterile powders which can be re-dissolved into sterile injectable solutions or dispersions. Suitable aqueous and non-aqueous carriers, diluents, solvents or excipients include water, ethanol, polyols and any suitable mixtures thereof.

Dosage forms for the compounds of the invention for topical administration include ointments, powders, patches, sprays and inhalants. The active ingredient is mixed under sterile conditions with a physiologically acceptable carrier and any preservatives, buffers, or propellants which may be required if necessary.

The compounds of the invention can be administered alone or in combination with other pharmaceutically acceptable compounds (e.g., anti-inflammatory agents).

The treatment method of the present invention can be administered alone or in combination with other treatment means or therapeutic drugs.

When the pharmaceutical composition is used, a safe and effective amount of the compound of the present invention is administered to a mammal(such as a human) in need of treatment, wherein the dosage at the time of administration is the pharmaceutically effective dosage, for people having a body weight of 60kg, the daily dose is usually 1~2000mg, preferably 50~1000mg. Of course, specific doses should also consider factors such as the administration route, the health of the patient, etc., which are within the skill of the skilled physician.

Compared with the prior art, the present invention has the following main advantages:
(1) The compounds of the present invention have excellent TYK2 inhibition activity;
(2) The compounds of the present invention have no inhibitory activity against JAK1, JAK2, and JAK3 kinases, and its JAK family kinase selectivity is very high;
(3) The compounds of the present invention have excellent IFN-α inflammatory pathway inhibition activity;
(4) The compounds of the present invention have no hERG potassium channel inhibitory activity , and have a low risk of cardiotoxicity;
(5) The compounds of the present invention have excellent properties of drug metabolism and druggability;
(6) The compounds of the present invention have excellent solubility in water.

The present invention will be further illustrated below with reference to the specific examples. It should be understood that these examples are only to illustrate the invention but not to limit the scope of the invention. Experimental methods in which the specific conditions are not specified in the following examples are usually in accordance with conventional conditions such as the conditions described in Sambrook et al., Molecular Cloning: Laboratory Manual (New York: Cold Spring Harbor Laboratory Press, 1989), or in accordance with the conditions recommended by the manufacturer. Unless indicated otherwise, percentage and parts are calculated by weight.

Unless otherwise defined, all professional and scientific terminology used in the text have the same meanings as known to the skilled in the art. In addition, any methods and materials similar or equal with the recorded content can apply to the methods of the invention. The method of the preferred embodiment described herein and the material are only for demonstration purposes.

In the present invention, the structures of the compounds were determined by nuclear magnetic resonance (NMR) or/and mass spectrometry (MS). NMR was determined with a Bruker AVANCE-400 NMR instrument. LCMS was determined using a Waters 2695 liquid phase mass spectrometer (MS type: Micromass ZQ).

HPLC was determined with Agilent 1100 high pressure liquid chromatograph (ZORBAX SP-C18 250x4.6mm column, Eclipse Plus-C18 250x4.6mm column).

The known starting materials of the present invention can be synthesized using or according to methods known in the art, or can be purchased from AcrosOrganics, Aldrich Chemical Company, J&Chem, Shaoyuan Chemical Technology, Darui Chemicals, Energy Chemical and others.

Embodiments were carried out under argon atmosphere or nitrogen atmosphere without special indication.

A CEM Discover-SP 909155 microwave reactor was used for the microwave reaction.

The temperature of the reaction was room temperature (20°C to 35°C) without special indication in the embodiments.

The silica gel plate used for thin layer chromatography (TLC) in the embodiments is 0.2mm±0.03mm; the specification of the thin layer chromatography (prep-TLC) used for purification of compounds is 0.4mm~0.5mm; the column chromatography generally uses a carrier from Yantai Huanghai silica gel of 200~300 mesh; A fully automated medium-pressure rapid purification instrument (Combi Flash Rf+UV-VIS) was used, and the separation columns were Silica Flash Column 4g, 12g, 25g. The systems of eluents for column chromatography and the unfolding systems for thin-layer chromatography included: A: dichloromethane and methanol system; B: petroleum ether and ethyl acetate system. The volume ratio of the solvents was adjusted according to the polarity of the compounds, and could also be adjusted by adding small amounts of alkaline or acidic reagents, such as ammonia or acetic acid.

The purification of the final sample compounds in the embodiments was carried out using a high-pressure preparative liquid chromatograph (manufacturer: Shimadzu, model: LC-20AP) with an Ultimate XB-C18 column (150x30mm, 5µm). The mobile phase systems were: A: acetonitrile and water (with 0.1% trifluoroacetic acid) system; B: acetonitrile and water (with 0.1% acetic acid) system.

### Example 1

### Preparation of N-(4-((3-(N,S-dimethylsulfoximino)pyridin-2-yl)amino)-5-propionylpyridin-2-yl)cyclopropylcarboxamide

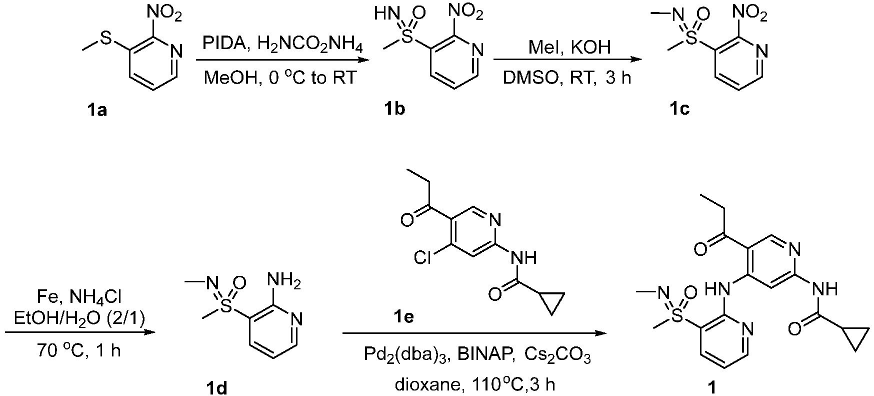

### Step 1: Synthesis of 3-(S-methylsulfoximino)-2-nitropyridine (1b)

The synthesis of 3-(methylthio)-2-nitropyridine (1a) referenced WO2020/086616.

Ammonium carbamate (867 mg, 11.1 mmol) was added to a solution of 3-(methylthio)-2-nitropyridine (1a) (1.26 g, 7.41 mmol) dissolved in methanol (20 mL), cooled down to 0°C, iodophenylene diacetic acid (4.77 g, 14.8 mmol) was added in batches, heated up to room temperature, and stirred for 2 hours. The solvent was spin-dried, 3-(S-methylsulfoximino)-2-nitropyridine (1b) (1.3 g, light yellow oil) was obtained by Flash separation (ethyl acetate/petroleum ether = 4:1), yield 79%.

1H NMR (400 MHz, CDCl₃) δ 8.71 (d, *J* = 4.7 Hz, 1H), 8.61 (d, *J* = 7.9 Hz, 1H), 7.78 (dd, *J =* 7.4, 5.1 Hz, 1H), 3.00 (brs, 1H), 3.39 (s, 3H).

MS *m*/*z* [M+H]⁺: 202.

### Step 2: Synthesis of 3-(N,S-dimethylsulfoximino)-2-nitropyridine (1c)

3-(S-methylsulfoximino)-2-nitropyridine (1b) (23.3 g, 115.9 mmol) was dissolved in DMF (230 mL), cooled in an ice bath, and sodium hydride (6.96 g, 174 mmol) was added in batches under nitrogen protection, followed by continued stirring for 20 min in an ice bath. Iodomethane (19.8 g, 139.1 mmol) was slowly added dropwise and the solution was continued to be stirred for 30 min in an ice bath. Ice water (200 mL) and saturated ammonium chloride (200 mL) were added to the reaction solution, extracted with ethyl acetate, the organic phase was washed with saturated brine, dried over anhydrous sodium sulfate, and concentrated to obtain 3-(N,S-dimethylsulfoximino)-2-nitropyridine (1c) crude (30 g).

MS *m*/*z* [M+H]⁺: 216.

### Step 3: Synthesis of 3-(N,S-dimethylsulfoximino)pyridin-2-amine (1d)

Crude 3-(N,S-dimethylsulfoximino)-2-nitropyridine (1c) (30 g) was dissolved in a solvent mixture of ethanol/water (1/1, 300 mL), ammonium chloride (62.6 g, 1.16 mol) was added, heated up to 70°C, iron powder (22.7 g, 405.8 mmol) was added in batches with stirring, and continued to stir for 1 hour at 70°C. The reaction solution was cooled to room temperature, then suction filtered, the filter cake was washed with a mixture of methanol/dichloromethane (1:10), the filtrate was concentrated and separated by column chromatography (methanol/ethyl acetate/petroleum ether = 1:9:10) to obtain 3-(*N,S*-dimethylsulfoximino) pyridin-2- amine (1d) (7.3 g, light yellow solid), two-step yield 34%.

1H NMR (400 MHz, CDCl₃) δ 8.25 (d, *J* = 3.5 Hz, 1H), 7.99 (d, *J* = 7.7 Hz, 1H), 6.79 (dd, *J* = 7.4*,* 5.0 Hz, 1H), 6.02 (s, 2H), 3.07 (s, 3H), 2.68 (s, 3H).

MS *m*/*z* [M+H]⁺: 186.

### Step 4: Synthesis of N-(4-((3-(N,S-dimethylsulfoximino)pyridin-2-yl)amino)-5-propionylpyridin-2-yl) cyclopropylcarboxamide (1)

The synthesis of *N*-(4-chloro-5-propionylpyridin-2-yl)cyclopropylcarboxamide (1e) referenced WO2020/086616.

*N*-(4-chloro-5-propionylpyridin-2-yl)cyclopropylcarboxamide (1e) (90 mg, 0.36 mmol) was dissolved in 1,4-dioxane (4 mL), 3-(*N,*S-dimethylsulfoximino)pyridin-2-amine (1d) (61 mg, 0.21 mmol), Pb₂(dba)₃ (33 mg, 0.035 mmol), BINAP (44 mg, 0.071 mmol) and CsCO₃ (232 mg, 0.71 mmol) were added. The reaction was carried out at 110°C for 3 hours under nitrogen protection. After the reaction solution was cooled to room temperature, suction filtered by diatomaceous earth, and the filter cake was washed with ethyl acetate. The filtrate was concentrated, the crude product (30 mg, yellow solid) by Flash separation (methanol/dichloromethane = 1:20), *N*-(4-((3-(*N,S-*dimethylsulfoximino)pyridin-2-yl)amino)-5-propionylpyridin-2-yl)cyclopropylcarboxamide (1) (12 mg, white solid) was obtained by prep-HPLC preparation, yield 8%.

1H NMR (400 MHz, DMSO-d6) δ 11.79 (s, 1H), 10.92 (s, 1H), 9.04 (s, 1H), 8.90 (s, 1H), 8.52 (d, *J =* 4.4 Hz, 1H), 8.21 (d, *J =* 7.7 Hz, 1H), 7.30 - 7.24 (m, 1H), 3.17 (s, 3H), 3.09 (dd, *J =* 14.0, 6.8 Hz, 2H), 2.70 (s, 3H), 2.06 - 1.98 (m, 1H), 1.09 (t, *J =* 7.1 Hz, 3H), 0.80 (d, *J* = 6.6 Hz, 4H).

MS (M+H)⁺:402.

### Example 2

### Preparation of 6-(cyclopropylcarboxamido)-4-((3-(N,S-dimethylsulfoximino)pyridin-2-yl)amino)-N-(methyl-d3)pyridazine-3-carboxamide

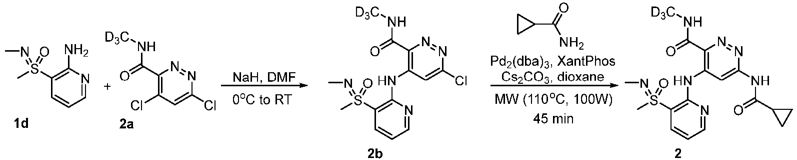

### Step 1: Synthesis of 6-chloro-4- ((3- (N,S-dimethylsulfoximino) pyridin-2-yl) amino) - N - (methyl-d3) pyridazin-3-carboxamide (2b)

Synthesis of 4,6-dichloro-*N*-(methyl-*d3*)pyridazine-3-carboxamide (2a) referenced US2019/152948.

3-(*N,S*-dimethylsulfoximino)pyridin-2-amine (1d) (130 mg, 0.70 mmol) was dissolved in DMF, cooled down to 0°C and sodium hydride (57 mg, 1.41 mmol) was added, stirred at 0°C for 5 min, 4,6-dichloro-*N*-(methyl-*d3*)pyridazine-3-carboxamide (2a) (145 mg, 0.7 mmol) was added, and the solution was continued to be stirred for 1 h at room temperature. Saturated ammonium chloride solution (15 mL) was added to the reaction solution and extracted with ethyl acetate, the combined organic phases were washed with saturated brine and dried over anhydrous sodium sulfate. After concentration, 6-chloro-4-((3-(*N,S*-dimethylsulfoximino)pyridin-2-yl)amino)-*N-*(methyl-*d3*)pyridazine-3-carboxamide (2b) (89 mg, white solid) was obtained by Flash separation (methanol: dichloromethane = 1:30), yield 35%.

1H NMR (400 MHz, CDCl3) δ 12.42 (s, 1H), 9.09 (s, 1H), 8.56 (d, *J =* 4.4 Hz, 1H), 8.32 (d, *J =* 7.7 Hz, 1H), 8.25 (s, 1H), 7.22 - 7.17 (m, 1H), 3.24 (s, 3H), 2.85 (s, 3H).

MS *m*/*z* [M+H]⁺: 358.

### Step 2: Synthesis of 6-(cyclopropylcarboxamido)-4-((3-(N,S-dimethylsulfoximino)pyridin-2-yl)amino)-N-(methyl-d3)pyridazine-3-carboxamide (2)

6-Chloro-4-((3-(*N,S*-dimethylsulfoximino)pyridin-2-yl)amino)-*N*-(methyl-d3)pyridazine-3-carboxamide (2b) (89 mg, 230 mmol) was dissolved in 1,4-dioxane (2 mL), cyclopropamide (40 mg, 46 mmol), Pd₂(dba)₃ (43 mg, 0.046 mmol), xantphos (41 mg, 070 mmol) and CsCO₃ (152 mg, 0.460 mmol) were added, and the reaction was carried out under microwave (110°C, 100W) for 45 min under nitrogen atmosphere. After the reaction solution was cooled to room temperature, filtered through diatomaceous earth, the filter cake was washed with ethyl acetate, the filtrate was concentrated and Flash separated (methanol/dichloromethane = 1:10) to obtain the crude product. 6-(cyclopropylcarboxamido)-4-((3-(N,S-dimethylsulfoximino)pyridin-2-yl)amino)-*N*-(methyl-*d3*)pyridazine-3-carboxamide (2) (45 mg, white solid) was prepared by prep-HPLC, yield 44%.

1H NMR (400 MHz, dmso) δ 11.87 (s, 1H), 11.38 (s, 1H), 9.46 (s, 1H), 9.07 (s, 1H), 8.53 (d, *J* = 4.3 Hz, 1H), 8.22 (d, *J* = 7.7 Hz, 1H), 7.28 (dd, *J* = 7.3, 5.0 Hz, 1H), 3.22 (s, 3H), 2.67 (s, 3H), 2.14 - 2.05 (m, 1H), 0.84 (d, *J =* 6.4 Hz, 4H).

MS *m*/*z* [M+H]⁺: 407.

### Example 3

### Preparation of 6-(cyclopropylcarboxamido)-4-((2-(N,S-dimethylsulfoximino)phenyl)amino)-N-methylpyridazine-3-carboxamide

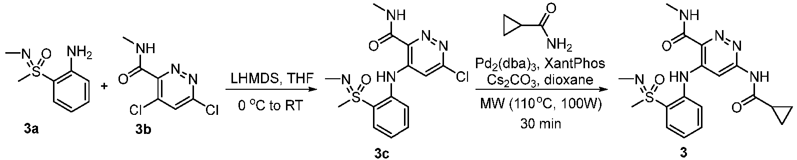

### Step 1: Synthesis of 6-chloro-4-((2-(N,S-dimethylsulfoximino)phenyl)amino)-N-methylpyridazine-3-carboxamide (3c)

### Synthesis of 2-(N,S-dimethylsulfoximino)aniline (3a) referenced WO2018/075937.

### Synthesis of 4,6-dichloro-N-methylpyridazine-3-carboxamide (3b) referenced WO2020/086616.

2-(N,S-dimethylsulfoximino)aniline (3a) (155 mg, 0.84 mmol) and 4,6-dichloro-N-methylpyridazine-3-carboxamide (3b) (175 mg, 0.84 mmol) were dissolved in tetrahydrofuran (2 mL), and LiHMDS (2.53 mL, 2.53 mmol) was slowly added to the reaction in an ice bath under nitrogen atmosphere. The reaction was slowly brought to room temperature and reacted for 10 min. The reaction was quenched with 1 N HCl (1 mL), diluted with water (20 mL) and extracted with ethyl acetate (60 mL), the organic phases were combined, washed with saturated brine and dried over anhydrous sodium sulfate. After being concentrated, 6-chloro-4-((2-(N,S-dimethylsulfoximino)phenyl)amino)-*N*-methylpyridazine-3-carboxamide (3c) (98 mg, yellow solid) was obtained by Flash separation (ethyl acetate: petroleum ether = 1:1), yield 33%.

1H NMR (400 MHz, CDCl₃) δ 11.50 (s, 1H), 8.17 (s, 1H), 8.10 (d, *J* = 7.8 Hz, 1H), 7.65 (t, *J* = 7.4 Hz, 1H), 7.55 (d, *J* = 7.9 Hz, 1H), 7.41 (t, *J* = 7.5 Hz, 1H), 7.27 (s, 1H), 3.11 - 3.03 (m, 6H), 2.80 (s, 3H).

MS *m*/*z* [M+H]⁺: 354.

### Step 2: Synthesis of 6-(cyclopropylcarboxamido)-4-((2-(N,S-dimethylsulfoximino)phenyl)amino)-N-methylpyridazine-3-carboxamide (3)

The synthesis referenced the synthesis of 6-(cyclopropylcarboxamido)-4-((3-(N,S-dimethylsulfoximino)pyridin-2-yl)amino)-*N*-(methyl-*d3*)pyridazine-3-carboxamide (Example 2), yield 40%.

1H NMR (400 MHz, dmso) δ 11.33 (s, 1H), 11.03 (s, 1H), 9.02 (d, *J* = 4.7 Hz, 1H), 8.15 (s, 1H), 7.91 (d, *J* = 8.1 Hz, 1H), 7.67 (d, *J =* 14.1 Hz, 2H), 7.41 - 7.35 (m, 1H), 3.04 (s, 3H), 2.82 (d, *J =* 4.7 Hz, 3H), 2.59 (s, 3H), 2.03 (dd, *J =* 10.9, 5.3 Hz, 1H), 0.81 - 0.75 (m, 4H).

MS *m*/*z* [M+H]⁺: 403.

### Example 4

### Preparation of 6-(cyclopropylcarboxamido)-4-((2-(N-ethyl-S-methylsulfoximino)phenyl)amino)-N-methylpyridazine-3-carboxamide

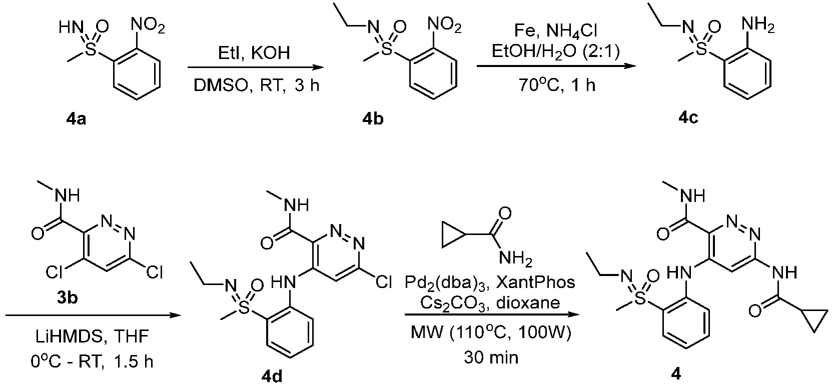

### Step 1: Synthesis of 1-(N-ethyl-S-methylsulfoximino)-2-nitrobenzene (4b)

The synthesis of 1-(S-methylsulfoximino)-2-nitrobenzene (4a) referenced WO2018/075937.

1-(S-methylsulfoximino)-2-nitrobenzene (4a) (260 mg, 1 mmol) was dissolved in DMSO (5 mL), KOH (112 mg, 2 mmol) was added, stirred for 5 min at room temperature, ethyl iodide (360 mg, 2.3 mmol) was added, and stirred for 3 h at room temperature. The reaction was quenched by water (10 mL), extracted with ethyl acetate, the organic phases were combined, washed with saturated brine and dried over anhydrous sodium sulfate. After being concentrated, 1-(N-ethyl-S-methylsulfoximino)-2-nitrobenzene (4b) (200 mg, yellow oil) was obtained by Flash separation (ethyl acetate/petroleum ether = 40% - 60%), yield 68%.

MS *m*/*z* [M+H]⁺: 229.

### Step 2: Synthesis of 2-(N-ethyl-S-methylsulfoximino)aniline (4c)

1-(*N*-ethyl-*S*-methylsulfoximino)-2-nitrobenzene (4b) (200 mg, 0.877 mmol) was dissolved in ethanol/water (2/1, 9 mL), ammonium chloride (511 mg, 9.65 mmol) was added, and iron powder (172 mg, 3.07 mmol) was added in batches at 75°C. After addition, the solution was continued to be stirred at 75°C for 1 hour. After the solution was cooled to room temperature, the solution was filtered with diatomaceous earth, and the filter cake was washed with ethanol. The filtrate was concentrated and Flash separated (methanol/dichloromethane = 5% - 10%) to obtain 2-(N-ethyl-S-methylsulfoximino)aniline (4c) (150 mg, colorless oil), yield 86%.

1H NMR (400 MHz, CDCl3) δ 7.73 (d, *J =* 8.0 Hz, 1H), 7.33 (t, *J* = 7.8 Hz, 1H), 6.81 (t, *J =* 7.6 Hz, 1H), 6.72 (d, *J =* 8.1 Hz, 1H), 5.29 (s, 2H), 3.05 (s, 3H), 2.95 - 2.80 (m, 2H), 1.21 (t, *J* = 7.2 Hz, 3H).

MS *m*/*z* [M+H]⁺: 199.

### Step 3: Synthesis of 6-chloro-4-((2-(N-ethyl-S-methylsulfoximino)phenyl)amino)-N-methylpyridazine-3-carboxamide (4d)

The synthesis referenced the synthesis of 6-chloro-4-((2-(*N,S-*dimethylsulfoximino)phenyl)amino)-*N*-methylpyridazine-3-carboxamide (3c), yield 26%.

MS *m*/*z* [M+H]⁺: 368.

### Step 4: Synthesis of 6-(cyclopropylcarboxamido)-4-((2-(N-ethyl-S-methylsulfoximino)phenyl)amino)-N-methylpyridazine-3-carboxamide (4)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide (Example 2), yield 41.0%.

1H NMR (400 MHz, DMSO-d6) δ 11.32 (s, 1H), 10.96 (s, 1H), 9.01 (d, *J =* 5.3 Hz, 1H), 8.11 (s, 1H), 7.92 (d, *J =* 7.8 Hz, 1H), 7.73 - 7.59 (m, 2H), 7.38 (t, *J =* 7.4 Hz, 1H), 3.04 (s, 3H), 2.98 - 2.85 (m, 2H), 2.81 (d, *J =* 4.8 Hz, 3H), 2.09 - 1.97 (m, 1H), 1.06 (t, *J* = 7.2 Hz, 3H), 0.85 - 0.70 (m, 4H).

MS *m*/*z* [M+H]⁺: 417.

### Example 5

### Preparation of 6-(cyclopropylcarboxamido)-4-((3-(N,S-dimethylsulfoximino)-6-methylpyridin-2-yl)amino)-N-methylpyridazine-3-carboxamide

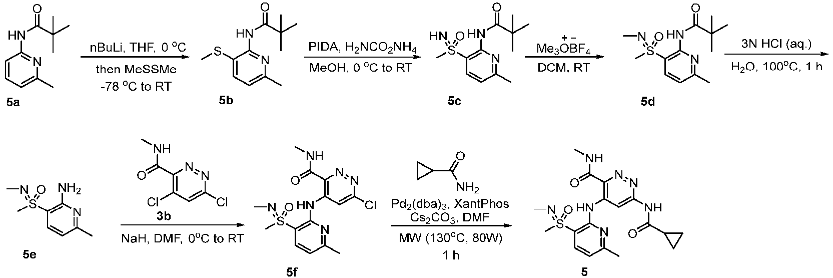

### Step 1: Synthesis of N-(6-methyl-3-methylthiopyridin-2-methyl)trimethylacetamide (5b)

The synthesis of *N*-(6-methylpyridin-2-methyl)trimethylacetamide (5a) referenced Org. Process Res. Dev. 2010, 14(1), 263-271.

*N*-(6-methylpyridin-2-methyl)trimethylacetamide (5a) (5.5 g, 28.6 mmol) was dissolved in anhydrous tetrahydrofuran (30 mL), cooled down to 0°C under nitrogen atmosphere, n-butyllithium in n-hexane solution (35 mL, 2.5 M, 85.9 mmol) was slowly added dropwise, heated up to room temperature, and stirred for 2 hours. The reaction solution was cooled down to -78°C, dimethyl disulfide (5.5 mL, 57.2 mL) was added slowly dropwise, after the addition, then the solution was slowly heated up to room temperature, and the solution was continued to be stirred for 1 hour. The reaction was quenched by the addition of saturated ammonium chloride in an ice water bath, extracted with ethyl acetate, the organic phase was washed with saturated brine and dried over anhydrous sodium sulfate. After being concentrated, the solution was Flash separated (ethyl acetate: petroleum ether = 1:1) to obtain N-(6-methyl-3-methylthiopyridin-2-methyl)trimethylacetamide (5b) (2.95 g, white solid), yield 43%.

MS *m*/*z* [M+H]⁺: 239.

### Step 2: Synthesis of N-(6-methyl-3-(S-methylsulfoximino)pyridin-2-methyl)trimethylacetamide (5c)

The synthesis referenced the synthesis of 3-(S-methylsulfoximino)-2-nitropyridine, yield 12%.

1H NMR (400 MHz, DMSO-d6) δ 10.78 (s, 1H), 8.11 (d, *J* = 8.0 Hz, 1H), 7.21 (d, *J* = 8.0 Hz, 1H), 5.04 (s, 1H), 3.10 (s, 3H), 2.48 (s, 3H), 1.23 (s, 9H).

MS *m*/*z* [M+H]⁺: 270.

### Step 3: Synthesis of N-(6-methyl-3-(N,S-dimethylsulfoximino)pyridin-2-methyl)trimethylacetamide (5d)

*N*-(6-methyl-3-(S-methylsulfoximino)pyridin-2-methyl)trimethylacetamide (5c) (370 mg, 1.38 mmol) was dissolved in dichloromethane (6 mL), trimethoxynium tetrafluoroborate (265 mg, 1.79 mmol) was added, and the reaction was stirred for 1 h at room temperature. The reaction was quenched by water and stirred for 10 minutes. The aqueous phase was extracted with dichloromethane, the organic phases were combined and dried over anhydrous sodium sulfate. After being concentrated, N-(6-methyl-3-(*N,S*-dimethylsulfoximino)pyridin-2-methyl)trimethylacetamide (5d) (190 mg, brown viscous liquid) was obtained by plate chromatography separation (methanol: dichloromethane = 1:10), yield 49%.

MS *m*/*z* [M+H]⁺: 284.

### Step 4: Synthesis of 3-(N,S-dimethylsulfoximino)-6-methylpyridin-2-amine (5e)

*N*-(6-methyl-3-(*N,S*-dimethylsulfoximino)pyridin-2-methyl)trimethylacetamide (5d) (190 mg, 0.67 mmol) was dissolved in 2 N hydrochloric acid solution (4 mL) and stirred at 100°C for 30 min. The solution was cooled to room temperature, pH = 8~9 was adjusted using saturated aqueous sodium bicarbonate. The aqueous phase was extracted with ethyl acetate, the organic phases were combined, washed with saturated brine and dried over anhydrous sodium sulfate. After being concentrated, 3-(N,S-dimethylsulfoximino)-6-methylpyridin-2-amine (5e) (79 mg, yellow liquid) was obtained by plate chromatography separation (methanol: dichloromethane = 1:10), yield 59%.

1H NMR (400 MHz, DMSO-d6) δ 7.69 (d, *J =* 7.9 Hz, 1H), 6.73 (s, 2H), 6.58 (d, *J =* 7.8 Hz, 1H), 3.01 (s, 3H), 2.46 (s, 3H), 2.29 (s, 3H).

MS *m*/*z* [M+H]⁺: 200.

### Step 5: Synthesis of 6-chloro-4-((3-(N,S-dimethylsulfoximino)-6-methylpyridin-2-yl)amino)-N-methylpyridazine-3-carboxamide (5f)

3-(*N,S*-dimethylsulfoximino)-6-methylpyridin-2-amine (5e) (63 mg, 0.316 mmol) was dissolved in anhydrous DMF (1 mL), cooled down to 0°C under nitrogen atmosphere, sodium hydride (25 mg, 60% w/w, 0.632 mmol) was added, then stirred for 20 min, 4,6-dichloro-N-methylpyridazin-3-carboxamide (3b) (65 mg, 0.316 mmol) was added, heated up to room temperature, stirred for 2 hours. The reaction was quenched by saturated ammonium chloride, extracted with ethyl acetate, the organic phases were combined, washed with saturated brine and dried over anhydrous sodium sulfate. After being concentrated, 6-chloro-4-((3-(*N,S*-dimethylsulfoximino)-6-methylpyridin-2-yl)amino)-N-methylpyridazine-3-carboxamide (5f) (40 mg, white solid) was obtained by Flash separation (methanol: dichloromethane = 1:10), yield 34%.

1H NMR (400 MHz, DMSO-*d*₆) δ 12.03 (s, 1H), 9.28 (d, *J* = 3.8 Hz, 1H), 8.97 (s, 1H), 8.12 (d, *J* = 7.9 Hz, 1H), 7.21 (d, *J =* 7.9 Hz, 1H), 3.19 (s, 3H), 2.85 (d, *J =* 4.6 Hz, 3H), 2.68 (s, 3H), 2.54 (s, 3H).

MS *m*/*z* [M+H]⁺: 369.

### Step 6: Synthesis of 6-(cyclopropylcarboxamido)-4-((3-(N,S-dimethylsulfoximino)-6-methylpyridin-2-yl)amino)-N-methylpyridazine-3-carboxamide (5)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide (Example 2), yield 33%.

1H NMR (400 MHz, DMSO-d6) δ 11.88 (s, 1H), 11.36 (s, 1H), 9.82 (s, 1H), 9.08 (d, *J =* 4.6 Hz, 1H), 8.08 (d, *J =* 7.9 Hz, 1H), 7.12 (d, *J = 7.9* Hz, 1H), 3.19 (s, 3H), 2.84 (d, *J = 4.7* Hz, 3H), 2.66 (s, 3H), 2.51 (s, 3H), 2.11 (dt, *J* = 12.5, 6.2 Hz, 1H), 0.88 - 0.81 (m, 4H).

MS *m*/*z* [M+H ]⁺: 418.

### Example 6

### Preparation of 4-((6-carbamoyl-3-(N,S-dimethylsulfoximino)pyridin-2-yl)amino)-6-(cyclopropylcarboxamido)-N-methylpyridazine-3-carboxamide

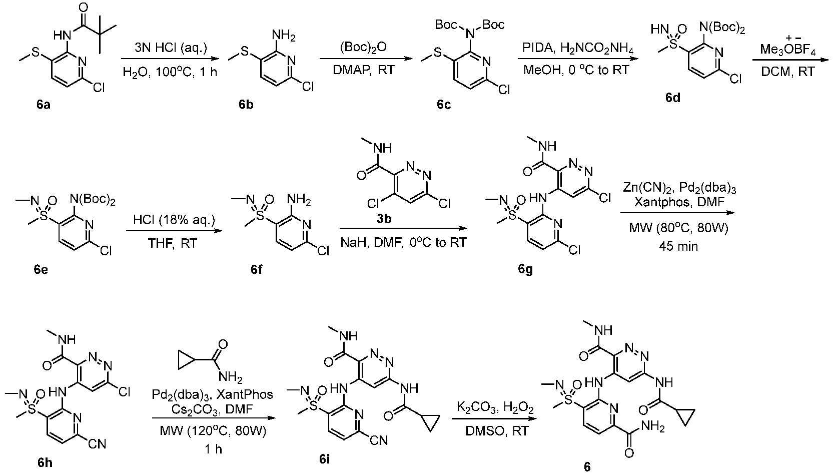

### Step 1: Synthesis of 6-chloro-3-(methylthio)pyridin-2-amine (6b)

The synthesis of *N*-(6-chloro-3-(methylthio)pyridin-2-yl)trimethylacetamide (6a) referenced J. Org. Chem. 1983, 48(20), 3401-3408.

The synthesis referenced to the synthesis of 3-(N,S-dimethylsulfoximino)-6-methylpyridin-2-amine, yield 89%.

1H NMR (400 MHz, CDCl₃) δ 7.50 (d, *J =* 7.8 Hz, 1H), 6.63 (d, *J =* 7.8 Hz, 1H), 5.25 (s, 2H), 2.34 (s, 3H).

MS *m*/*z* [M+H]⁺: 175.

### Step 2: Synthesis of N,N-(di-tert-butoxycarbonyl)-6-chloro-3-(methylthio)pyridin-2-amine (6c)

6-Chloro-3-(methylthio)pyridin-2-amine (6b) (6.0 g, 34.4 mmol) was placed in a round bottomed vial, Boc anhydride (21.7 g, 99.7 mmol) was added slowly, stirred at room temperature for 10 min, then DMAP (840 mg, 6.88 mmol) was added, the solution was continued to be stirred for 1 hour at room temperature. The solution was diluted with petroleum ether, after being concentrated, and then the solution was passed over a short column of silica gel to remove the Boc anhydride, yielding 14.7 g of a crude product. Petroleum ether was added to the crude product, beated, suction filtered and washed with petroleum ether, dried to obtain *N,N*-(di-tert-butoxycarbonyl)-6-chloro-3-(methylthio)pyridin-2-amine (6c) (12.3 g, white solid), yield 96%.

1H NMR (400 MHz, CDCl₃) δ 7.55 (d, *J =* 8.2 Hz, 1H), 7.28 (d, *J=* 9.2 Hz, 1H), 2.45 (s, 3H), 1.42 (s, 18H).

MS *m*/*z* [M+Na]⁺: 397.

### Step 3: Synthesis of N,N-(di-tert-butoxycarbonyl)-6-chloro-3-(S-methylsulfoximino)pyridin-2-amine (6d)

The synthesis referenced the synthesis of 3-(S-methylsulfoximino)-2-nitropyridine, yield 84%.

1H NMR (400 MHz, CDCl₃) δ 8.43 (d, *J =* 8.1 Hz, 1H), 7.52 (d, *J* = 8.2 Hz, 1H), 3.17 (s, 3H), 1.80 (s, 1H), 1.50 (s, 9H), 1.47 (s, 9H).

MS *m*/*z* [M+H]⁺: 406.

### Step 4: Synthesis of N,N-(di-tert-butoxycarbonyl)-6-chloro-3-(N,S-dimethylsulfoximino)pyridin-2-amine (6e)

The synthesis referenced the synthesis of N-(6-methyl-3-(*N,S-*dimethylsulfoximino)pyridin-2-methyl)trimethylacetamide, the crude product was not isolated and directly proceeded to the next step.

### Step 5: Synthesis of 6-chloro-3-(N,S-dimethylsulfoximino)pyridin-2-amine (6f)

The synthesis referenced the synthesis of 3-(*N,S*-dimethylsulfoximino)-6-methylpyridin-2-amine, yield in two steps 34%.

1H NMR (400 MHz, DMSO-*d6*) δ 7.83 (d, *J=* 8.0 Hz, 1H), 7.22 (s, 2H), 6.77 (d, *J =* 8.0 Hz, 1H), 3.08 (s, 3H), 2.49 (s, 3H).

MS *m*/*z* [M+H]⁺: 220.

### Step 6: Synthesis of 6-chloro-4-((6-chloro-3-(N,S-dimethylsulfoximino)pyridin-2-yl)amino)-N-methylpyridazine-3-carboxamide (6g)

The synthesis referenced the synthesis of 6-chloro-4-((3-(*N,S-*dimethylsulfoximino)-6-methylpyridin-2-yl)amino)-*N*-methylpyridazine-3-carboxamide, yield 39%.

1H NMR (400 MHz, DMSO-*d6*) δ 12.20 (s, 1H), 9.34 (d, *J* = 4.5 Hz, 1H), 8.77 (s, 1H), 8.22 (d, *J =* 8.1 Hz, 1H), 7.42 (d, *J =* 8.1 Hz, 1H), 3.23 (s, 3H), 2.84 (d, *J* = 4.7 Hz, 3H), 2.68 (s, 3H).

MS *m*/*z* [M+H]⁺: 389.

### Step 7: Synthesis of 6-chloro-4-((6-cyano-3-(N,S-dimethylsulfoximino)pyridin-2-yl)amino)-N-methylpyridazine-3-carboxamide (6h)

6-Chloro-4-((6-chloro-3-(*N,S*-dimethylsulfoximino)pyridin-2-yl)amino)-*N-*methylpyridazine-3-carboxamide (6 g) (133 mg, 0. 343 mmol), Zn(CN)₂ (22 mg, 0.189 mmol), Pd₂(dba)₃ (31 mg, 0.0343 mmol) and xantphos (40 mg, 0.0686 mmol) were dissolved in anhydrous DMF (2 mL), and reacted for 45 min under nitrogen atmosphere at 80°C, 80W microwave. After cooled to room temperature, the reaction was quenched with water, extracted with ethyl acetate, the organic phases were combined, washed with saturated brine and dried over anhydrous sodium sulfate. After being concentrated, 6-chloro-4-((6-cyano-3-(N,S-dimethylsulfoximino)pyridin-2-yl)amino)-N-methylpyridazine-3-carboxamide (6h) (118 mg, yellow solid) was obtained by plate chromatography separation (ethyl acetate: petroleum ether = 2:1), yield 91%.

1H NMR (400 MHz, DMSO-d6) δ 12.25 (s, 1H), 9.37 (d, *J* = 4.4 Hz, 1H), 8.77 (s, 1H), 8.41 (d, *J =* 7.4 Hz, 1H), 7.93 (d, *J =* 7.4 Hz, 1H), 3.28 (s, 3H), 2.88 (s, 3H), 2.69 (s, 3H).

MS *m*/*z* [M+H]⁺: 380.

### Step 8: Synthesis of 4-((6-cyano-3-(N,S-dimethylsulfoximino)pyridin-2-yl)amino)-6-(cyclopropylcarboxamido)-N-methylpyridazine-3-carboxamide (6i)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 21%.

1H NMR (400 MHz, DMSO-d6) δ 12.09 (s, 1H), 11.47 (s, 1H), 9.47 (s, 1H), 9.18 (d, *J =* 4.8 Hz, 1H), 8.38 (d, *J* = 7.9 Hz, 1H), 7.86 (d, *J =* 7.8 Hz, 1H), 3.30 (s, 3H), 2.85 (d, *J =* 4.7 Hz, 3H), 2.68 (s, 3H), 2.18 - 2.08 (m, 1H), 0.87 - 0.83 (m, 4H).

MS *m*/*z* [M+H]⁺: 429.

### Step 9: Synthesis of 4-((6-carbamoyl-3-(N,S-dimethylsulfoximino)pyridin-2-yl)amino)-6-(cyclopropylcarboxamido)-N-methylpyridazine-3-carboxamide (6)

4-((6-cyano-3-(*N,S*-dimethylsulfoximino)pyridin-2-yl)amino)-6-(cyclopropylcarboxamido)-*N*-methylpyridazine-3-carboxamide (6i) (15 mg, 0.035 mmol) was dissolved in DMSO (1 mL), anhydrous potassium carbonate (12 mg, 0.088 mmol) was added, and stirred at room temperature for 5 min, and then 30% hydrogen peroxide aqueous solution (39 mg, 0.35 mmol) was added, and the solution was continued to be stirred for 30 min at room temperature. The reaction solution was poured into distilled water and extracted with ethyl acetate, organic phases were combined, washed with saturated brine and dried over anhydrous sodium sulfate. After being concentrated, 4-((6-carbamoyl-3-(*N,S*-dimethylsulfoximino)pyridin-2-yl)amino)-6-(cyclopropylcarboxamido)-*N*-methylpyridazine-3-carboxamide (6) (4.5 mg, white solid) was obtained by prep-HPLC separation, yield 29%.

1H NMR (400 MHz, DMSO-d6) δ 11.99 (s, 1H), 11.60 (s, 1H), 9.48 (s, 1H), 9.13 (s, 1H), 8.38 (d, *J =* 8.0 Hz, 1H), 7.97 (s, 1H), 7.87 (d, *J* = 7.9 Hz, 1H), 7.75 (s, 1H), 3.27 (s, 3H), 2.85 (d, *J =* 4.8 Hz, 3H), 2.69 (s, 3H), 2.14 - 2.08 (m, 1H), 0.89 - 0.81 (m, 4H).

MS *m*/*z* [M+H]⁺: 447.

### Example 7

### Preparation of 4-((6-amino-3-(N,S-dimethylsulfoximino)pyridin-2-yl)amino)-6-(cyclopropylcarboxamido)-N-methylpyridazine-3-carboxamide

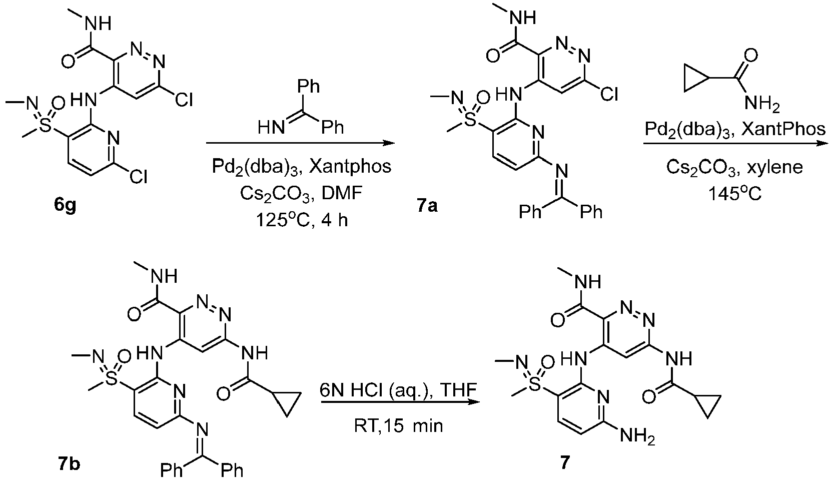

### Step 1: Synthesis of 6-chloro-4-((3-(N,S-dimethylsulfoximino)-6-((diphenylmethylene)amino)pyridin-2-yl)amino)-N-methylpyridazine-3-carboxamide (7a)

6-Chloro-4-((6-chloro-3-(*N,S*-dimethylsulfoximino)pyridin-2-yl)amino)-*N-*methylpyridazine-3-carboxamide (6 g) (4.1 g, 10.6 mmol), Pd₂(dba)₃ (484 mg, 0.53 mmol), xantphos (611 mg, 1.06 mmol) and Cs₂CO₃ (10.3 g, 31.7 mmol) were dissolved in anhydrous DMF (30 mL), benzophenone imine (3.8 g, 21.1 mmol) was added, and the reaction solution was stirred at 125°C for 4 h under nitrogen atmosphere. The reaction was quenched by addition of water, extracted with dichloromethane, and the organic phases were combined, washed with distilled water and saline respectively, dried over anhydrous sodium sulfate, and separated by column chromatography (ethyl acetate: petroleum ether = 1:1 ~ methanol: dichloromethane = 1:20) to obtain 6-chloro-4-((3-(*N,S*-dimethylsulfoximino)-6-((diphenylmethylene)amino)pyridin-2-yl)amino)-N-methylpyridazine-3-carboxamide (7a) (4.17 g, earthy yellow solid), yield 74%.

MS *m*/*z* [M+H]⁺: 534.

### Steps 2 and 3: Synthesis of 4-((6-amino-3-(N,S-dimethylsulfoximino)pyridin-2-yl)amino)-6-(cyclopropylcarboxamido)-N-methylpyridazine-3-carboxamide (7)

6-Chloro-4-((3-(*N,S*-dimethylsulfoximino)-6-((diphenylmethylene)amino)pyridin-2-yl)amino)-N-methylpyridazine-3-carboxamide (7a) (4.67 g, 8.75 mmol) was dissolved in xylene (50 mL), cyclopropanamide (1.48 g, 17.4 mmol), Pd₂(dba)₃ ( 500 mg, 0.875 mmol), xantphos (760 mg, 1.31 mmol) and Cs₂CO₃ (5.66 g, 17.4 mmol) were added, and stirred at 145°C for 3 h under nitrogen atmosphere. After being cooled to room temperature, the solution was diluted by a solvent mixture of methanol/dichloromethane (1:10), filtered through diatomaceous earth, and the filter cake was washed with a solvent mixture of methanol/dichloromethane (1:10). The crude product 6-(cyclopropylcarboxamido)-4-((3-(*N,S*-dimethylsulfoximino)-6-((diphenylmethylene)amino)pyridin-2-yl)amino)-N-methylpyridazine-3-carboxamide (7b) was obtained after being concentrated, which was directly used in the next step of the reaction without separation.

The crude 6-(cyclopropylcarboxamido)-4-((3-(N,S-dimethylsulfoximino)-6-((diphenylmethylene)amino)pyridin-2-yl)amino)-N-methylpyridazine-3-carboxamide (7b) was dissolved in tetrahydrofuran (40 mL), and 6 N hydrochloric acid (30 mL) was added in an ice bath, and the solution was heated up to room temperature and stirred for 1 hour. Anhydrous potassium carbonate was added slowly in batches to adjust pH = 9~10, suction filtered and concentrated. 4-((6-amino-3-(N,S-dimethylsulfoximino)pyridin-2-yl)amino)-6-(cyclopropylcarboxamido)-N-methylpyridazine-3-carboxamide (7) (1.87 g, earthy yellow solid) was obtained by column chromatography separation (methanol: dichloromethane = 1:20 - 1:7), yield 51%.

1H NMR (400 MHz, DMSO-d6) δ 11.55 (s, 1H), 11.31 (s, 1H), 9.51 (s, 1H), 9.01 (d, *J =* 4.7 Hz, 1H), 7.74 (d, *J =* 8.4 Hz, 1H), 6.54 (s, 2H), 6.24 (d, *J =* 8.7 Hz, 1H), 3.06 (s, 3H), 2.84 (d, *J =* 4.4 Hz, 3H), 2.63 (s, 3H), 2.16 - 2.06 (m, 1H), 0.92 - 0.80 (m, 4H).

MS *m*/*z* [M+H]⁺: 419.

### Example 8

### Preparation of 4-((5-cyano-3-(N,S-dimethylsulfoximino)pyridin-2-yl)amino)-6-(cyclopropylcarboxamide)-N-methylpyridazine-3-carboxamide

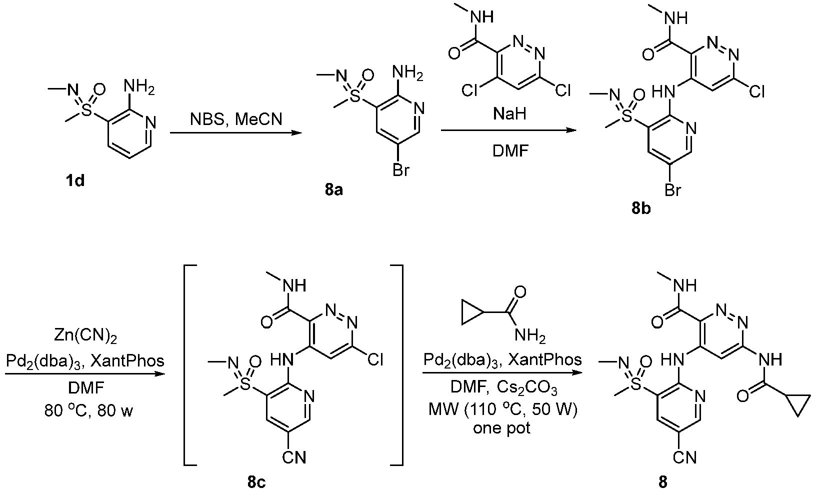

### Step 1: Synthesis of 5-bromo-3-(N,S-dimethylsulfoximino)pyridin-2-amine (8a)

3-(*N,S*-dimethylsulfoximino)pyridin-2-amine (1d) (500 mg, 2.7 mmol) was dissolved in acetonitrile (15 mL), NBS (504 mg, 2.83 mmol) was added slowly, and stirred at room temperature for 1 h. TLC detection showed that the reaction was completed. Acetonitrile was spin-dried, the reaction was quenched with water, extracted with ethyl acetate, washed with saturated brine, dried over anhydrous sodium sulfate, concentrated and Flash separated (ethyl acetate/petroleum ether = 40 - 90%) to obtain 5-bromo-3-(*N,S*-dimethylsulfoximino)pyridin-2-amine (8a) (640 mg, white solid), yield 89%.

1H NMR (400 MHz, CDCl₃) δ 8.29 (d, *J =* 2.1 Hz, 1H), 8.10 (d, *J =* 2.0 Hz, 1H), 6.07 (brs, 2H), 3.08 (s, 3H), 2.68 (s, 3H).

MS *m*/*z* [M+H]⁺: 264.

### Step 2: Synthesis of 4-((5-bromo-3-(N,S-dimethylsulfoximino)pyridin-2-yl)amino)-6-chloro-N-methylpyridazine-3-carboxamide (8b)

The synthesis referenced the synthesis of 6-chloro-4-((3-(*N,S-*dimethylsulfoximino)-6-methylpyridin-2-yl)amino)-*N*-methylpyridazine-3-carboxamide, yield 61%.

1H NMR (400 MHz, CDCl₃) δ 12.43 (s, 1H), 8.97 (s, 1H), 8.59 (s, 1H), 8.42 (s, 1H), 8.28 (brs, 1H), 3.26 (s, 3H), 3.07 (d, *J =* 5.1 Hz, 3H), 2.83 (s, 3H).

MS *m*/*z* [M+H]⁺: 433.

### Steps 3 and 4: Synthesis of 4-((5-cyano-3-(N,S-dimethylsulfoximino)pyridin-2-yl)amino)-6-(cyclopropylcarboxamide)-N-methylpyridazine-3-carboxamide (8)

4-((5-bromo-3-(*N,S*-dimethylsulfoximino)pyridin-2-yl)amino)-6-chloro-*N-*methylpyridazine-3-carboxamide (8b) (205 mg, 0.472 mmol), Zn(CN)₂ (28 mg, 0.236 mmol), Pd₂(dba)₃ (43 mg, 0.0472 mmol) and xantphos (55 mg, 0.0944 mmol) were dissolved in anhydrous DMF (3 mL), and the solution was reacted under nitrogen conditions at 60°C, 50W microwave for 1 h and then 80°C, 80W for 15 min. The reaction solution was cooled to room temperature, and cyclopropanamide (44 mg, 0.519 mmol), Pd₂(dba)₃ (43 mg, 0.0472 mmol), xantphos (55 mg, 0.0944 mmol) and Cs₂CO₃ (308 mg, 0.944 mmol) were added to the reaction system under nitrogen atmosphere, and the solution was reacted at 110°C, 50W microwave for 1 hour. The reaction was quenched with water, extracted with ethyl acetate, washed with saturated brine, dried over anhydrous sodium sulfate, concentrated and Flash separated (methanol/dichloromethane = 5 - 7%) to obtain 4-((5-cyano-3-(*N,S-*dimethylsulfoximino)pyridin-2-yl)amino)-6-(cyclopropylcarboxamide)-*N-*methylpyridazine-3-carboxamide(8) ( 114 mg, white solid), yield 56%.

1H NMR (400 MHz, DMSO-*d6*) δ 12.20 (s, 1H), 11.50 (s, 1H), 9.46 (s, 1H), 9.16 (s, 1H), 8.93 (s, 1H), 8.49 (s, 1H), 3.29 (s, 3H), 2.84 (d, *J =* 4.4 Hz, 3H), 2.66 (s, 3H), 2.15 - 2.07 (s, 1H), 0.92 - 0.80 (m, 4H).

MS *m*/*z* [M+H]⁺: 429.

### Example 9

### Preparation of 4-((5-amino-3-(N,S-dimethylsulfoximino)pyridin-2-yl)amino)-6-(cyclopropylcarboxamido)-N-methylpyridazine-3-carboxamide

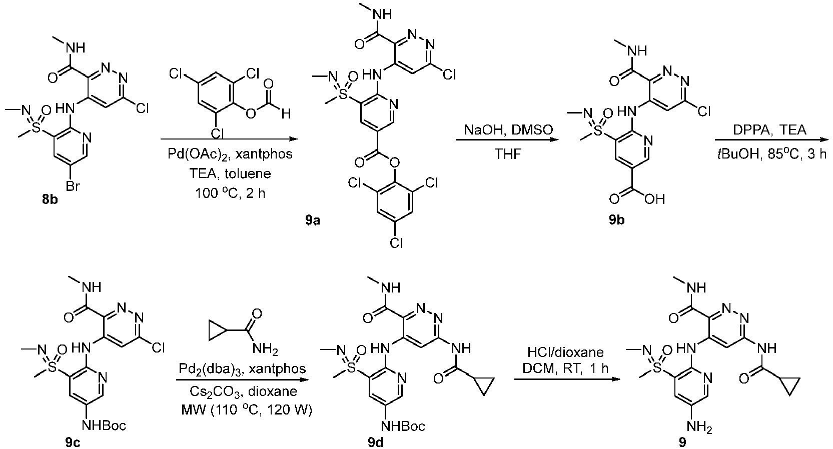

### Step 1: Synthesis of 6-((6-chloro-3-(methylcarbamoyl)pyridazin-4-yl)amino)-5-(N,S-dimethylsulfoximino)nicotinic acid-2,4,6-trichlorophenyl ester (9a)

4-((5-bromo-3-(*N,S*-dimethylsulfoximino)pyridin-2-yl)amino)-6-chloro-N-methylpyridazine-3-carboxamide (8b) (560 mg, 1.3 mmol), 2,4,6-trichlorophenyl formate (349 mg, 1.55 mmol), Pd(OAc)₂ (63 mg, 0.26 mmol), xantphos (243 mg, 0.42 mmol) and triethylamine (263 mg, 2.6 mmol) were added to a solution of toluene (28 mL), and stirred at 100°C for 2 hours under nitrogen atmosphere. The reaction solution was cooled to room temperature, filtered with diatomaceous earth and washed with ethyl acetate. The filtrate was concentrated and Flash separated (ethyl acetate/petroleum ether = 40 - 80%) to obtain 6-((6-chloro-3-(methylcarbamoyl)pyridazin-4-yl)amino)-5-(N,S-dimethylsulfoximino)nicotinic acid-2,4,6-trichlorophenyl ester (9a) (380 mg, pale yellow solid), yield 51%.

1H NMR (400 MHz, DMSO-d6) δ 12.47 (s, 1H), 9.42 (d, *J* = 4.7 Hz, 1H), 9.37 (d, *J =* 1.8 Hz, 1H), 9.02 (s, 1H), 8.75 (d, *J* = 1.9 Hz, 1H), 7.95 (s, 2H), 5.74 (s, 1H), 3.36 (s, 3H), 2.87 (d, *J* = 4.7 Hz, 3H), 2.72 (s, 3H).

MS *m*/*z* [M+H]⁺: 577.

### Step 2: Synthesis of 6-((6-chloro-3-(methylcarbamoyl)pyridazin-4-yl)amino)-5-(N,S-dimethylsulfoximino)nicotinic acid (9b)

6-((6-Chloro-3-(methylcarbamoyl)pyridazin-4-yl)amino)-5-(N,S-dimethylsulfoximino)nicotinic acid-2,4,6-trichlorophenyl ester (9a) (175 mg, 0.3 mmol) was dissolved in DMSO (6.5 mL), and an aqueous 40% sodium hydroxide (500 mg, 40 % w/w) was added to the mixture in an ice bath, after the addition, and the solution was stirred at room temperature for 20 min. The pH was adjusted to 3~4 with 1N hydrochloric acid, extracted with dichloromethane, the organic phases were combined and dried over anhydrous sodium sulfate. The crude 6-((6-chloro-3-(methylcarbamoyl)pyridazin-4-yl)amino)-5-(N,S-dimethylsulfoximino)nicotinic acid (9b) (179 mg, yellowish solid) was obtained by concentration, which was used directly in the next step without separation.

MS *m*/*z* [M+H]⁺: 399.

### Step 3: Synthesis of tert-butyl (6-((6-chloro-3-(methylcarbamoyl)pyridazin-4-yl)amino)-5-(N,S-dimethylsulfoximino)pyridin-3-yl)carbamate (9c)

Triethylamine (50 mg, 0.495 mmol) and DPPA (136 mg, 0.495 mmol) were added to a crude 6-((6-chloro-3-(methylcarbamoyl)pyridazin-4-yl)amino)-5-(*N,S-*dimethylsulfoximino)nicotinic acid (9b) (179 mg) in tert-butanol (12 mL), and stirred at 85°C for 2 hours under N₂ atmosphere. The reaction solution was cooled to room temperature and concentrated and Flash separated (ethyl acetate/petroleum ether = 40 - 90%) to obtain tert-butyl (6-((6-chloro-3-(methylcarbamoyl)pyridazin-4-yl)amino)-5-(*N,S*-dimethylsulfoximino)pyridin-3-yl)carbamate (9c) (69 mg, pale yellow solid), yield 33%.

MS *m*/*z* [M+H]⁺: 470.

### Step 4: Synthesis of tert-butyl (6-((6-(cyclopropylcarboxamido)-3-(methylcarbamoyl)pyridazin-4-yl)amino)-5-(N,S-dimethylsulfoximino)pyridin-3-yl)carbamate (9d)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 29%.

MS *m*/*z* [M+H]⁺: 519.

### Step 5: Synthesis of 4-((5-amino-3-(N,S-dimethylsulfoximino)pyridin-2-yl)amino)-6-(cyclopropylcarboxamido)-N-methylpyridazine-3-carboxamide (9)

Tert-butyl (6-((6-(cyclopropylcarboxamido)-3-(methylcarbamoyl)pyridazin-4-yl)amino)-5-(*N,S*-dimethylsulfoximino)pyridin-3-yl)carbamate (9d) (22 mg, 0.042 mmol) was dissolved in dichloromethane (1.5 mL), 4 M dioxane hydrochloride solution (1.5 mL, 6 mmol) was added, and stirred for 1 h at room temperature. The solvent was spin-dried, diluted with water (1.5 mL), pH > 11 was adjusted using 33% aqueous potassium carbonate, extracted with ethyl acetate, the organic phases were combined and dried over anhydrous sodium sulfate. The crude product was obtained after filtration and concentration, 4-((5-amino-3-(*N,S*-dimethylsulfoximino)pyridin-2-yl)amino)-6-(cyclopropylcarboxamido)-*N*-methylpyridazine-3-carboxamide (9) (6.7 mg, white solid) was obtained by prep-HPLC preparation, yield 38%.

1H NMR (400 MHz, DMSO-*d6*) δ 11.34 (s, 1H), 11.23 (s, 1H), 9.00 (d, *J* = 4.9 Hz, 1H), 8.92 (s, 1H), 7.91 (d, *J =* 2.8 Hz, 1H), 7.53 (d, *J =* 2.8 Hz, 1H), 5.66 (s, 2H), 3.10 (s, 3H), 2.82 (d, *J =* 4.8 Hz, 3H), 2.63 (s, 3H), 2.11 - 2.03 (m, 1H), 0.82 (d, *J =* 6.7 Hz, 4H).

MS *m*/*z* [M+H]⁺: 419.

### Example 10

### Preparation of 4-((5-amino-3-(N,S-dimethylsulfoximino)pyridin-2-yl)amino)-6-(cyclopropylcarboxamido)-N-(methyl-d3)pyridazine-3-carboxamide

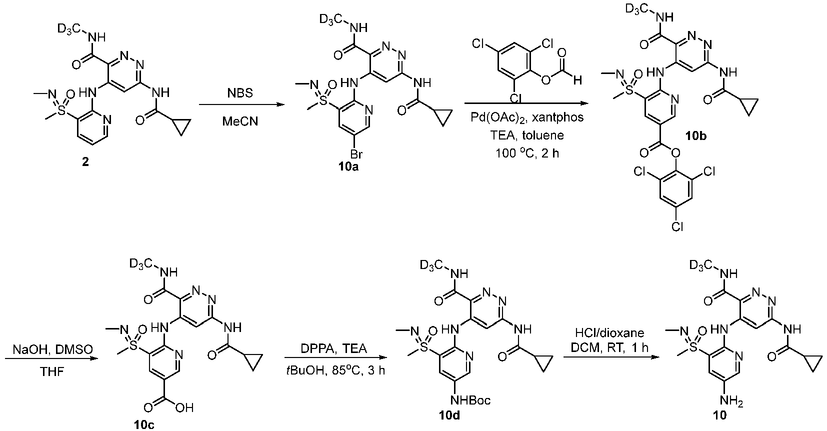

### Step 1: Synthesis of 4-((5-bromo-3-(N,S-dimethylsulfoximino)pyridin-2-yl)amino)-6-(cyclopropylcarboxamido)-N-methylpyridazine-3-carboxamide (10a)

The synthesis referenced the synthesis of 5-bromo-3-(N,S-dimethylsulfoximino)pyridin-2-amine, yield 16%.

MS *m*/*z* [M+H]⁺: 485.

### Step 2: Synthesis of 6-((6-(cyclopropylcarboxamido)-3-(methylcarbamoyl)pyridazin-4-yl)amino)-5-(N,S-dimethylsulfoximino)nicotinic acid-2,4,6-trichlorophenyl ester (10b)

The synthesis referenced the synthesis of 6-((6-chloro-3-(methylcarbamoyl)pyridazin-4-yl)amino)-5-(*N,S*-dimethylsulfoximino)nicotinic acid-2,4,6-trichlorophenyl ester, yield 82%.

MS *m*/*z* [M+H]⁺: 629.

### Step 3: Synthesis of 6-((6-(cyclopropylcarboxamido)-3-(methylcarbamoyl)pyridazin-4-yl)amino)-5-(N,S-dimethylsulfoximino)nicotinic acid (10c)

The synthesis referenced the synthesis of 6-((6-chloro-3-(methylcarbamoyl)pyridazin-4-yl)amino)-5-(N,S-dimethylsulfoximino)nicotinic acid, the crude product was directly used in the next step without separation.

MS *m*/*z* [M+H]⁺: 402.

### Step 4: Synthesis of tert-butyl (6-((6-(cyclopropylcarboxamido)-3-((methyl-d3)carbamoyl))pyridazin-4-yl)amino)-5-(N,S-dimethylsulfoximino)pyridin-3-yl)carbamate (10d)

The synthesis referenced the synthesis of tert-butyl (6-((6-chloro-3-(methylcarbamoyl)pyridazin-4-yl)amino)-5-(*N,S*-dimethylsulfoximino)pyridin-3-yl)carbamate, two-step yield 25%.

MS *m*/*z* [M+H]⁺: 522.

### Step 5: Synthesis of 4-((5-amino-3-(N,S-dimethylsulfoximino)pyridin-2-yl)amino)-6-(cyclopropylcarboxamido)-N-(methyl-d3)pyridazine-3-carboxamide (10)

The synthesis referenced the synthesis of 4-((5-amino-3-(*N,S-*dimethylsulfoximino)pyridin-2-yl)amino)-6-(cyclopropylcarboxamido)-*N-*methylpyridazine-3-carboxamide, yield 16%.

1H NMR (400 MHz, DMSO-*d6*) δ 11.34 (s, 1H), 11.23 (s, 1H), 8.97 (s, 1H), 8.92 (s, 1H), 7.91 (d, *J* = 2.7 Hz, 1H), 7.53 (d, *J =* 2.7 Hz, 1H), 5.66 (s, 2H), 3.10 (s, 3H), 2.63 (s, 3H), 2.10 - 2.04 (m, 1H), 0.82 (d, *J =* 6.6 Hz, 4H).

MS *m*/*z* [M+H]⁺: 422.

### Example 11

### Preparation of 6-(cyclopropylcarboxamido)-4-((3-(N,S-dimethylsulfoximino)-5-hydroxypyridin-2-yl)amino)-N-methylpyridazine-3-carboxamide

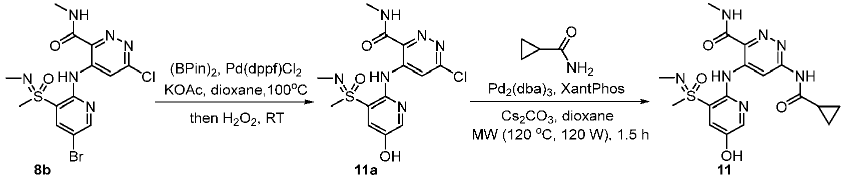

### Step 1: Synthesis of 6-chloro-4-((3-(N,S-dimethylsulfoximino)-5-hydroxypyridin-2-yl)amino)-N-methylpyridazine-3-carboxamide (11a)

4-((5-bromo-3-(*N,S*-dimethylsulfoximino)pyridin-2-yl)amino)-6-chloro-*N-*methylpyridazine-3-carboxamide (8b) (200 mg, 0.463 mmol), bis(pinacolato)diboron (151 mg, 0.594 mmol), Pd(dppf)Cl₂ (34 mg, 0.046 mmol) and anhydrous potassium acetate (133 mg, 1.400 mmol) were dissolved in dioxane and stirred at 100°C for 1 h under nitrogen atmosphere. After being cooled to room temperature, 30% aqueous hydrogen peroxide (103 mg, 0.909 mmol) was added to the reaction solution and stirred at room temperature for 30 min. The reaction solution was diluted by adding ethyl acetate and water, the aqueous phase was extracted with ethyl acetate, the organic phases were combined, washed with saturated brine and dried over anhydrous sodium sulfate. After being concentrated, 6-chloro-4-((3-(*N,S*-dimethylsulfoximino)-5-hydroxypyridin-2-yl)amino)-*N*-methylpyridazine-3-carboxamide (11a) (124 mg, yellow solid) was obtained by column chromatography separation (methanol: dichloromethane = 1:20), yield 73%.

1H NMR (400 MHz, DMSO-d6) δ 11.79 (s, 1H), 10.45 (s, 1H), 9.26 (d, *J =* 4.6 Hz, 1H), 8.56 (s, 1H), 8.21 (d, *J =* 2.8 Hz, 1H), 7.69 (d, *J =* 2.8 Hz, 1H), 3.19 (s, 3H), 2.84 (d, *J =* 4.7 Hz, 3H), 2.67 (s, 3H).

MS *m*/*z* [M+H]⁺: 371.

### Step 2: Synthesis of 6-(cyclopropylcarboxamido)-4-((3-(N,S-dimethylsulfoximino)-5-hydroxypyridin-2-yl)amino)-N-methylpyridazine-3-carboxamide (11)

The synthesis referenced the synthesis of 6-(cyclopropylcarboxamido)-4-((3-(*N,S-*dimethylsulfoximino)pyridin-2-yl)amino)-*N*-(methyl-d3)pyridazine-3-carboxamide, yield 69%.

1H NMR (400 MHz, DMSO-*d6*) δ 11.52 (s, 1H), 11.28 (s, 1H), 9.07 (s, 1H), 9.04 (d, *J =* 4.9 Hz, 1H), 8.10 (d, *J* = 2.9 Hz, 1H), 7.66 (d, *J =* 2.9 Hz, 1H), 3.16 (s, 3H), 2.83 (d, *J* = 4.8 Hz, 3H), 2.64 (s, 3H), 2.12 - 2.03 (m, 1H), 0.83 (d, *J* = 6.6 Hz, 4H).

MS *m*/*z* [M+H]⁺: 420.

### Example 12

### Preparation of 6-(cyclopropylcarboxamido)-4-((3-(N,S-dimethylsulfoximino)-5-fluoropyridin-2-yl)amino)-N-methylpyridazine-3-carboxamide

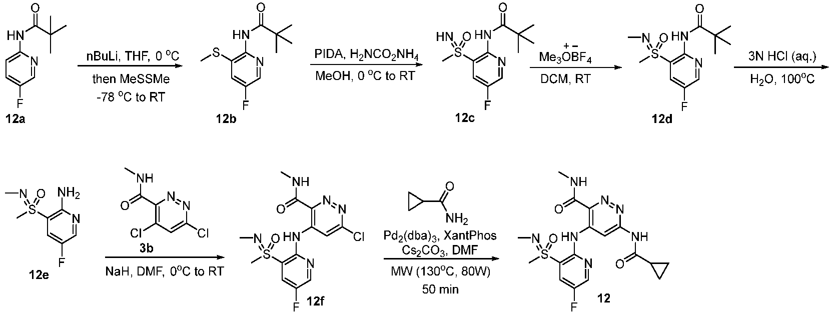

Preparation of 6-(cyclopropylcarboxamido)-4-((3-(N,S-dimethylsulfoximino)-5-fluoropyridin-2-yl)amino)-N-methylpyridazine-3-carboxamide (12) referenced to Example 5 for the preparation of 6-(cyclopropylcarboxamido)-4-((3-(N,S-dimethylsulfoximino)-6-methylpyridin-2-yl)amino)-N-methylpyridazine-3 - carboxamide, *N*-(6-methylpyridin-2-methyl)trimethylacetamide was replaced with N-(5-fluoropyridin-2-yl)trimethylacetamide (12a) as starting material.

### Step 1: Synthesis of N-(5-fluoro-3-methylthiopyridin-2-yl)trimethylacetamide (12b), yield 26%.

The synthesis of *N*-(5-fluoropyridin-2-yl)trimethylacetamide (12a) referenced Org. Lett. 2013, 15(13), 3460-3463.

1H NMR (400 MHz, DMSO-d6) δ 9.50 (s, 1H), 8.16 (d, *J =* 2.2 Hz, 1H), 7.70 - 7.63 (d, *J =* 8.0, 2.2 Hz, 1H), 2.42 (s, 3H), 1.21 (s, 9H).

MS *m*/*z* [M+H]⁺: 243.

### Step 2: Synthesis of N-(3-(S-methylsulfoximino)-5-fluoropyridin-2-yl)trimethylacetamide (12c), yield 28%.

1H NMR (400 MHz, DMSO-d6) δ 10.57 (s, 1H), 8.66 (s, 1H), 8.12 (d, *J* = 7.4 Hz, 1H), 7.68 (d, *J =* 13.8 Hz, 1H), 3.17 (s, 3H), 1.22 (s, 9H).

MS *m*/*z* [M+H]⁺: 274.

### Step 3: Synthesis of N-(3-(N,S-dimethylsulfoximino)-5-fluoropyridin-2-yl)trimethylacetamide (12d), yield 14%.

1H NMR (400 MHz, DMSO-*d6*) δ 10.30 (s, 0.65H), 9.81 (s, 0.35H), 8.78 (s, 0.35H), 8.68 (d, *J =* 2.4 Hz, 0.65H), 8.31 (d, *J =* 7.5 Hz, 0.35H), 8.09 (dd, *J =* 7.6, 2.4 Hz, 0.65H), 3.25 (s, 3H), 2.56 (s, 3H), 1.23 (d, *J =* 11.5 Hz, 9H).

MS *m*/*z* [M+H]⁺: 288.

### Step 4: Synthesis of 3-(N,S-dimethylsulfoximino)-5-fluoropyridin-2-amine (12e), yield 47%.

1H NMR (400 MHz, DMSO-d6) δ 8.34 (s, 1H), 7.79 (d, *J =* 7.6 Hz, 1H), 6.82 (s, 2H), 3.19 (s, 3H), 2.57 (s, 3H).

MS *m*/*z* [M+H]⁺: 204.

### Step 5: Synthesis of 6-chloro-4-((3-(N,S-dimethylsulfoximino)-5-fluoropyridin-2-yl)amino)-N-methylpyridazine-3-carboxamide (12f), yield 32%.

1H NMR (400 MHz, DMSO-d6) δ 12.03 (s, 1H), 9.31 (s, 1H), 8.71 (s, 2H), 8.12 (d, *J =* 5.7 Hz, 1H), 3.27 (s, 3H), 2.85 (d, *J =* 4.4 Hz, 3H), 2.68 (s, 3H).

MS *m*/*z* [M+H]⁺: 373.

### Step 6: Synthesis of 6-(cyclopropylcarboxamido)-4-((3-(N,S-dimethylsulfoximino)-5-fluoropyridin-2-yl)amino)-N-methylpyridazine-3-carboxamide (12), yield 29%.

1H NMR (400 MHz, DMSO-d6) δ 11.82 (s, 1H), 11.39 (s, 1H), 9.29 (s, 1H), 9.11 (d, *J =* 4.8 Hz, 1H), 8.60 (d, *J =* 3.0 Hz, 1H), 8.08 (dd, *J =* 7.7, 3.0 Hz, 1H), 3.26 (s, 3H), 2.84 (d, *J =* 4.8 Hz, 3H), 2.66 (s, 3H), 2.15 - 2.05 (m, 1H), 0.90 - 0.78 (m, 4H).

MS *m*/*z* [M+H]⁺: 422.

### Example 13

### Preparation of 4-((5-bromo-3-(N,S-dimethylsulfoximino)pyridin-2-yl)amino)-6-(cyclopropylcarboxamido)-N-methylpyridazine-3-carboxamide

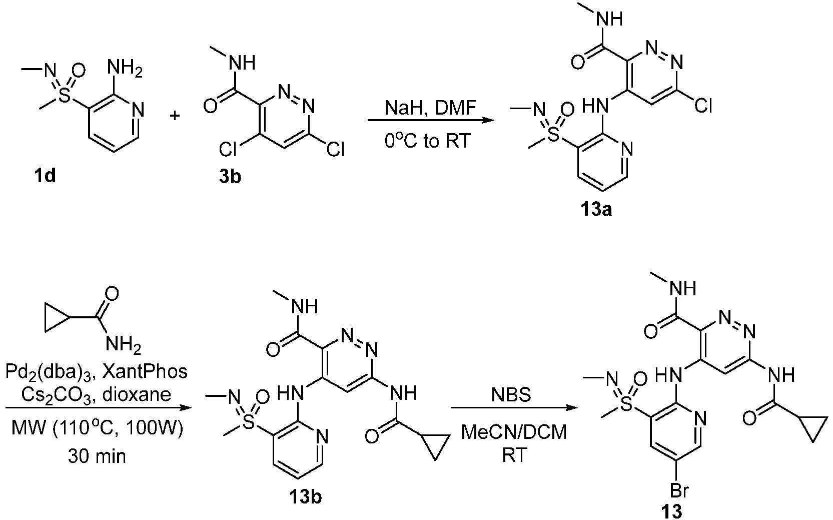

### Step 1: Synthesis of 6-chloro-4-((3-(N,S-dimethylsulfoximino)pyridin-2-yl)amino)-N-methylpyridazine-3-carboxamide (13a)

The synthesis referenced the synthesis of 6-chloro-4-((3-(*N,S-*dimethylsulfoximino)-6-methylpyridin-2-yl)amino)-*N*-methylpyridazine-3-carboxamide, yield 40%.

1H NMR (400 MHz, DMSO-d6) δ 12.07 (s, 1H), 9.28 (s, 1H), 8.88 (s, 1H), 8.63 (d, *J* = 3.5 Hz, 1H), 8.24 (d, *J* = 7.6 Hz, 1H), 7.35 (dd, *J =* 7.3, 5.1 Hz, 1H), 3.21 (s, 3H), 2.84 (d, *J* = 4.5 Hz, 3H), 2.68 (s, 3H).

MS *m*/*z* [M+H]⁺: 355.

### Step 2: Synthesis of 6-(cyclopropylcarboxamido)-4-((3-(N,S-dimethylsulfoximino)pyridin-2-yl)amino)-N-methylpyridazine-3-carboxamide (13b)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 44%.

1H NMR (400 MHz, DMSO-*d6*) δ 11.85 (s, 1H), 11.37 (s, 1H), 9.45 (s, 1H), 9.08 (d, *J =* 5.3 Hz, 1H), 8.52 (d, *J =* 4.7 Hz, 1H), 8.21 (d, *J =* 7.7 Hz, 1H), 7.27 (dd, *J =* 7.8, 4.9 Hz, 1H), 3.20 (s, 3H), 2.83 (d, *J =* 4.6 Hz, 3H), 2.66 (s, 3H), 2.13 - 2.04 (m, 1H), 0.83 (d, *J =* 7.0 Hz, 4H).

MS *m*/*z* [M+H]⁺: 404.

### Step 3: Synthesis of 4-((5-bromo-3-(N,S-dimethylsulfoximino)pyridin-2-yl)amino)-6-(cyclopropylcarboxamido)-N-methylpyridazine-3-carboxamide (13)

6-(Cyclopropylcarboxamido)-4-((3-(*N,S*-dimethylsulfoximino)pyridin-2-yl)amino)-*N*-methylpyridazine-3-carboxamide (13b) (490 mg, 1.21 mmol) was dissolved in a solvent mixture of acetonitrile (10 mL) and dichloromethane (10 mL), and NBS (183 mg, 1.03 mmol) was added to the mixture under an ice water bath, and stirred at room temperature for 2 hours. The reaction was quenched with saturated ammonium chloride (35 mL), diluted with water, extracted with ethyl acetate, the organic phases were combined, washed with saturated brine and dried over anhydrous sodium sulfate. After being concentrated, 4-((5-bromo-3-(*N,S-*dimethylsulfoximino)pyridin-2-yl)amino)-6-(cyclopropylcarboxamido)-*N-*methylpyridazine-3-carboxamide (13) (100 mg, pale yellow solid) was obtained by Flash separation (methanol/dichloromethane = 10%), yield 17%.

1H NMR (400 MHz, DMSO-*d6*) δ 11.88 (s, 1H), 11.42 (s, 1H), 9.34 (s, 1H), 9.12 (d, *J =* 4.8 Hz, 1H), 8.65 (d, *J* = 2.4 Hz, 1H), 8.26 (d, *J* = 2.4 Hz, 1H), 3.27 (s, 3H), 2.84 (d, *J* = 4.8 Hz, 3H), 2.65 (s, 3H), 2.15 - 2.05 (m, 1H), 0.89 - 0.80 (m, 4H).

MS *m*/*z* [M+H]⁺: 482.

### Example 14

### Preparation of 6-(cyclopropylamido)-4-((3-(N,S-dimethylsulfoximino)-5-methoxypyridin-2-yl)amino)-N-methylpyridazine-3-carboxamide

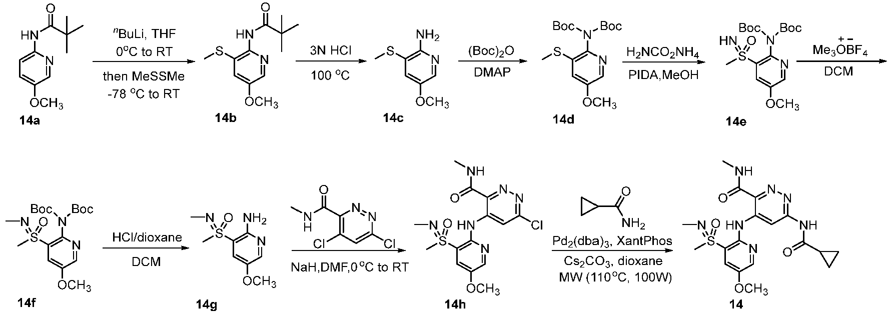

### Step 1: Synthesis of N-(5-methoxy-3-methylthio-pyridin-2-yl)trimethylacetamide (14b)

The synthesis of *N*-(5-methoxy-pyridin-2-yl)trimethylacetamide (14a) referenced US2011/178053.

The synthesis of 14b referenced the synthesis of *N*-(6-methyl-3-methylthio-pyridin-2-methyl)trimethylacetamide, yield 58%.

1H NMR (400 MHz, CDCl₃) δ 7.95 (d, *J* = 2.3 Hz, 1H), 7.77 (s, 1H), 7.14 (d, *J*= 2.1 Hz, 1H), 3.86 (s, 3H), 2.42 (s, 3H), 1.34 (s, 9H).

MS *m*/*z* [M+H]⁺: 255.

### Step 2: Synthesis of 5-methoxy-3-methylthiopyridin-2-amine (14c)

The synthesis referenced the synthesis of 3-(N,S-dimethylsulfoximino)-6-methylpyridin-2-amine, yield 84%.

1H NMR (400 MHz, CDCl₃) δ 7.70 - 7.67 (m, 1H), 7.20 - 7.17 (m, 1H), 4.65 (s, 2H), 3.78 (s, 3H), 2.39 (s, 3H).

MS *m*/*z* [M+H]⁺: 171.

### Step 3: Synthesis of N,N-(di-tert-butoxycarbonyl)-5-methoxy-3-methylthiopyridin-2-amine 14d

The synthesis referenced the synthesis of *N,N*-(di-tert-butoxycarbonyl)-6-chloro-3-methylthiopyridin-2-amine, yield 70%.

1H NMR (400 MHz, CDCl₃) δ 7.93 (s, 1H), 7.07 (s, 1H), 3.89 (s, 3H), 2.43 (s, 3H), 1.41 (s, 18H).

MS *m*/*z* [M+H]⁺: 371.

### Step 4: Synthesis of N,N-(di-tert-butoxycarbonyl)-3-(S-methylsulfoximino)-5-methoxypyridin-2 amine (14e)

The synthesis referenced the synthesis of 3-(*S*-methylsulfoximino)-2-nitropyridine, yield 85%.

1H NMR (400 MHz, CDCl₃) δ 8.36 (s, 1H), 7.94 (s, 1H), 3.94 (s, 3H), 3.16 (s, 3H), 2.04 (s, 1H), 1.47 (d, *J =* 9.8 Hz, 18H).

MS *m*/*z* [M+H]⁺: 402.

### Step 5: Synthesis of N,N-(di-tert-butoxycarbonyl)-3-(N,S-dimethylsulfoximino)-5-methoxypyridin-2-amine (14f)

The synthesis referenced the synthesis of *N*-(6-methyl-3-(*N,S-*dimethylsulfoximino)pyridin-2-methyl)trimethylacetamide, yield 80%.

MS *m*/*z* [M+H]⁺: 416.

### Step 6: Synthesis of 3-(N,S-dimethylsulfoximino)-5-methoxypyridin-2-amine (14g)

The synthesis referenced the synthesis of 6-chloro-3-(*N,S-*dimethylsulfoximino)pyridin-2-amine, yield 23%.

1H NMR (400 MHz, CDCl₃) δ 8.04 (s, 1H), 7.61 (s, 1H), 5.64 (s, 2H), 3.82 (s, 3H), 3.10 (s, 3H), 2.68 (s, 3H).

MS *m*/*z* [M+H]⁺: 202.

### Step 7: Synthesis of 6-chloro-4-((3-(N,S-dimethylsulfoximino)-5-methoxypyridin-2-yl)amino)-N-methylpyridazine-3-carboxamide (14h)

The synthesis referenced the synthesis of 6-chloro-4-((3-(*N,S-*dimethylsulfoximino)-6-methylpyridin-2-yl)amino)-*N-*methylpyridazine-3-carboxamide, yield 14%.

MS *m*/*z* [M+H]⁺: 385.

### Step 8: Synthesis of 6-(cyclopropylcarboxamido)-4-((3-(N,S-dimethylsulfoximino)-5-methoxypyridin-2-yl)amino)-N-methylpyridazine-3-carboxamide (14)

The synthesis referenced the synthesis of 6-(cyclopropylcarboxamido)-4-((3-(*N,S-*dimethylsulfoximino)pyridin-2-yl)amino)-*N*-(methyl-d3)pyridazine-3-carboxamide, yield 79%.

1H NMR (400 MHz, CDCl₃) δ 11.65 (s, 1H), 11.32 (s, 1H), 9.17 (s, 1H), 9.06 (d, *J =* 4.7 Hz, 1H), 8.31 (d, *J =* 2.8 Hz, 1H), 7.75 (d, *J =* 2.8 Hz, 1H), 3.90 (s, 3H), 3.21 (s, 3H), 2.84 (d, *J* = 4.7 Hz, 3H), 2.66 (s, 3H), 2.14 - 2.05 (m, 1H), 0.84 (d, *J* = 7.1 Hz, 4H).

MS *m*/*z* [M+H]⁺: 434.

### Example 15

### Preparation of 6-(cyclopropylcarboxamido)-4-((3-(N,S-dimethylsulfoximino)-5-methylpyridin-2-yl)amino)-N-methylpyridazine-3-carboxamide

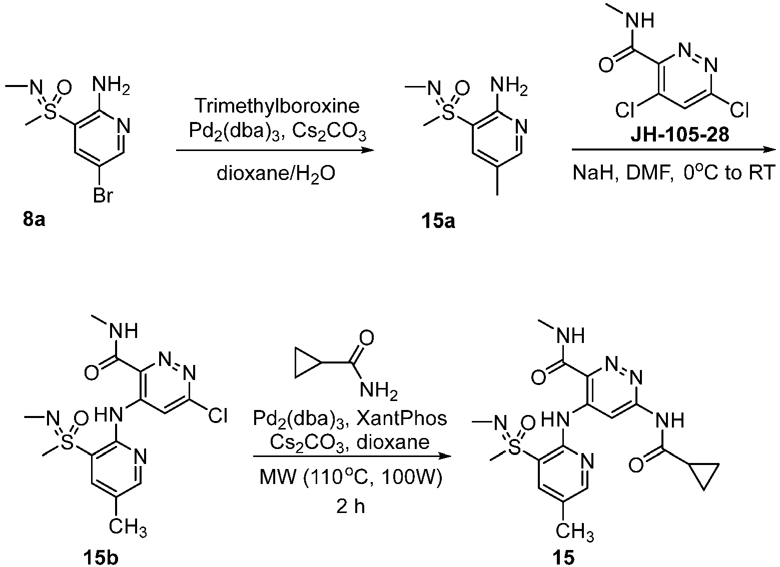

### Step 1: Synthesis of 3-(N,S-dimethylsulfoximino)-5-methylpyridin-2-amine (15a)

5-Bromo-3-(*N,S*-dimethylsulfoximino)pyridin-2-amine (8a) (100 mg, 0.38 mmol), palladium tetrakis(triphenylphosphine) (88 mg, 0.076 mmol) and cesium carbonate (124 mg, 0.38 mmol) were dissolved in a mixed solvent of dioxane (1.8 mL) and water (0.2 mL), and 3 M trimethyl epoxide borane in tetrahydrofuran (0.2 mL, 0.6 mmol)was added to the reaction solution, and the solution was reacted for 3 hours at 100°C under nitrogen atmosphere. The reaction was cooled to room temperature, quenched with water, extracted with ethyl acetate, washed with saturated brine and dried over anhydrous sodium sulfate. After being concentrated, 3-(N,S-dimethylsulfoximino)-5-methylpyridin-2- amine (15a) (50 mg, white solid) was obtained by plate chromatography separation (methanol: dichloromethane = 1:10), yield 66%.

1H NMR (400 MHz, CDCl₃) δ 8.09 (s, 1H), 7.82 (s, 1H), 5.82 (s, 2H), 3.07 (s, 3H), 2.68 (s, 3H), 2.26 (s, 3H).

MS *m*/*z* [M+H]⁺: 200.

### Step 2: Synthesis of 6-chloro-4-((3-(N,S-dimethylsulfoximino)-5-methylpyridin-2-yl)amino)-N-methylpyridazine-3-carboxamide (15b)

The synthesis referenced the synthesis of 6-chloro-4-((3-(*N,S-*dimethylsulfoximino)-6-methylpyridin-2-yl)amino)-N-methylpyridazine-3-carboxamide, yield 22%.

MS *m*/*z* [M+H]⁺: 369.

### Step 3: Synthesis of 6-(cyclopropylcarboxamido)-4-((3-(N,S-dimethylsulfoximino)-5-methylpyridin-2-yl)amino)-N-methylpyridazine-3-carboxamide (15)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 62%.

1H NMR (400 MHz, DMSO-*d6*) δ 11.77 (s, 1H), 11.34 (s, 1H), 9.38 (s, 1H), 9.06 (*d*, J = 4.3 Hz, 1H), 8.39 (s, 1H), 8.05 (s, 1H), 3.19 (s, 3H), 2.84 (d, *J* = 4.1 Hz, 3H), 2.66 (s, 3H), 2.34 (s, 3H), 2.14 - 2.04 (m, 1H), 0.84 (d, *J* = 6.4 Hz, 4H).

MS *m*/*z* [M+H]⁺: 418.

### Example 16

### Preparation of 6-(cyclopropylcarboxamido)-4-((3-(N,S-dimethylsulfoximino)-5-(3-methoxypropoxy)pyridin-2-yl)amino)-N-methylpyridazine-3-carboxamide

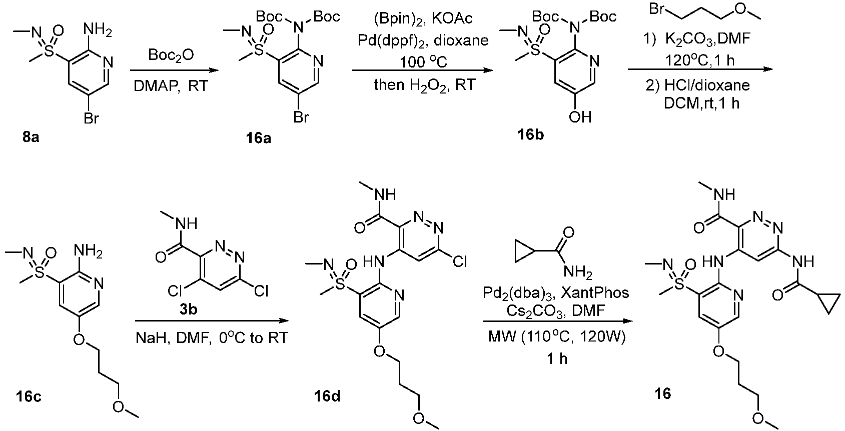

### Step 1: Synthesis of N,N-(di-tert-butoxycarbonyl)-5-bromo-3-(N,S-dimethylsulfoximino)pyridin-2-amine (16a)

The synthesis referenced the synthesis of N,N-(di-tert-butoxycarbonyl)-6-chloro-3-methylthiopyridin-2-amine, yield 91%.

1H NMR (400 MHz, CDCl₃) δ 8.76 (s, 1H), 8.44 (s, 1H), 3.14 s, 3H), 2.63 (s, 3H), 1.52 (s, 9H), 1.44 (s, 9H).

MS *m*/*z* [M+H]⁺: 464.

### Step 2: Synthesis of N,N-(di-tert-butoxycarbonyl)-3-(N,S-dimethylsulfoximino)-5-hydroxypyridin-2-amine (16b)

The synthesis referenced the synthesis of 6-chloro-4-((3-(*N,S-*dimethylsulfoximino)-5-hydroxypyridin-2-yl)amino)*-N*-methylpyridazine-3-carboxamide, yield 96%.

1H NMR (400 MHz, CDCl₃) δ 8.38 (s, 1H), 7.83 (s, 1H), 3.16 (s, 3H), 2.70 (s, 3H), 1.53 (s, 9H), 1.44 (s, 9H).

MS *m*/*z* [M+H]⁺: 402.

### Step 3: Synthesis of 3-(N,S-dimethylsulfoximino)-5-(3-methoxypropoxy)pyridin-2-amine (16c)

Anhydrous potassium carbonate (1.04 g, 7.5 mmol) was added to *N,N*-(di-tert-butoxycarbonyl)-3-(*N,S*-dimethylsulfoximino)-5-hydroxypyridin-2-amine (16b) (500 mg, 1.25 mmol) in anhydrous DMF (10 mL), and stirred at room temperature for 10 min, 1-bromo-3-methoxypropane (230 mg, 1.5 mmol) was added to the reaction solution, and stirred at 120°C for 1 hour. The reaction solution was cooled to room temperature, diluted with water (50 mL), extracted with ethyl acetate, the organic phases were combined, washed with saturated brine, dried over anhydrous sodium sulfate, and concentrated to obtain the crude product as a yellow oil. The yellow oily crude was dissolved in dichloromethane (4 mL), and 4 M hydrogen chloride in dioxane (4 mL) was added at 0°C, heated up to room temperature and stirred for 1 hour. The solvent was concentrated and spin-dried, diluted with water, pH = 8 ~ 9 was adjusted by adding saturated sodium bicarbonate, and the aqueous phase was extracted with ethyl acetate. The organic phases were combined, washed with saturated brine and dried over anhydrous sodium sulfate. After being concentrated, 3-(*N,S-*dimethylsulfoximino)-5-(3-methoxypropoxy)pyridin-2-amine (16c) (202 mg, yellow solid) was obtained by Flash separation (methanol/dichloromethane = 3 - 5%), yield 59%.

1H NMR (400 MHz, DMSO-d6) δ 8.03 (d, *J =* 2.9 Hz, 1H), 7.45 (d, *J =* 2.9 Hz, 1H), 6.40 (s, 2H), 3.99 (t, *J =* 6.4 Hz, 2H), 3.45 (t, *J =* 6.3 Hz, 2H), 3.24 (s, 3H), 3.08 (s, 3H), 2.50 (s, 3H), 1.95 - 1.87 (m, 2H).

MS *m*/*z* [M+H]⁺: 274.

### Step 4: Synthesis of 6-chloro-4-((3-(N,S-dimethylsulfoximino)-5-(3-methoxypropoxy)pyridin-2-yl)amino)-N-methylpyridazine-3-carboxamide (16d)

The synthesis referenced the synthesis of 6-chloro-4-((3-(*N,S-*dimethylsulfoximino)-6-methylpyridin-2-yl)amino)-*N*-methylpyridazine-3-carboxamide, yield 39%.

MS *m*/*z* [M+H]⁺: 443.

### Step 5: Synthesis of 6-(cyclopropylcarboxamido)-4-((3-(N,S-dimethylsulfoximino)-5-(3-methoxypropoxy)pyridin-2-yl)amino)-N-methylpyridazine-3-carboxamide (16)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 58%.

1H NMR (400 MHz, DMSO-d6) δ 11.65 (s, 1H), 11.32 (s, 1H), 9.16 (s, 1H), 9.06 (d, *J* = 4.9 Hz, 1H), 8.29 (d, *J* = 2.7 Hz, 1H), 7.75 (d, *J* = 2.7 Hz, 1H), 4.16 (t, *J* = 6.2 Hz, 2H), 3.48 (t, *J* = 6.2 Hz, 2H), 3.25 (s, 3H), 3.21 (s, 3H), 2.84 (d, *J* = 4.7 Hz, 3H), 2.66 (s, 3H), 2.13 - 2.06 (m, 1H), 2.02 - 1.92 (m, 2H), 0.84 (d, *J* = 7.1 Hz, 4H).

MS *m*/*z* [M+H]⁺: 492.

### Example 17

### Preparation of 6-(cyclopropylcarboxamido)-4-((3-(N,S-dimethylsulfoximino)-5-(2-(epoxybutan-3-yl)ethoxy)pyridin-2-yl)amino)-N-methylpyridazine-3-carboxamide

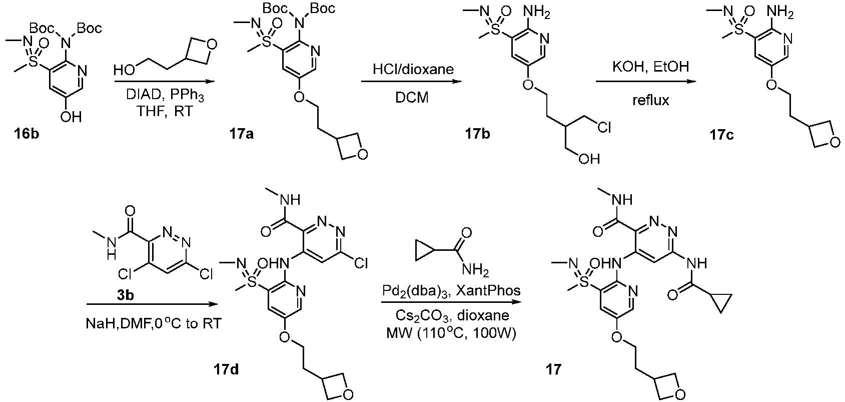

### Steps 1, 2 and 3: Synthesis of 3-(N,S-dimethylsulfoximino)-5-(2-(epoxybutan-3-yl)ethoxy)pyridin-2-amine (17c)

*N,N*-(di-tert-butoxycarbonyl)-3-(*N,S*-dimethylsulfoximino)-5-hydroxypyridin-2-amine (16b) (300 mg, 0.748 mmol), 3-hydroxyethyl oxetane (76 mg, 0.748 mmol) and triphenylphosphine (393 mg, 1.5 mmol) were dissolved in anhydrous tetrahydrofuran (5 mL), and DIAD (303 mg, 1.5 mmol) was added under nitrogen atmosphere, and the reaction was carried out at room temperature for 2 hours. The reaction was quenched with water, extracted with ethyl acetate, washed with saturated brine, and dried over anhydrous sodium sulfate. After being concentrated, crude *N,N*-(di-tert-butoxycarbonyl)-3-(*N,S*-dimethylsulfoximino)-5-(2-(epoxybutan-3-yl)ethoxy)pyridin-2- amine (17a) (954 mg) was obtained by Flash separation (methanol/dichloromethane = 4 - 5%).

Crude *N,N*-(di-tert-butoxycarbonyl)-3-(*N,S*-dimethylsulfoximino)-5-(2-(epoxybutan-3-yl)ethoxy)pyridin-2-amine (17a) was dissolved in dichloromethane (10 mL), 4 N HCl in dioxane (10 mL) was added, and the reaction was carried out for 30 min at room temperature, and concentrated to give crude 4-((6-amino-5-(*N,S-*dimethylsulfoximino)pyridin-3-yl)-oxy)-2-(chloromethyl)-1-ol (17b).

The crude 4-((6-amino-5-(*N,S*-dimethylsulfoximino)pyridin-3-yl)-oxy)-2-(chloromethyl)-1-ol (17b) was dissolved in ethanol (10 mL), potassium hydroxide (210 mg, 3.74 mmol) was added, and refluxed for 4 h. LCMS detection showed that the reaction was completed. The reaction solution was cooled to room temperature, ethanol was spin-dried, the reaction was quenched with water, extracted with ethyl acetate, washed with saturated brine, dried over anhydrous sodium sulfate, concentrated and Flash separated (methanol/dichloromethane = 3~10%) to give 3-(*N,S-*dimethylsulfoximino)-5-(2-(epoxybutan-3-yl)ethoxy)pyridin-2-amine (17c) (62 mg, yellow solid), three-step yield 29%.

1H NMR (400 MHz, CDCl₃) δ 7.99 (d, *J* = 2.9 Hz, 1H), 7.57 (d, *J=* 3.0 Hz, 1H), 5.65 (s, 2H), 4.84 (dd, *J* = 7.8, 6.0 Hz, 2H), 4.49 (t, *J* = 6.2 Hz, 2H), 3.93 (t, *J* = 6.1 Hz, 2H), 3.22 (dt, *J =* 14.5, 7.2 Hz, 1H), 3.09 (s, 3H), 2.67 (s, 3H), 2.21 - 2.13 (m, 2H).

MS *m*/*z* [M+H]⁺: 286.

### Step 4: Synthesis of 6-(chloro-4-((3-(N,S-dimethylsulfoximino)-5-(2-(epoxybutan-3-yl)ethoxy)pyridin-2-yl)amino)-N-methylpyridazine-3-carboxamide (17d)

The synthesis referenced the synthesis of 6-chloro-4-((3-(*N,S-*dimethylsulfoximino)-6-methylpyridin-2-yl)amino)-*N*-methylpyridazine-3-carboxamide, yield 18%.

MS *m*/*z* [M+H]⁺: 455.

### Step 5: Synthesis of 6-(cyclopropylcarboxamido)-4-((3-(N,S-dimethylsulfoximino)-5-(2-(epoxybutan-3-yl)ethoxy)pyridin-2-yl)amino)-N-methylpyridazine-3-carboxamide (17)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 60%.

1H NMR (400 MHz, DMSO-d6) δ 11.64 (s, 1H), 11.32 (s, 1H), 9.16 (s, 1H), 9.06 (d, *J* = 4.9 Hz, 1H), 8.27 (d, *J* = 3.0 Hz, 1H), 7.71 (d, *J* = 3.0 Hz, 1H), 4.67 (dd, *J* = 7.9, 5.9 Hz, 2H), 4.36 (t, *J=* 6.1 Hz, 2H), 4.09 (td, *J* = 6.4, 2.4 Hz, 2H), 3.20 (s, 3H), 3.17 - 3.11 (m, 1H), 2.83 (d, *J =* 4.8 Hz, 3H), 2.65 (s, 3H), 2.14 - 2.04 (m, 3H), 0.87 - 0.78 (m, 4H).

MS *m*/*z* [M+H]⁺: 504.

### Example 18

### Preparation of 6-(cyclopropylcarboxamido)-4-((3-(N,S-dimethylsulfoximino)-5-(2-morpholinoethoxy)pyridin-2-yl)amino)-N-(methyl-d3)pyridazine-3-carboxamide

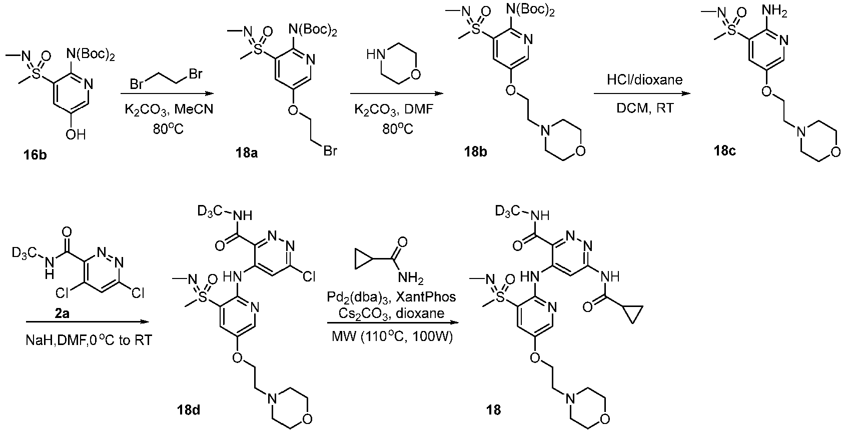

### Step 1: Synthesis of 5-(2-bromoethoxy)-3-(N,S-dimethylsulfoximino)pyridin-2-amine (18a)

*N,N*-(di-tert-butoxycarbonyl)-3-(*N,S*-dimethylsulfoximino)-5-hydroxypyridin-2-amine (16b) (400 mg, 1.0 mmol) was dissolved in acetonitrile (20 mL), anhydrous potassium carbonate (688 mg, 5.0 mmol) and 1,3-dibromoethane (1.87 g, 10.0 mmol) were added, and stirred at 80 °C for 45 minutes. The reaction solution was cooled to room temperature, filtered and the filter cake was washed with acetonitrile. The filtrate was concentrated and separated by column chromatography (ethyl acetate/petroleum ether = 10 - 65%) to obtain 5-(2-bromoethoxy)-3-(*N,S*-dimethylsulfoximino)pyridin-2-amine (18a) (462 mg, colorless oil), yield 91%.

1H NMR (400 MHz, CDCl₃) δ 8.40 (d, *J =* 2.9 Hz, 1H), 7.83 (d, *J =* 2.5 Hz, 1H), 4.42 (t, *J = 5.9* Hz, 2H), 3.69 (t, *J* = 5.9 Hz, 2H), 3.15 (s, 3H), 2.62 (s, 3H), 1.51 (s, 9H), 1.43 (s, 9H).

MS *m*/*z* [M+H]⁺: 508.

### Step 2: Synthesis of N,N-(di-tert-butoxycarbonyl)-3-(N,S-dimethylsulfoximino)-5-(2-morpholinoethoxy)pyridin-2-amine (18b)

5-(2-Bromoethoxy)-3-(*N,S*-dimethylsulfoximino)pyridin-2-amine (18a) (300 mg, 0.59 mmol) was dissolved in DMF (4 mL), anhydrous potassium carbonate (245 mg, 1.77 mmol) and morpholine (154 mg, 1.77 mmol) were added, and stirred at 80 °C for 1 hour. The reaction solution was cooled to room temperature, filtered, and the filter cake was washed with ethyl acetate. The filtrate was concentrated and separated by column chromatography (methanol/dichloromethane = 0 - 6%) to obtain *N,N*-(di-tert-butoxycarbonyl)-3-(*N,S*-dimethylsulfoximino)-5-(2-morpholinoethoxy)pyridin-2-amine (18b) (273 mg, colorless oil), yield 90%.

MS *m*/*z* [M+H]⁺: 515.

### Step 3: Synthesis of 3-(N,S-dimethylsulfoximino)-5-(2-morpholinoethoxy)pyridin-2-amine (18c)

4 M hydrogen chloride in dioxane (2.5 mL) was slowly added dropwise to *N,N-*(di-tert-butoxycarbonyl)-3-(*N,S*-dimethylsulfoximino)-5-(2-morpholinoethoxy)pyridin-2- amine (18b) (287 mg, 0.558 mmol) in dichloromethane (5 mL) at 0°C, and stirred for 1 hour at room temperature. pH = 8~9 was adjusted by adding saturated sodium bicarbonate to the reaction solution, the solvent was spin-dried, 3-(*N,S*-dimethylsulfoximino)-5-(2-morpholinoethoxy)pyridin-2-amine (18c) (156 mg, light yellow solid) was obtained by column chromatography separation (methanol/dichloromethane = 0-9%), yield 89%.

MS *m*/*z* [M+H]⁺: 315.

### Step 4: Synthesis of 6-chloro-4-((3-(N,S-dimethylsulfoximino)-5-(2-morpholinylethoxy)pyridin-2-yl)amino)-N-(methyl-d3)pyridazine-3-carboxamide (18d)

The synthesis referenced the synthesis of 6-chloro-4-((3-(*N,S-*dimethylsulfoximino)pyridin-2-yl)amino)-*N*-(methyl-*d3*)pyridazine-3-carboxamide, yield 41%.

1H NMR (400 MHz, CDCl₃) δ 12.21 (s, 1H), 8.83 (s, 1H), 8.27 (d, *J* = 3.0 Hz, 1H), 8.21 (s, 1H), 7.91 (d, *J =* 3.0 Hz, 1H), 4.21 (t, *J =* 5.5 Hz, 2H), 3.75 (t, *J* = 4.7 Hz, 4H), 3.22 (s, 3H), 2.87 - 2.81 (s, 5H), 2.59 (d, *J =* 4.9 Hz, 4H).

MS *m*/*z* [M+H]⁺: 487.

### Step 5: Synthesis of 6-(cyclopropylcarboxamido)-4-((3-(N,S-dimethylsulfoximino)-5-(2-morpholinylethoxy)pyridin-2-yl)amino)-N-(methyl-d3)pyridazine-3-carboxamide (18)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 28%.

1H NMR (400 MHz, DMSO-d6) δ 11.65 (s, 1H), 11.32 (s, 1H), 9.16 (s, 1H), 9.04 (s, 1H), 8.31 (d, *J =* 2.9 Hz, 1H), 7.77 (d, *J* = 2.9 Hz, 1H), 4.27 - 4.18 (m, 2H), 3.61 - 3.54 (m, 4H), 3.20 (s, 3H), 2.70 (t, *J =* 5.5 Hz, 2H), 2.65 (s, 3H), 2.47 - 2.42 (m, 4H), 2.12 - 2.04 (m, 1H), 0.89 - 0.77 (m, 4H).

MS *m*/*z* [M+H]⁺: 536.

### Example 19

### Preparation of 4-((5-(3-cyanopropoxy)-3-(N,S-dimethylsulfoximino)pyridin-2-yl)amino)-6-(cyclopropylcarboxamido)-N-(methyl-d3)pyridazine-3-carboxamide

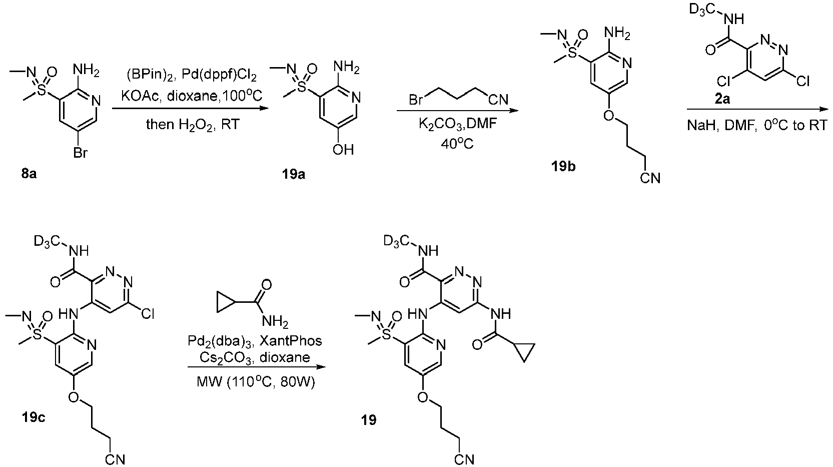

### Step 1: Synthesis of 6-amino-5-(N,S-dimethylsulfoximino)-3-hydroxypyridine (19a)

The synthesis referenced the synthesis of 6-chloro-4-((3-(*N,S-*dimethylsulfoximino)-5-hydroxypyridin-2-yl)amino)-*N*-methylpyridazine-3-carboxamide, yield 63%.

1H NMR (400 MHz, DMSO-d6) δ 9.29 (s, 1H), 7.86 (d, *J* = 2.9 Hz, 1H), 7.36 (d, *J =* 2.9 Hz, 1H), 6.16 (s, 2H), 3.05 (s, 3H), 2.48 (s, 3H).

MS *m*/*z* [M+H]⁺: 202.

### Step 2: Synthesis of 4-((6-amino-5-(N,S-dimethylsulfoximino)pyridin-3-yl)oxybutyronitrile (19b)

4-bromobutyronitrile (172 mg, 1.16 mmol) was added to 6-amino-5-(*N,S-*dimethylsulfoximino)-3-hydroxypyridine (19a) (195 mg, 0.968 mmol) in DMF (4 mL), anhydrous potassium carbonate (535 mg, 3.87 mmol) was added, and the reaction solution was heated up to 40°C and stirred for 30 min. The reaction solution was cooled to room temperature and filtered, washed with ethyl acetate, the filtrate was concentrated and separated by column chromatography (methanol/dichloromethane = 1 - 2.5%) to obtain 4-((6-amino-5-(*N,S*-dimethylsulfoximino)pyridin-3-yl)oxybutyronitrile (19b) (148 mg, yellow solid), yield 57%.

¹H NMR (400 MHz, DMSO-d6) δ 8.06 (d, *J =* 3.0 Hz, 1H), 7.49 (d, *J =* 3.1 Hz, 1H), 6.44 (s, 2H), 4.03 (t, *J* = 6.0 Hz, 2H), 3.09 (s, 3H), 2.65 (t, *J* = 7.2 Hz, 2H), 2.51 (s, 3H), 1.99 (tt, *J =* 7.2, 6.0 Hz, 2H).

MS *m*/*z* [M+H]⁺: 269.

### Step 3: Synthesis of 6-chloro-4-((5-(3-cyanopropoxy)-3-(N,S-dimethylsulfoximino)pyridin-2-yl)amino)- N-(methyl-d3)pyridazine-3-carboxamide (19c)

The synthesis referenced the synthesis of 6-chloro-4-((3-(*N,S-*dimethylsulfoximino)pyridin-2-yl)amino)-*N*-(methyl-*d3*)pyridazine-3-carboxamide, yield 49%.

1H NMR (400 MHz, CDCl₃) δ 12.23 (s, 1H), 8.84 (s, 1H), 8.27 (s, 1H), 8.22 (s, 1H), 7.89 (s, 1H), 4.21 (t, *J =* 5.7 Hz, 2H), 3.23 (s, 3H), 2.84 (s, 3H), 2.64 (t, *J =* 6.9 Hz, 2H), 2.26 - 2.17 (m, 2H).

MS *m*/*z* [M+H]⁺: 441.

### Step 4: Synthesis of 4-((5-(3-cyanopropoxy)-3-(N,S-dimethylsulfoximino)pyridin-2-yl)amino)-6-(cyclopropylcarboxamido)-N-(methyl-d3)pyridazine-3-carboxamide (19)

The synthesis referenced the synthesis of 6-(cyclopropylcarboxamido)-4-((3-(*N,S-*dimethylsulfoximino)pyridin-2-yl)amino)-*N*-(methyl-*d3*)pyridazine-3-carboxamide, yield 28%.

1H NMR (400 MHz, DMSO-d6) δ 11.67 (s, 1H), 11.33 (s, 1H), 9.19 (s, 1H), 9.04 (s, 1H), 8.31 (d, *J* = 2.9 Hz, 1H), 7.77 (d, *J =* 2.9 Hz, 1H), 4.24 - 4.14 (m, 2H), 3.21 (s, 3H), 2.69 (t, *J =* 7.1 Hz, 2H), 2.66 (s, 3H), 2.13 - 2.00 (m, 3H), 0.88 - 0.79 (m, 4H).

MS *m*/*z* [M+H]⁺: 490.

### Example 20

### Preparation of 6-(cyclopropylcarboxamido)-4-((3-(N,S-dimethylsulfoximino)-5-((2-methoxyethyl)amino)pyridin-2-yl)amino)-N-methylpyridazine-3-carboxamide

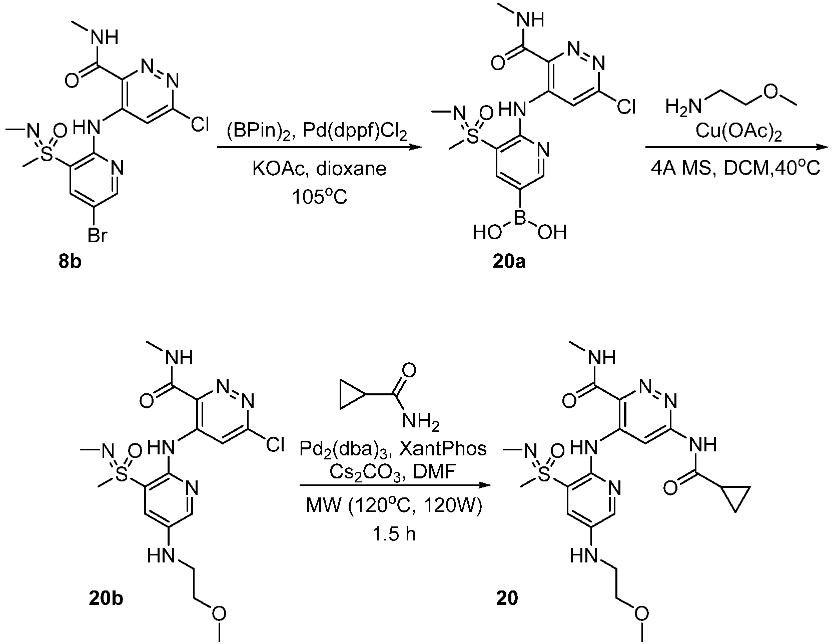

### Step 1: Synthesis of (6-((6-chloro-3-(methylcarbamoyl)pyridazin-4-yl)amino)-5-(N,S-dimethylsulfoximino)pyridin-3-yl)boronic acid (20a)

4-((5-bromo-3-(*N,S*-dimethylsulfoximino)pyridin-2-yl)amino)-6-chloro-*N-*methylpyridazine-3-carboxamide (8b) (140 mg, 0.324 mmol), bis(pinacolato)diboron (107 mg, 0.421 mmol), Pd(dppf)Cl₂ (24 mg, 0.032 mmol) and anhydrous potassium acetate (92 mg, 0.972 mmol) were dissolved in dioxane (4 mL), and stirred at 105°C for 1 hour under nitrogen atmosphere. After cooled to room temperature, 30% aqueous hydrogen peroxide (103 mg, 0.909 mmol) was added to the reaction solution, and stirred at room temperature for 30 min. The reaction solution was diluted with water, extracted with dichloromethane, and the organic phases were combined, washed with saturated brine and dried over anhydrous sodium sulfate. (6-((6-chloro-3-(methylcarbamoyl)pyridazin-4-yl)amino)-5-(*N,S*-dimethylsulfoximino) pyridin-3-yl)boronic acid (20a) (108 mg, gray solid) was obtained after being concentrated and beated with ethyl acetate, yield 83%.

MS *m*/*z* [M+H]⁺: 399.

### Step 2: Synthesis of 6-chloro-4-((3-(N,S-dimethylsulfoximino)-5-((2-methoxyethyl)amino)pyridin-2-yl)amino)-N-methylpyridazine-3-carboxamide (20b)

Copper acetate monohydrate (10 mg, 0.063 mmol), 4A molecular sieve (20 mg) and 2-methoxyethylamine (19 mg, 0.252 mmol) were added in sequence to (6-((6-chloro-3-(methylcarbamoyl)pyridazin-4-yl)amino)-5-(*N,S-*dimethylsulfoximino)pyridin-3-yl)boronic acid (20a) (50 mg, 0.126 mmol) in dichloromethane (2 mL), and stirred overnight at 40°C under oxygen atmosphere. The reaction solution was concentrated and separated by plate chromatography (methanol: dichloromethane = 1:10) to obtain 6-chloro-4-((3-(*N,S*-dimethylsulfoximino)-5-((2-methoxyethyl)amino)pyridin-2-yl)amino)-N-methylpyridazine-3-carboxamide (20b) (5 mg, yellow solid), yield 9%.

MS *m*/*z* [M+H]⁺: 428.

### Step 3: Synthesis of 6-(cyclopropylcarboxamido)-4-((3-(N,S-dimethylsulfoximino)-5-((2-methoxyethyl)amino)pyridin-2-yl)amino)-N-methylpyridazine-3-carboxamide (20)

The synthesis referenced the synthesis of 6-(cyclopropylcarboxamido)-4-((3-(*N,S-*dimethylsulfoximino)pyridin-2-yl)amino)-*N*-(methyl-d3)pyridazine-3-carboxamide, yield 45%.

1H NMR (400 MHz, DMSO-d6) δ 11.37 (s, 1H), 11.23 (s, 1H), 9.00 (s, 1H), 8.92 (s, 1H), 7.98 (d, *J* = 2.9 Hz, 1H), 7.49 (d, *J* = 2.9 Hz, 1H), 6.29 (s, 1H), 3.50 (t, *J* = 5.3 Hz, 2H), 3.30 - 3.27 (m, 5H), 3.11 (s, 3H), 2.83 (d, *J=* 4.8 Hz, 3H), 2.64 (s, 3H), 2.10 - 2.05 (m, 1H), 0.84 - 0.77 (m, 4H).

MS *m*/*z* [M+H]⁺: 477.

### Example 21

### Preparation of 4-((5-bromo-3-(N,S-dimethylsulfoximino)-6-methylpyridin-2-yl)amino)-6-(cyclopropylcarboxamido)-N-methylpyridazine-3-carboxamide

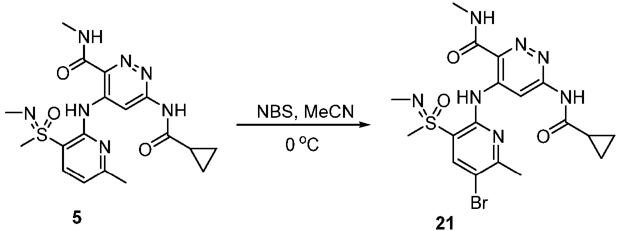

6-(cyclopropylcarboxamido)-4-((3-(*N,S*-dimethylsulfoximino)-6-methylpyridin-2-yl)amino)-N-methylpyridazine-3-carboxamide (5) (10 mg, 0.024 mmol) was dissolved in acetonitrile (0.5 mL), and NBS (4.3 mg, 0.024 mmol) in acetonitrile (0.5 mL)was added at 0°C, and stirred at 0°C for 1 hour. The reaction was quenched with water, extracted with ethyl acetate, washed with saturated brine and dried over anhydrous sodium sulfate. The obtained was concentrated and prepared to obtain 21 (6 mg, yellow solid), yield 50%.

1H NMR (400 MHz, DMSO-d6) δ 11.95 (s, 1H), 11.41 (s, 1H), 9.84 (s, 1H), 9.12 (d, *J =* 4.9 Hz, 1H), 8.17 (s, 1H), 3.26 (s, 3H), 2.84 (d, *J =* 4.7 Hz, 3H), 2.64 (s, 3H), 2.63 (s, 3H), 2.15 - 2.07 (m, 1H), 0.86 (d, *J =* 6.3 Hz, 4H).

MS *m*/*z* [M+H]⁺: 496.

### Example 22

### Preparation of 4-((6-amino-3-(N,S-dimethylsulfoximino)-5-fluoropyridin-2-yl)amino)-6-(cyclopropylcarboxamido)-N-methylpyridazine-3-carboxamide

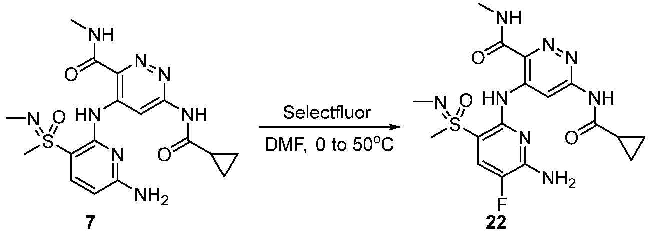

4-((6-Amino-3-(*N,S*-dimethylsulfoximino)pyridin-2-yl)amino)-6-(cyclopropylcarboxamido)-*N*-methylpyridazine-3-carboxamide (7) (168 mg, 0.402 mmol) was dissolved in DMF (4 mL), and Selectfluor (284 mg, 0.802 mmol) was added at 0°C, stirred at 35°C for 1 hour and 50°C for 1 hour. The DMF was evaporated to dryness under reduced pressure, separated by plate chromatography (methanol: dichloromethane = 1:15) and prepared by prep-HPLC to obtain 4-((6-amino-3-(N,S-dimethylsulfoximino)-5-fluoropyridin-2-yl)amino)-6-(cyclopropylcarboxamido)-*N-*methylpyridazin-3-carboxamide (22) (12 mg, white solid), yield 7%.

1H NMR (400 MHz, DMSO-d6) δ 11.48 (s, 1H), 11.32 (s, 1H), 9.22 (s, 1H), 9.03 (d, *J =* 4.8 Hz, 1H), 7.60 (d, *J =* 10.3 Hz, 1H), 6.95 (s, 2H), 3.09 (s, 3H), 2.82 (d, *J* = 4.7 Hz, 3H), 2.60 (s, 3H), 2.13 - 2.05 (m, 1H), 0.84 (d, *J =* 6.5 Hz, 4H).

MS *m*/*z* [M+H]⁺: 437.

### Example 23

### Preparation of 4-(6-amino-5-chloro-3-(N,S-dimethylsulfoximino)pyridin-2-yl)amino)-6-(cyclopropylcarboxamido)-N-methylpyridazine-3-carboxamide

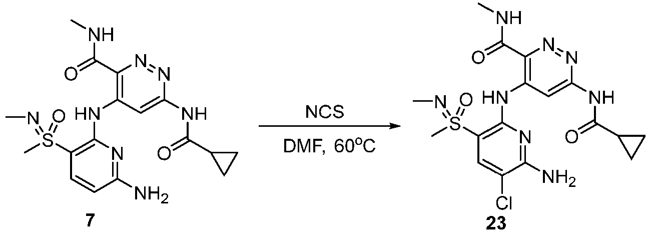

4-((6-Amino-3-(*N,S*-dimethylsulfoximino)pyridin-2-yl)amino)-6-(cyclopropylcarboxamido)-*N*-methylpyridazine-3-carboxamide (7) (9 mg, 0.022 mmol) was dissolved in anhydrous DMF (1 mL), and NCS (3.5 mg, 0.024 mmol) was added in an ice bath, after the addition, the reaction was carried out at 60°C for 3 hours. The reaction solution was cooled to room temperature, quenched by saturated sodium bicarbonate (20 mL) and water (20 mL), extracted with ethyl acetate, washed with saturated brine and dried over anhydrous sodium sulfate. The obtained was concentrated and prepared by prep-HPLC to obtain 4-(6-amino-5-chloro-3-(N,S-dimethylsulfoximino)pyridin-2-yl)amino)-6-(cyclopropylcarboxamido)-*N-*methylpyridazine-3-carboxamide (23) (4 mg, white solid), yield 41%.

1H NMR (400 MHz, DMSO-d6) δ 11.59 (s, 1H), 11.36 (s, 1H), 9.41 (s, 1H), 9.06 (d, *J =* 4.9 Hz, 1H), 7.78 (s, 1H), 6.88 (s, 2H), 3.12 (s, 3H), 2.82 (d, *J =* 4.7 Hz, 3H), 2.60 (s, 3H), 2.17 - 2.02 (m, 1H), 0.85 (d, *J =* 6.9 Hz, 4H).

MS *m*/*z* [M+H]⁺: 453.

### Example 24

### Preparation of 4-((6-amino-5-bromo-3-(N,S-dimethylsulfoximino)pyridin-2-yl)amino)-6-(cyclopropylcarboxamido)-N-methylpyridazine-3-carboxamide

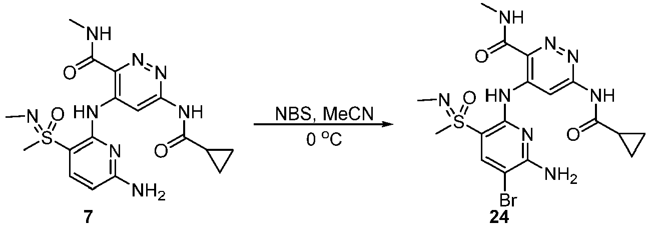

4-((6-Amino-3-(*N,S*-dimethylsulfoximino)pyridin-2-yl)amino)-6-(cyclopropylcarboxamido)-*N*-methylpyridazine-3-carboxamide (7) (15 mg, 0.0359 mmol) was dissolved in dichloromethane (1 mL), and NBS (6.3 mg, 0.0359 mmol) was added at 0°C, and stirred for 3 hours at 0°C. After being concentrated and Flash separated to obtain (methanol/dichloromethane = 0 - 5.5%), 4-((6-amino-5-bromo-3-(*N,S*-dimethylsulfoximino)pyridin-2-yl)amino)-6-(cyclopropylcarboxamido)-*N-*methylpyridazine-3-carboxamide (24) (15 mg, yellow solid) was obtained, yield 44%.

1H NMR (400 MHz, DMSO-d6) δ 11.60 (s, 1H), 11.35 (s, 1H), 9.47 (s, 1H), 9.05 (d, *J =* 5.0 Hz, 1H), 7.90 (s, 1H), 6.77 (s, 2H), 3.13 (s, 3H), 2.82 (d, *J =* 4.8 Hz, 3H), 2.61 (s, 3H), 2.10 (q, *J* = 6.2 Hz, 1H), 0.86 (d, *J* = 6.6 Hz, 4H).

MS *m*/*z* [M+H]⁺: 497.

### Example 25

### Preparation of 4-(6-amino-5-chloro-3-(N,S-dimethylsulfoximino)pyridin-2-yl)amino)-6-(cyclopropylcarboxamide)-N-(methyl-d3)pyridazine-3-carboxamide

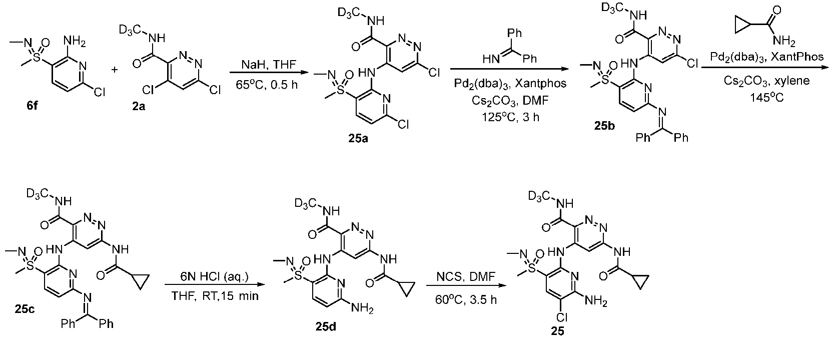

### Step 1: Synthesis of 6-chloro-4-((6-chloro-3-(N,S-dimethylsulfoximino)pyridin-2-yl)amino)-N-(methyl-d3)pyridazine-3-carboxamide (25a)

Sodium hydride (2.7 g, 67.1 mmol) was added in batches to 6-chloro-3-(N,S-dimethylsulfoximino)pyridin-2-amine (6f) (4.9 g, 22.37 mmol) in tetrahydrofuran (120 mL), and stirred for 15 min, 4,6-dichloro-*N*-(methyl-*d3*)pyridazine-3-carboxamide (2a) (5.6 g, 26.8 mmol) was added, after the addition, and stirred at 65°C for 1 hour. The reaction was quenched by ammonium chloride (80 mL) and water (40 mL), extracted with dichloromethane (300 mL), washed with saturated brine (50 mL), and dried over anhydrous sodium sulfate. The obtained was concentrated, the crude product was beated with methyl tert-butyl ether, suction filtered, the filter cake was washed with methyl tert-butyl ether, and dried to obtain 6-chloro-4-((6-chloro-3-(N,S-dimethylsulfoximino)pyridin-2-yl)amino)-*N*-(methyl-*d3*)pyridazine-3-carboxamide (25a) (6.8 g, earthy brown solid), yield 77%.

1H NMR (400 MHz, DMSO-d6) δ 12.22 (s, 1H), 9.34 (s, 1H), 8.78 (s, 1H), 8.22 (d, *J* = 8.2 Hz, 1H), 7.42 (d, *J* = 8.1 Hz, 1H), 3.24 (s, 3H), 2.68 (s, 3H).

MS *m*/*z* [M+H]⁺: 393.

### Step 2: Synthesis of 6-chloro-4-((3-(N,S-dimethylsulfoximino)-6-((diphenylmethylene)amino)pyridin-2-yl)amino)-N-(methyl-d3)pyridazine-3-carboxamide (25b)

The synthesis referenced the synthesis of 6-chloro-4-((3-(*N,S-*dimethylsulfoximino)-6-((diphenylmethylene)amino)pyridin-2-yl)amino)*-N-*methylpyridazine-3-carboxamide, yield 82%.

¹H NMR (400 MHz, DMSO-d6) δ 11.95 (s, 1H), 9.24 (s, 1H), 8.47 (s, 1H), 8.05 (d, *J* = 8.3 Hz, 1H), 7.41 (s, 10H), 6.79 (d, *J* = 8.3 Hz, 1H), 3.16 (s, 3H), 2.62 (s, 3H).

MS *m*/*z* [M+H]⁺: 538.

### Step 3: Synthesis of 6-(cyclopropylcarboxamido)-4-((3-(N,S-dimethylsulfoximino)-6-((diphenylmethylene)amino)pyridin-2-yl)amino)-N-(methyl-d3)pyridazine-3-carboxamide (25c)

The synthesis referenced the synthesis of 6-(cyclopropylcarboxamido)-4-((3-(*N,S-*dimethylsulfoximino)-6-((diphenylmethylene)amino)pyridin-2-yl)amino)-*N-*methylpyridazine-3-carboxamide, the crude product was not separated and directly used in the next step of the reaction.

MS *m*/*z* [M+H]⁺: 586.

### Step 4: Synthesis of 4-((6-amino-3-(N,S-dimethylsulfoximino)pyridin-2-yl)amino)-6-(cyclopropylcarboxamido)-N-(methyl-d3)pyridazine-3-carboxamide (25d)

The synthesis referenced the synthesis of 4-((6-amino-3-(*N,S-*dimethylsulfoximino)pyridin-2-yl)amino)-6-(cyclopropylcarboxamido)*-N-*methylpyridazine-3-carboxamide, two-step yield 63%.

¹H NMR (400 MHz, DMSO-d6) δ 11.55 (s, 1H), 11.32 (s, 1H), 9.49 (s, 1H), 8.99 (s, 1H), 7.73 (d, *J =* 8.5 Hz, 1H), 6.56 (s, 2H), 6.23 (d, *J =* 8.7 Hz, 1H), 3.05 (s, 3H), 2.61 (s, 3H), 2.09 (s, 1H), 0.85 (d, *J =* 6.7 Hz, 4H).

MS *m*/*z* [M+H]⁺: 422.

### Step 5: Synthesis of 4-(6-amino-5-chloro-3-(N,S-dimethylsulfoximino)pyridin-2-yl)amino)-6-(cyclopropylcarboxamido)-N-(methyl-d3)pyridazine-3-carboxamide (25)

The synthesis referenced the synthesis of 4-(6-amino-5-chloro-3-(*N,S-*dimethylsulfoximino)pyridin-2-yl)amino)-6-(cyclopropylcarboxamido)-N-methylpyridazine-3-carboxamide, yield 50%.

1H NMR (400 MHz, DMSO-d6) δ 11.61 (s, 1H), 11.37 (s, 1H), 9.43 (s, 1H), 9.04 (s, 1H), 7.79 (s, 1H), 6.89 (s, 2H), 3.13 (s, 3H), 2.61 (s, 3H), 2.15 - 2.08 (m, 1H), 0.86 (d, *J* = 7.1 Hz, 4H).

MS *m*/*z* [M+H]⁺: 456.

### Example 26

### Preparation of 4 -((6-amino-3-(N,S-dimethylsulfoximino)-5-methylpyridin-2-yl)amino)-6-(cyclopropylcarboxamido)-N-methylpyridazine-3-carboxamide

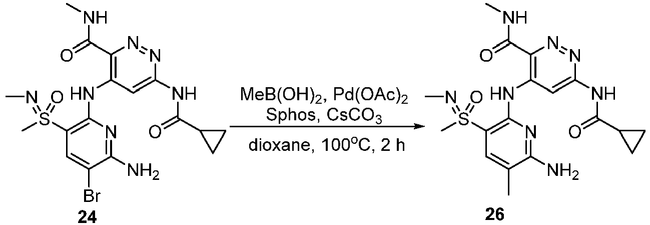

4-((6-Amino-5-bromo-3-(*N*,*S*-dimethylsulfoximino)pyridin-2-yl)amino)-6-(cyclopropylcarboxamido)-*N*-methylpyridazine-3-carboxamide (24) (160 mg, 0.322 mmol) was dissolved in dioxane (4 mL), tetrakis(triphenylphosphine)palladium (110 mg, 0.096 mmol), anhydrous potassium carbonate (230 mg, 1.66 mmol) and methylboronic acid (496 mg, 1.66 mmol) were added, and the reaction was carried out in microwave (120°C, 120W) under nitrogen atmosphere for 1 hour. After the reaction solution was cooled to room temperature, dichloromethane was added, stirred and then filtered, the filter cake was washed with dichloromethane, and the filtrate was concentrated and Flash separated (methanol/dichloromethane = 1:20) to obtain 4 -((6-amino-3-(*N*,*S*-dimethylsulfoximino)-5-methylpyridin-2-yl)amino)-6-(cyclopropylcarboxamido)-*N*-methylpyridazin-3-carboxamide (26) ( 89 mg, yellow solid), yield 64%.

1H NMR (400 MHz, DMSO-d6) δ 11.51 (s, 1H), 11.30 (s, 1H), 9.51 (s, 1H), 9.00 (d, *J =* 4.9 Hz, 1H), 7.58 (s, 1H), 6.36 (s, 2H), 3.04 (s, 3H), 2.82 (d, J = 4.6 Hz, 3H), 2.60 (s, 3H), 2.09 (dd, *J =* 11.3, 5.5 Hz, 1H), 2.06 (s, 3H), 0.85 (d, *J =* 7.0 Hz, 4H).

MS *m*/*z* [M+H]⁺: 433.

### Example 27

### Preparation of 4-((6-amino-3-(N,S-dimethylsulfoximino)-5-methoxypyridin-2-yl)amino)-6-(cyclopropylcarboxamido)-N-methylpyridazine-3-carboxamide

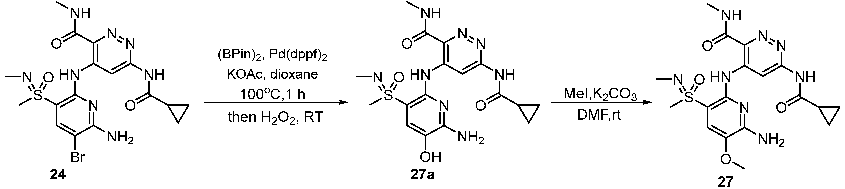

### Step 1: Synthesis of 4-((6-amino-3-(N,S-dimethylsulfoximino)-5-hydroxypyridin-2-yl)amino)-6-(cyclopropylcarboxamido)-N-methylpyridazine-3-carboxamide (27a)

Bis(pinacolato)diboron (77 mg, 0.30 mmol, 1.5 eq), potassium acetate (49 mg, 0.50 mmol) and Pd(PPh)₂Cl₂ (28 mg, 0.04 mmol) were added to a suspension of 4-((6-amino-5-bromo-3-(*N,S*-dimethylsulfoximino)pyridin-2-yl)amino)-6-(cyclopropylcarboxamido)-N-methylpyridazine-3-carboxamide (24) (100 mg, 0.20 mmol) in toluene (3 mL), and the reaction was carried out at 100°C under nitrogen for 1 hour, followed by addition of Pd(PPh)₂Cl₂ (28 mg, 0.04 mmol, 0.2 eq) and continued to react for 1 hour. The reaction solution was cooled to room temperature, tetrahydrofuran (3 mL) and hydrogen peroxide (0.10 mL, 0.88 mmol) were added, and the reaction solution was stirred at room temperature for 30 min. The reaction was quenched with water (20 mL), diluted with dichloromethane, suction filtered and the filter cake was washed with dichloromethane. The filtrate was separated by Flash (methanol/dichloromethane = 1:10) to obtain 4-((6-amino-3-(*N*,*S-*dimethylsulfoximino)-5-hydroxypyridin-2-yl)amino)-6-(cyclopropylcarboxamido)-*N-*methylpyridazine-3-carboxamide (27a) (30 mg, brownish yellow solid), yield 28%.

¹H NMR (400 MHz, DMSO-d6) δ 11.27 (s, 1H), 11.23 (s, 1H), 9.88 (s, 1H), 9.12 (s, 1H), 8.99 (d, *J =* 4.7 Hz, 1H), 7.24 (s, 1H), 6.29 (s, 2H), 3.01 (s, 3H), 2.81 (d, *J* = 4.6 Hz, 3H), 2.59 (s, 3H), 2.12 - 2.04 (m, 1H), 0.83 (d, *J =* 7.2 Hz, 4H).

MS *m*/*z* [M+H]⁺: 435.

### Step 2: Synthesis of 4-((6-amino-3-(N,S-dimethylsulfoximino)-5-methoxypyridin-2-yl)amino)-6-(cyclopropylcarboxamido)-N-methylpyridazine-3-carboxamide (27)

4-((6-Amino-3-(*N,S*-dimethylsulfoximino)-5-hydroxypyridin-2-yl)amino)-6-(cyclopropylcarboxamido)-*N*-methylpyridazine-3-carboxamide (27a) (10 mg, 0.023 mmol) was dissolved in DMF (0.6 mL), potassium carbonate (5 mg, 0.034 mmol) and methyl iodide (3.3 mg 0.023 mmol) were added, stirred at room temperature for 20 minutes. The reaction solution was quenched with water (5 mL), extracted with ethyl acetate, the organic phases were combined, washed with saturated brine, and dried over anhydrous sodium sulfate. The obtained was concentrated and prepared by prep-HPLC to obtain 4-((6-amino-3-(*N,S*-dimethylsulfoximino)-5-methoxypyridin-2-yl)amino)-6-(cyclopropylcarboxamido)-*N*-methylpyridazine-3-carboxamide (27) (2.2 mg, white solid), yield 22%.

¹H NMR (400 MHz, DMSO-d6) δ 11.35 (s, 1H), 11.26 (s, 1H), 9.13 (s, 1H), 9.00 (d, *J =* 4.8 Hz, 1H), 7.27 (s, 1H), 6.54 (s, 2H), 3.81 (s, 3H), 3.05 (s, 3H), 2.82 (d, *J* = 4.5 Hz, 3H), 2.60 (s, 3H), 2.12 - 2.04 (m, 1H), 0.83 (d, *J =* 6.6 Hz, 4H).

MS *m*/*z* [M+H]⁺: 449.

### Example 28

### Preparation of (R)-4-((6-amino-5-chloro-3-(N,S-dimethylsulfoximino)pyridin-2-yl)amino)-6-(cyclopropylcarbonyl)-N-(methyl-d3)pyridazine-3-carboxamide

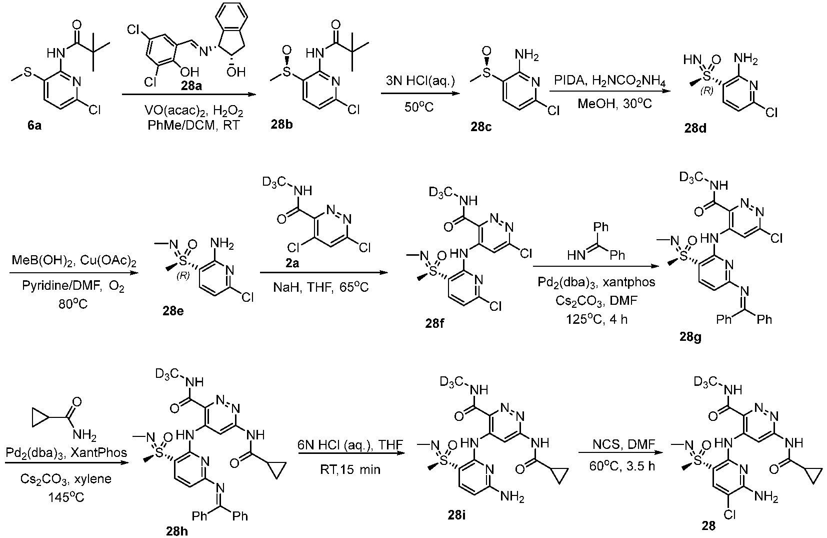

### Step 1: Synthesis of (R)-N-(6-chloro-3-(methylsulfinyl)pyridin-2-yl)trimethylacetamide (28b)

The synthesis of *(1R,2S)*-1-(3,5-dichloro-2-hydroxybenzylidene)amino-2-indanol (28a) referenced Comptes Rendus Chimie, 2014, 17(5), 403 - 412.

*(1R,2S)*-1-Amino-2-indanol (61.5 g, 0.413 mmol) and 3,5-dichlorosalicylaldehyde (78.8 g, 0.413 mmol) were dissolved in anhydrous methanol (1.5 L), and stirred at 70°C for 1 hour. Methanol was removed under reduced pressure, beated with petroleum ether, suction filtered and dried to obtain *(1R,2S)*-1-(3,5-dichloro-2-hydroxybenzylidene)amino-2-indanol (28a) (131 g, yellow solid), yield 99%.

1H NMR (400 MHz, DMSO-*d6*) δ 14.79 (s, 1H), 8.70 (s, 1H), 7.56 (t, *J* = 2.0 Hz, 1H), 7.48 (t, *J =* 1.9 Hz, 1H), 7.31 (q, *J =* 7.7, 7.3 Hz, 2H), 7.24 (d, *J =* 5.8 Hz, 2H), 5.55 (d, *J =* 4.8 Hz, 1H), 5.06 (d, *J =* 5.3 Hz, 1H), 4.60 (t, *J =* 4.6 Hz, 1H), 3.15 (dd, *J* = 16.1, 5.7 Hz, 1H), 2.91 (dd, *J =* 16.1, 4.0 Hz, 1H).

MS *m*/*z* [M+H]⁺: 322.

*(1R,2S)*-1-(3,5-dichloro-2-hydroxybenzylidene)amino-2-indanol (28a) (4.62 g, 14.4 mmol) and alum acetylacetonate oxide (2.38 g, 9.0 mmol) were added to a solvent mixture of toluene and dichloromethane (10:1, 930 mL), and stirred for 20 min until completely dissolved. *N*-(6-chloro-3-(methylthio)pyridin-2-yl)trimethylacetamide (6a) (46.4 g, 179.8 mmol) was added and stirred for 20 min. Hydrogen peroxide (26.5 g, 234.0 mmol) was added dropwise in an ice bath, after the addition, and stirred at room temperature for 2 h. Hydrogen peroxide (3.6 mL, 36.0 mmol) was added and the reaction was continued for 1 hour. The reaction was quenched with saturated sodium sulfite solution (150 mL), extracted with ethyl acetate, the organic phases were combined, washed with saturated brine and dried over anhydrous sodium sulfate. The obtained was concentrated and beated with a mixture of isopropanol (40 ml) and petroleum ether (280 mL), suction filtered, and the filtrate was separated by Flash (ethyl acetate/petroleum ether = 1:1) to obtain (R)-*N*-(6-chloro-3-(methylsulfinyl)pyridin-2-yl)trimethylacetamide (28b) (51 g, light yellow solid), with an ee value of 96.5% and a yield of 72%.

1H NMR (400 MHz, CDCl₃) δ 8.59 (s, 1H), 8.11 (d, *J* = 8.2 Hz, 1H), 7.27 (d, *J* = 8.2 Hz, 1H), 2.94 (s, 3H), 1.26 (s, 9H).

MS *m*/*z* [M+H]⁺: 275.

### Step 2: Synthesis of (R)-6-chloro-3-(methylsulfinyl)-pyridin-2-amine (28c)

(*R*)-*N*-(6- chloro-3-(methylsulfinyl)pyridin-2-yl)trimethylacetamide (28b) (50.2 g, 0.183 mol) was added to an aqueous 3 N hydrochloric acid solution (610 mL, 1.832 mol), and stirred at 50°C for 5 hours. In an ice bath, pH = 9 ~ 10 was adjusted with aqueous potassium carbonate (40%), the aqueous phase was extracted with ethyl acetate, the organic phases were combined, washed with saturated brine and dried over anhydrous sodium sulfate. The obtained was concentrated and beated with a mixture of dichloromethane/petroleum ether (2:3, 100 mL), suction filtered, the filter cake was washed with petroleum ether to obtain (R)-6-chloro-3-(methylsulfinyl)-pyridin-2-amine (28c) (28.7 g, white solid), with an ee value of 98.2% and a yield of 80%.

1H NMR (400 MHz, CDCl₃) δ 7.42 (d, *J =* 7.8 Hz, 1H), 6.69 (d, *J =* 7.8 Hz, 1H), 6.07 (s, 2H), 2.89 (s, 3H).

MS *m*/*z* [M+H]⁺: 191.

### Step 3: Synthesis of (R)-6-chloro-3-(S-methylsulfoximino)pyridin-2-amine (28d)

The synthesis referenced the synthesis of 3-(*S*-methylsulfoximino)-2-nitropyridine, yield 70%.

1H NMR (400 MHz, CDCl₃) δ 7.98 (d, *J* = 8.1 Hz, 1H), 6.76 (d, *J* = 8.1 Hz, 1H), 6.35 (s, 2H), 3.11 (s, 3H), 2.46 (s, 1H).

MS *m*/*z* [M+H]⁺: 206.

### Step 4: Synthesis of (R)-6-chloro-3-(N,S-dimethylsulfoximino)pyridin-2-amine (28e)

(*R*)-6-chloro-3-(S-methylsulfoximino)pyridin-2-amine (28d) (11.63 g, 56.7 mmol), methylboronic acid (8.16 g, 136.1 mmol) and copper acetate (15.5 g, 85 mmol) were dissolved in a mixture of anhydrous DMF (69 mL) and pyridine (46 mL), and the reaction was carried out under oxygen atmosphere at 80°C for 4 hours.

The reaction solution was cooled to room temperature, suction filtered, and the filter cake was washed with ethyl acetate, the filtrate was added with water (200 mL) and ammonia (60 mL), extracted with ethyl acetate, and the organic phases were combined, washed with saturated brine and dried over anhydrous sodium sulfate. The obtained was concentrated and separated by column chromatography (ethyl acetate/petroleum ether = 40 - 50%) to obtain (*R*)-6-chloro-3-(*N*,*S-*dimethylsulfoximino)pyridin-2- amine (28e) (8.69 g, light yellow solid), ee value 98.2%, yield 70%.

1H NMR (400 MHz, CDCl₃) δ 7.91 (d, *J* = 8.0 Hz, 1H), 6.78 (d, *J* = 8.1 Hz, 1H), 6.26 (s, 2H), 3.07 (s, 3H), 2.68 (s, 3H).

MS *m*/*z* [M+H]⁺: 220.

### Step 5: Synthesis of (R)-6-chloro-4-((6-chloro-3-(N,S-dimethylsulfoximino)pyridin-2-yl)amino)-N-(methyl-d3)pyridazine-3-carboxamide (28f)

The synthesis referenced the synthesis of 6-chloro-4-((6-chloro-3-(*N*,*S-*dimethylsulfoximino)pyridin-2-yl)amino)-*N*-(methyl-*d3*)pyridazine-3-carboxamide, yield 79%.

1H NMR (400 MHz, DMSO-d6) δ 12.21 (s, 1H), 9.32 (s, 1H), 8.78 (s, 1H), 8.22 (d, *J=* 8.1 Hz, 1H), 7.42 (d, *J* = 8.1 Hz, 1H), 3.24 (s, 3H), 2.68 (s, 3H).

MS *m*/*z* [M+H]⁺: 393.

### Step 6: Synthesis of (R)-6-chloro-4-((3-(N,S-dimethylsulfoximino)-6-((diphenylmethylene)amino)pyridin-2-yl)amino)-N-(methyl-d3)pyridazine-3-carboxamide (28g)

The synthesis referenced the synthesis of 6-chloro-4-((3-(*N*,*S-*dimethylsulfoximino)-6-((diphenylmethylene)amino)pyridin-2-yl)amino)-*N-*methylpyridazine-3-carboxamide, yield 68%.

MS *m*/*z* [M+H]⁺: 538.

### Step 7: Synthesis of (R)-6-(cyclopropylcarboxamido)-4-((3-(N,S-dimethylsulfoximino)-6-((diphenylmethylene)amino)pyridin-2-yl)amino)-N-(methyl-d3)pyridazine-3-carboxamide (28h)

The synthesis referenced the synthesis of 6-(cyclopropylcarboxamido)-4-((3-(*N,S-*dimethylsulfoximino)-6-((diphenylmethylene)amino)pyridin-2-yl)amino)-*N-*methylpyridazine-3-carboxamide, yield 91%.

MS *m*/*z* [M+H]⁺: 586.

### Step 8: Synthesis of (R)-4-((6-amino-3-(N,S-dimethylsulfoximino)pyridin-2-yl)amino)-6-(cyclopropylcarboxamido)-N-(methyl-d3)pyridazine-3-carboxamide (28i)

The synthesis referenced the synthesis of 4-((6-amino-3-(*N*,*S-*dimethylsulfoximino)pyridin-2-yl)amino)-6-(cyclopropylcarboxamido)-*N-*methylpyridazine-3-carboxamide, yield 69%.

1H NMR (400 MHz, DMSO-d6) δ 11.55 (s, 1H), 11.30 (s, 1H), 9.50 (s, 1H), 8.98 (s, 1H), 7.73 (d, *J =* 8.6 Hz, 1H), 6.54 (s, 2H), 6.23 (d, *J* = 8.6 Hz, 1H), 3.05 (s, 3H), 2.61 (s, 3H), 2.14 - 2.05 (m, 1H), 0.90 - 0.82 (m, 4H).

MS *m*/*z* [M+H]⁺: 422.

### Step 9: Synthesis of (R)-4-((6-amino-5-chloro-3-(N,S-dimethylsulfoximino)pyridin-2-yl)amino)-6-(cyclopropylformyl)-N-(methyl-d3)pyridazine-3-carboxamide (28)

The synthesis referenced the synthesis of 4-(6-amino-5-chloro-3-(*N*,*S-*dimethylsulfoximino)pyridin-2-yl)amino)-6-(cyclopropylformamido)-*N-*methylpyridazine-3-carboxamide with an ee value of 98.2% and a yield of 50%.

1H NMR (400 MHz, DMSO-d6) δ 11.59 (s, 1H), 11.34 (s, 1H), 9.43 (s, 1H), 9.01 (s, 1H), 7.78 (s, 1H), 6.86 (s, 2H), 3.12 (s, 3H), 2.61 (s, 3H), 2.15 - 2.06 (m, 1H), 0.85 (d, *J =* 6.1 Hz, 4H).

MS *m*/*z* [M+H]⁺: 456.

**The asymmetric synthesis of the chiral fragment (*R*)-6-chloro-3-(methylsulfinyl)-pyridin-2-amine (28c) can also be achieved by the following route:**

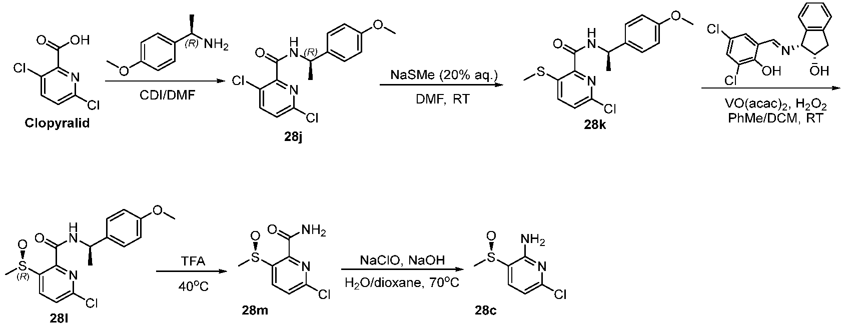

### Step 1: Synthesis of (R)-3,6-dichloro-N-(1-(4-methoxyphenyl)ethyl)-2-pyridinecarboxamide (28j)

3, 6-Dichloro-2-pyridinecarboxylic acid (5 g, 26.04 mmol) was dissolved in anhydrous DMF (20 mL), and *N*,*N*-carbonyldiimidazole (4.64 g, 28.65 mmol) was added in batches, after the addition, and the solution was stirred at room temperature for 20 minutes. The reaction solution was cooled to 0°C, and (*R*)-(+)-1-(4-methoxyphenyl)ethylamine (4.33 g, 28.65 mmol) was added dropwise, the internal temperature was controlled to be less than 10°C, after the addition, and the solution was continued to be stirred at room temperature for 1.5 hours. The reaction was quenched with water (60 mL), continued to be stirred for 1 hour, suction filtered, and the filter cake was washed with distilled water. The filter cake was dissolved in dichloromethane, washed with 1% HCl, and dried over anhydrous sodium sulfate. The obtained was concentrated to obtain (*R*)-3,6-dichloro-*N*-(1-(4-methoxyphenyl)ethyl)-2-pyridinecarboxamide (28j) (7.77 g, white solid), yield 92%.

1H NMR (400 MHz, CDCl₃) δ 7.80 (d, *J =* 7.3 Hz, 1H), 7.76 (d, *J =* 8.4 Hz, 1H), 7.39 - 7.31 (m, 3H), 6.90 (d, *J* = 8.5 Hz, 2H), 5.28 - 5.20 (m, 1H), 3.81 (s, 3H), 1.61 (d, *J* = 6.9 Hz, 3H).

MS *m*/*z* [M+H]⁺: 325.

### Step 2: Synthesis of (R)-6-chloro-N-(1-(4-methoxyphenyl)ethyl)-3-(methylthio)-2-pyridinecarboxamide (28k)

(*R*)-3,6-dichloro-*N*-(1-(4-methoxyphenyl)ethyl)-2-pyridinecarboxamide (28j) (7.2 g, 22.15 mmol) was dissolved in DMF (35 mL), and 20% aqueous sodium methyl mercaptide (11.6 g, 33.23 mmol) was slowly added dropwise at room temperature, after the addition, and the solution was continued to be stirred at room temperature for 1 hour. The reaction was quenched with water (100 mL) and stirred for 2 h at room temperature, suction filtered and the filter cake was washed with distilled water. The filter cake was dissolved in dichloromethane, washed with saturated brine and dried over anhydrous sodium sulfate. The obtained was concentrated and separated by column chromatography (ethyl acetate: petroleum ether = 1:1) to obtain (*R*)-6-chloro-*N*-(1-(4-methoxyphenyl)ethyl)-3-(methylthio)-2-pyridinecarboxamide (28k) (4.84 g, white solid), yield 65%.

1H NMR (400 MHz, CDCl₃) δ 8.03 (d, *J =* 7.5 Hz, 1H), 7.58 (d, *J =* 8.4 Hz, 1H), 7.41 - 7.29 (m, 3H), 6.89 (d, *J =* 8.0 Hz, 2H), 5.31 - 5.19 (m, 1H), 3.80 (s, 3H), 2.40 (s, 3H), 1.60 (d, *J =* 7.6 Hz, 3H).

MS *m*/*z* [M+H]⁺: 337.

### Step 3: Synthesis of 6-chloro-N-((R)-1-(4-methoxyphenyl)ethyl)-3-((R)-methylsulfinyl)-2-pyridinecarboxamide (281)

The synthesis referenced the synthesis of (*R*)-*N*-(6-chloro-3-(methylsulfinyl)pyridin-2-yl)trimethylacetamide, ethanol was used for recrystallization in post-treatment, yield 64% and *d.e.* value 99.9%.

1H NMR (400 MHz, CDCl₃) δ 8.55 (d, *J* = 8.3 Hz, 1H), 7.97 (d, *J* = 8.0 Hz, 1H), 7.63 (d, *J* = 8.2 Hz, 1H), 7.24 (d, *J =* 8.5 Hz, 2H), 6.83 (d, *J =* 8.4 Hz, 2H), 5.17 - 5.07 (m, 1H), 3.73 (s, 3H), 2.87 (s, 3H), 1.55 (d, *J =* 6.9 Hz, 3H).

MS *m*/*z* [M+H]⁺: 353.

### Step 4: Synthesis of (R)-6-chloro-3-(methylsulfinyl)-2-pyridinecarboxamide (28m)

6-Chloro-N-((*R*)-1-(4-methoxyphenyl)ethyl)-3-((*R*)-methylsulfinyl)-2-pyridinecarboxamide (28l) (6.0 g, 17.05 mmol) was dissolved in trifluoroacetic acid (20 mL), and stirred at 40°C for 1.5 hours. The solvent was evaporated under reduced pressure and diluted with dichloromethane (50 mL). The mixture was slowly added dropwise to saturated sodium bicarbonate solution (100 mL), the aqueous phase was extracted with dichloromethane, the organic phases were combined, washed with saturated brine and dried over anhydrous sodium sulfate. The obtained was concentrated, beated with a mixture of dichloromethane/petroleum ether (1: 10, 100 mL) and suction filtered to obtain (*R*)-6-chloro-3-(methylsulfinyl)-2-pyridinecarboxamide (28l) (3.4 g, white solid), yield 92%.

1H NMR (400 MHz, CDCl₃) δ 8.65 (d, *J* = 8.3 Hz, 1H), 7.81 - 6.76 (m, 2H), 6.03 (s, 1H), 2.92 (s, 3H).

MS *m*/*z* [M+H]⁺: 219.

### Step 5: Synthesis of (R)-6-chloro-3-(methylsulfinyl)-pyridin-2-amine (28c)

(*R*)-6-chloro-3-(methylsulfinyl)-2-pyridinecarboxamide (28l) (55 g, 0.252 mol) was dissolved in dioxane (250 mL), 10% aqueous sodium hydroxide (250 mL) precooled to 0°C was added, 7.5% aqueous sodium hypochlorite (180 g, 0.378 mol) was added dropwise at room temperature, after the addition, and stirred for 1 hour at 70°C. The reaction solution was cooled to room temperature and quenched by dropwise addition of saturated sodium sulfite solution (10 mL) until the starch potassium iodide test paper did not change color, and diluted by adding saturated saline. The obtained was extracted with ethyl acetate, the organic phases were combined, washed with saturated brine and dried over anhydrous sodium sulfate. The obtained was concentrated and beated by adding dichloromethane (100 mL) and petroleum ether (100 mL), suction filtered and washed with petroleum ether, dried to obtain (*R*)-6-chloro-3-(methylsulfinyl)-pyridin-2-yl-amine (28c) (39.4 g, white solid), yield 82%.

1H NMR (400 MHz, CDCl₃) δ 7.42 (d, *J =* 7.8 Hz, 1H), 6.69 (d, *J =* 7.8 Hz, 1H), 6.07 (s, 2H), 2.89 (s, 3H).

MS *m*/*z* [M+H]⁺: 191.

### Example 29

### Preparation of (R)-4-((6-amino-5-chloro-3-(S-methylsulfoximino)pyridin-2-yl)amino)-6-(cyclopropylcarboxamido)-N-(methyl-d3)pyridazine-3-carboxamide

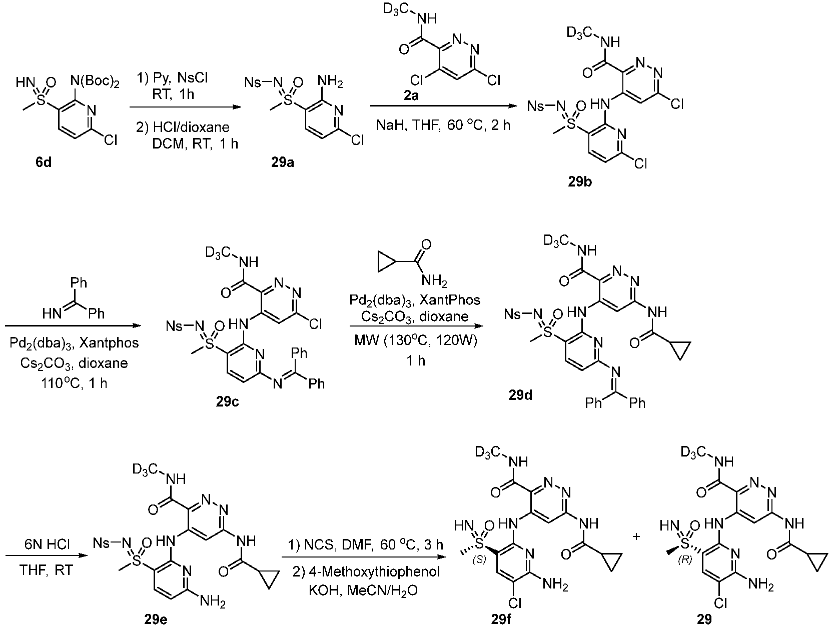

### Step 1: Synthesis of 6-chloro-3-(S-methyl-N-(4-nitrophenylsulfonyl)-sulfoximino)pyridin-2-amine (29a)

*N,N*-(di-tert-butoxycarbonyl)-6-chloro-3-(S-methylsulfoximino)pyridin-2-amine (6d) (2.05 g, 5.06 mmol) was dissolved in anhydrous pyridine (30 mL), and p-nitrobenzenesulfonyl chloride (1.34 g, 6.07 mmol) was added slowly at room temperature, and stirred for 2 hours. The reaction was quenched with water (60 mL), extracted with ethyl acetate, the organic phases were combined, washed with 1N hydrochloric acid and saturated brine, dried over anhydrous sodium sulfate, and concentrated to obtain the crude product (yellow solid, 3.3 g).

The crude product was dissolved in dichloromethane (10 mL), 4 M hydrogen chloride in dioxane (10 mL) was added in an ice-water bath, and stirred for 1 hour at room temperature. The obtained was concentrated and diluted with water, pH = 8~9 was adjusted with saturated potassium carbonate solution, and extracted with ethyl acetate. The organic phases were combined, dried over anhydrous sodium sulfate, concentrated and Flash separated (ethyl acetate: petroleum ether = 1:1) to obtain 6-chloro-3-(S-methyl-*N*-(4-nitrophenylsulfonyl)-sulfoximino)pyridin-2-amine (29a) (1.68 g, yellow solid), yield 85%.

1H NMR (400 MHz, DMSO-d6) δ 8.34 (d, *J =* 8.8 Hz, 2H), 8.00 (d, *J =* 8.8 Hz, 2H), 7.89 (d, *J =* 8.3 Hz, 1H), 7.28 (s, 2H), 6.82 (d, *J =* 8.3 Hz, 1H), 3.65 (s, 3H).

MS *m*/*z* [M+H]⁺: 391.

### Step 2: Synthesis of 6-chloro-4-((6-chloro-3-(S-methyl-N-(4-nitrobenzenesulfonyl)sulfoximino)pyridin-2-yl)amino)-N-(methyl-d3)pyridazine-3-carboxamide (29b)

The synthesis referenced the synthesis of 6-chloro-4-((6-chloro-3-(*N*,*S-*dimethylsulfoximino)pyridin-2-yl)amino)-*N*-(methyl-d3)pyridazine-3-carboxamide, yield 50%.

1H NMR (400 MHz, DMSO-d6) δ 12.29 (s, 1H), 9.50 (s, 1H), 8.71 (s, 1H), 8.38 (d, *J* = 8.4 Hz, 1H), 8.20 (d, *J* = 8.6 Hz, 2H), 7.93 (d, *J =* 8.7 Hz, 2H), 7.54 (d, *J* = 8.3 Hz, 1H), 3.82 (s, 3H).

MS *m*/*z* [M+H]⁺: 563.

### Step 3: Synthesis of 6-chloro-4-((6-((diphenylmethylene)amino)-3-(S-methyl-N-((4-nitrobenzenesulfonyl)sulfoximino)pyridin-2-yl)amino)-N-(methyl-d3)pyridazine-3-carboxamide (29c)

The synthesis referenced the synthesis of 6-chloro-4-((3-(*N*,*S-*dimethylsulfoximino)-6-((diphenylmethylene)amino)pyridin-2-yl)amino)-*N-*methylpyridazine-3-carboxamide, dioxane was used as the solvent instead of DMF, reaction temperature was 110 °C, yield 25%.

1H NMR (400 MHz, DMSO-d6) δ 11.89 (s, 1H), 9.40 (s, 1H), 8.27 (s, 1H), 8.21 (d, *J =* 8.5 Hz, 1H), 8.03 (d, *J =* 8.4 Hz, 2H), 7.68 (d, *J =* 8.4 Hz, 2H), 7.59 - 7.45 (m, 10H), 6.98 (d, *J* = 8.5 Hz, 1H), 3.76 (s, 3H).

MS *m*/*z* [M+H]⁺: 708.

### Step 4: Synthesis of 6-(cyclopropylcarboxamido)-4-((6-((diphenylmethylene)amino)-3-(S-methyl-N-((4-nitrophenylsulfonyl)sulfoximino)pyridin-2-yl)amino)-N-(methyl-d3)pyridazine-3-carboxamide (29d)

The synthesis referenced the synthesis of 6-(cyclopropylcarboxamido)-4-((3-(*N*,*S-*dimethylsulfoximino)pyridin-2-yl)amino)-*N*-(methyl-*d3*)pyridazine-3-carboxamide, the reaction was completed and processed without separation, and the crude product was used directly in the next step.

MS *m*/*z* [M+H]⁺: 757.

### Step 5: Synthesis of 4-((6-amino-3-(S-methyl-N-(4-nitrobenzenesulfonyl)-sulfoximino)pyridin-2-yl)amino)-6-(cyclopropylcarboxamido)-N-(methyl-d3)pyridazine-3-carboxamide (29e)

The synthesis referenced the synthesis of 4-((6-amino-3-(*N*,*S-*dimethylsulfoximino)pyridin-2-yl)amino)-6-(cyclopropylcarboxamido)-*N-*methylpyridazine-3-carboxamide using the crude product 29d instead of 6-(cyclopropylcarboxamido)-4-((3-(*N*,*S*-dimethylsulfoximino)-6-((diphenylmethylene)amino)pyridin-2-yl)amino)-*N*-methylpyridazine-3-carboxamide, two-step yield 43%.

MS *m*/*z* [M+H]⁺: 593.

### Step 6: Synthesis of (R)-4-((6-amino-5-chloro-3-(S-methylsulfoximino)pyridin-2-yl)amino)-6-(cyclopropylcarboxamido)-N-(methyl-d3)pyridazine-3-carboxamide (29)

NCS (248 mg, 1.86 mmol) was added to 4-((6-amino-3-(S-methyl-*N*-(4-nitrobenzenesulfonyl)-sulfoximino)pyridin-2-yl)amino)-6-(cyclopropylcarboxamido)-*N*-(methyl-*d3*)pyridazine-3-carboxamide (29e) (1.1 g, 1.86 mmol) in anhydrous DMF (20 mL), and stirred at 60°C for 1 hour. After cooled to room temperature, the reaction was quenched by adding saturated aqueous sodium sulfite solution (10 mL) and water (80 mL) to the reaction solution, stirred for 10 minutes and then suction filtered. The filter cake was washed with water, dried and then beated with dichloromethane and filtered to obtain the chlorinated crude product (380 mg, earthy yellow solid).

Aqueous potassium hydroxide (851 mg, 15.2 mmol) (1 mL) was added to p-methoxybenzenethiol (2.18 g, 15.6 mmol) in acetonitrile (5 mL), stirred for 5 minutes, and the chlorinated crude product (380 mg) was added, and continued to be stirred for 1 hour at room temperature. The reaction was quenched by the addition of saturated potassium carbonate solution, extracted with ethyl acetate, the organic phases were combined and dried over anhydrous sodium sulfate. The obtained was concentrated and Flash separated (methanol: dichloromethane = 1:10) to obtain the racemic compound 4-((6-amino-5-chloro-3-(S-methylsulfoximino)pyridin-2-yl)amino)-6-(cyclopropylcarboxamido)-*N*-(methyl-*d3*)pyridazine-3-carboxamide (250 mg, yellow solid), yield 30%.

The racemic compound was subjected to SFC chiral splitting to afford (*S*)-4-((6-amino-5-chloro-3-(*S*-methylsulfoximino)pyridin-2-yl)amino)-6-(cyclopropylcarboxamido)-*N*-(methyl-*d3*)pyridazine-3-carboxamide (29f) (85 mg, white solid, yield 10%) and (*R*)-4-((6-amino-5-chloro-3-(*S-*methylsulfoximino)pyridin-2-yl)amino)-6-(cyclopropylcarboxamido)-*N*-(methyl-*d3*)pyridazine-3-carboxamide (29) (85 mg, pale yellow solid, yield 10%).

29f: 1H NMR (400 MHz, DMSO-d6) δ 11.70 (s, 1H), 11.36 (s, 1H), 9.41 (s, 1H), 9.04 (s, 1H), 7.87 (s, 1H), 6.83 (s, 2H), 4.50 (s, 1H), 3.09 (s, 3H), 2.17 - 2.06 (m, 1H), 0.85 (d, *J* = 6.0 Hz, 4H).

MS *m*/*z* [M+H]⁺: 442.

29: 1H NMR (400 MHz, DMSO-d6) δ 11.71 (s, 1H), 11.36 (s, 1H), 9.41 (s, 1H), 9.04 (s, 1H), 7.87 (s, 1H), 6.83 (s, 2H), 4.50 (s, 1H), 3.09 (s, 3H), 2.17 - 2.06 (m, 1H), 0.85 (d, *J* = 5.2 Hz, 4H).

MS *m*/*z* [M+H]⁺: 442.

### Example 30

### Preparation of (R)-4-((6-amino-5-bromo-3-(N,S-dimethylsulfoximino)pyridin-2-yl)amino)-6-(cyclopropylformyl)-N-(methyl-d3)pyridazine-3-carboxamide (30)

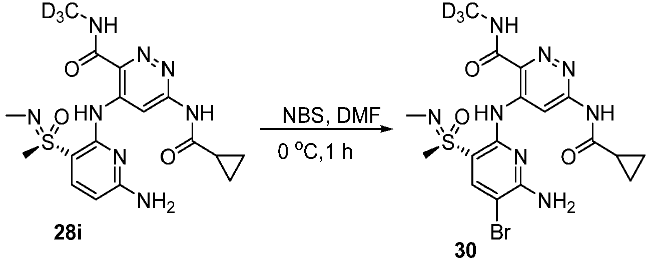

(*R*)-4-((6-amino-3-(*N,S*-dimethylsulfoximino)pyridin-2-yl)amino)-6-(cyclopropylcarboxamido)-*N*-(methyl-*d3*)pyridazine-3-carboxamide (28i) (7.95 g, 18.9 mmol) was dissolved in NMP (48 mL), and NBS (3.38 g, 19 mmol) was added in batches in an ice bath, and stirred for half an hour in an ice bath. After the reaction was completed, Na2SO3 saturated aqueous solution (6.5 mL) was added in an ice bath, stirred for 10 minutes, ice water (500 mL) was added in batches, solids was precipitated, stirred for 1 hour. The obtained was suction filtered, and the filter cake was washed with water (50 mL). The filter cake was dissolved in DCM/MeOH (10/1, 300 mL), dried over anhydrous sodium sulfate, filtered and concentrated. The obtained was beated with EtOH/DCM (1/10, 30 mL) to obtain 5.2 g of off-white solid. The mother liquor was mixed with silica gel and subjected to column chromatography (methanol/dichloromethane = 2~5%) to obtain 4.3 g of white solid. The obtained solids in two batches were combined, EA (97 mL) was added, heated for half an hour in oil bath at 75 degrees, slowly cooled to room temperature, suction filtered to obtain 8.15 g of gray-white solid, yield 86%.

1H NMR (400 MHz, DMSO-*d6*) δ 11.60 (s, 1H), 11.35 (s, 1H), 9.46 (s, 1H), 9.02 (s, 1H), 7.89 (s, 1H), 6.77 (s, 2H), 3.12 (s, 3H), 2.60 (s, 3H), 2.14-2.06 (m, 1H), 0.85 (d, *J* = 6.5 Hz, 4H).

MS *m*/*z* [M+H]⁺: 500.

### Example 31

### Preparation of 6-(cyclopropylcarboxamido)-4-((5-(N,S-dimethylsulfoximino)-1H-pyrrolo[2,3-b]pyridin-6-yl)amino)-N-methylpyridazine-3-carboxamide

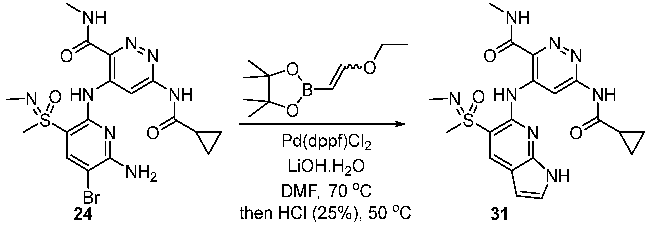

### Step 1: Synthesis of 6-(cyclopropylcarboxamido)-4-((5-(N,S-dimethylsulfoximino)-1H-pyrrolo[2,3-b]pyridin-6-yl)amino)-N-methylpyridazine-3-carboxamide (31)

The synthesis of 2-(2-ethoxyvinyl)-4,4,5,5-tetramethyl-1,3,2-dioxacyclopentaborane referenced WO2012/010538, 2012, A2.

4-((6-amino-5-bromo-3-(*N,S*-dimethylsulfoximino)pyridin-2-yl)amino)-6-(cyclopropylcarboxamido)-*N*-methylpyridazine-3-carboxamide (24) (12 mg, 0.0241 mmol), 2-(2-ethoxyethenyl)-4,4,5,5-tetramethyl-1,3,2-dioxacyclopentaborane (5.7 mg, 0.0290 mmol), Pd(dppf)Cl₂ (1.8 mg, 0.00241 mmol) and lithium hydroxide monohydrate (3.0 mg, 0.0723 mmol) were dissolved in anhydrous DMF (0.5 mL), and stirred at 70°C for 1 hour under nitrogen atmosphere. The reaction solution was cooled to room temperature, 25% aqueous hydrochloric acid (53 mg) was added, and the reaction was carried out at 50°C for 1 hour. The reaction was quenched with water, extracted with ethyl acetate, washed with saturated brine and dried over anhydrous sodium sulfate. After being concentrated, the obtained was Flash separated (methanol/dichloromethane = 4 - 7%), prep-HPLC prepared to obtain 6-(cyclopropylcarboxamido)-4-((5-(*N*,*S*-dimethylsulfoximino)-1H-pyrrolo[2,3-b]pyridin-6-yl)amino)-*N*-methylpyridazine-3-carboxamide (31) (1.2 mg, yellow solid), yield 11%.

1H NMR (400 MHz, DMSO-*d6*) δ 11.93 (s, 1H), 11.66 (s, 1H), 11.33 (s, 1H), 9.11 (s, 1H), 9.04 (d, *J=* 5.0 Hz, 1H), 8.48 (s, 1H), 7.48 (d, *J=* 3.4 Hz, 1H), 6.62 (d, *J*= 3.4 Hz, 1H), 3.14 (s, 3H), 2.84 (d, *J =* 4.6 Hz, 3H), 2.67 (s, 3H), 2.12 - 2.04 (m, 1H), 0.90 - 0.73 (m, 4H).

MS *m*/*z* [M+H]⁺: 443.

### Example 32

### Preparation of 4-((3-chloro-5-(N,S-dimethylsulfoximino)-1H-pyrrolo[2,3-b]pyridin-6-yl)amino)-6-(cyclopropylamido)-N-methylpyridazine-3-carboxamide

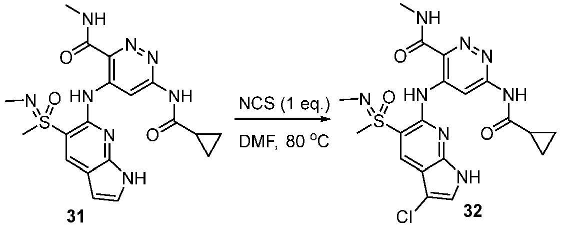

6-(Cyclopropylcarboxamido)-4-((5-(*N,S-*dimethylsulfoximino)-1H-pyrrolo[2,3-b]pyridin-6-yl)amino)-*N*-methylpyridazine-3-carboxamide (31) (16 mg, 0.0362 mmol) was dissolved in DMF (0.5 mL), and NCS (6 mg, 0.0434 mmol) was added at room temperature, stirred at 80°C for 1.5 hours, cooled down to room temperature, the reaction solution was subjected to direct prep-HPLC to obtain 4-((3-chloro-5-(*N*,*S-*dimethylsulfoximino)-1H-pyrrolo[2,3-b]pyridin-6-yl)amino)-6-(cyclopropylamido)-N-methylpyridazine-3-carboxamide (32) (5.3 mg, light yellow solid), yield 31%.

1H NMR (400 MHz, DMSO-*d6*) δ 12.24 (s, 1H), 11.74 (s, 1H), 11.38 (s, 1H), 9.14 (s, 1H), 9.09 (d, *J =* 4.6 Hz, 1H), 8.35 (s, 1H), 7.71 (s, 1H), 3.19 (s, 3H), 2.84 (d, *J=* 4.5 Hz, 3H), 2.67 (s, 3H), 2.13 - 2.05 (s, 1H), 0.92 - 0.74 (m, 4H).

MS *m*/*z* [M+H]⁺: 477.

### Example 33

### Preparation of 6-(cyclopropylamido)-4-((2,3-dichloro-5-(N,S-dimethylsulfoximino)-1H-pyrrolo[2,3-b]pyridin-6-yl)amino)-N-methylpyridazine-3-carboxamide

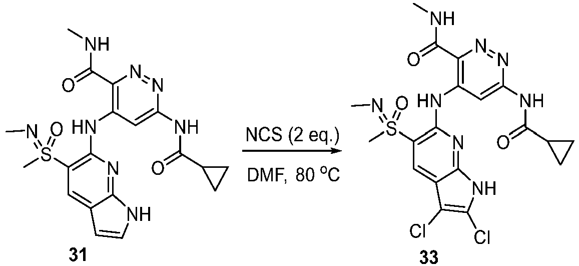

6-(Cyclopropylcarboxamido)-4-((5-(*N,S*-dimethylsulfoximino)-1H-pyrrolo[2,3-b]pyridin-6-yl)amino)-*N*-methylpyridazine-3-carboxamide (31) (21 mg, 0.0475 mmol) was dissolved in DMF (1 mL), and NCS (14 mg, 0.104 mmol) was added at room temperature, stirred at 80°C for 1 hour, and cooled to room temperature. The reaction was quenched with water, extracted with ethyl acetate, washed with saturated brine, dried over anhydrous sodium sulfate, concentrated and then Flash separated (methanol/dichloromethane = 3~5%), and prep-HPLC prepared to obtain 6-(cyclopropylamido)-4-((2,3-dichloro-5-(*N,S*-dimethylsulfoximino)-1H-pyrrolo[2,3-b]pyridin-6-yl)amino)-*N*-methylpyridazine-3-carboxamide (33) (1.0 mg, brown solid), yield 4%.

1H NMR (400 MHz, DMSO-*d6*) δ 13.29 (s, 1H), 11.74 (s, 1H), 11.37 (s, 1H), 9.05 (s, 2H), 8.27 (s, 1H), 3.18 (s, 3H), 2.84 (d, J = 4.7 Hz, 3H), 2.66 (s, 3H), 2.09 (s, 1H), 0.90 - 0.79 (m, 4H).

MS *m*/*z* [M+H]⁺: 511.

### Example 34

### Preparation of 4-((3-bromo-5-(N,S-dimethylsulfoximino)-1H-pyrrolo[2,3-b]pyridin-6-yl)amino)-6-(cyclopropylamido)-N-methylpyridazine-3-carboxamide

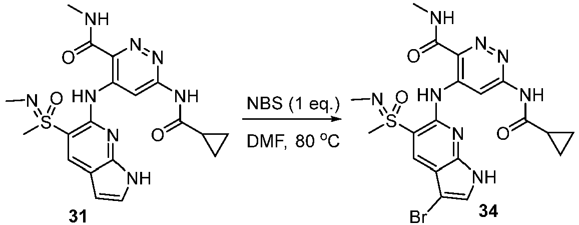

6-(Cyclopropylcarboxamido)-4-((5-(*N,S*-dimethylsulfoximino)-1H-pyrrolo[2,3-b]pyridin-6-yl)amino)-*N*-methylpyridazine-3-carboxamide (31) (67 mg, 0.151 mmol) was dissolved in dichloromethane (2 mL), and NBS (26.6 mg, 0.151 mmol) was added at room temperature, stirred at room temperature for 2 hours, the solvent was spin-dried, Flash separated (methanol/dichloromethane = 2~5%) to obtain 4-((3-bromo-5-(N,S-dimethylsulfoximino)-1H-pyrrolo[2,3-b]pyridin-6-yl)amino)-6-(cyclopropylamido)-N-methylpyridazine-3-carboxamide (34) (60 mg, yellow solid), yield 76%.

1H NMR (400 MHz, DMSO-*d6*) δ 12.29 (s, 1H), 11.73 (s, 1H), 11.37 (s, 1H), 9.14 (s, 1H), 9.08 (d, *J =* 4.7 Hz, 1H), 8.27 (s, 1H), 7.73 (s, 1H), 3.19 (s, 3H), 2.84 (d, *J* = 4.7 Hz, 3H), 2.67 (s, 3H), 2.18 - 2.02 (m, 1H), 0.89 - 0.75 (m, 4H).

MS *m*/*z* [M+H]⁺: 521.

### Example 35

### Preparation of (R)-4-((6-amino-5-chloro-3-(N,S-dimethylsulfoximino)pyridin-2-yl)amino)-6-(cyclopropylformyl)-N-(methyl-d3)pyridazine-3-carboxamide oxalate

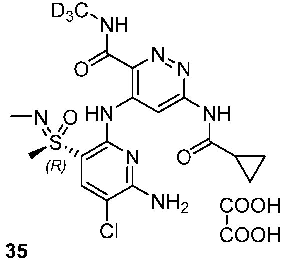

**(*R*)-4-((6-amino-5-chloro-3-(*N*,*S*-dimethylsulfoximino)pyridin-2-yl)amino)-6-(cyclopropylformyl)-*N*-(methyl-*d3*)pyridazine-3-carboxamide** (28) (100 mg, 0.22 mmol) was dissolved in DCM/MeOH (9:1, 6 mL), and oxalic acid dihydrate (27 mg, 0.22 mmol) in methanol (0.2 mL) was added, and stirred for 10 min at room temperature. The solvent was evaporated under reduced pressure, and DCM/THF (1:1, 3 mL) was added. The solid was precipitated, suction filtered, washed with DCM/THF (1:1, 1 mL) and rinsed with PE (3 mL). 72 mg of light yellow solid was obtained, yield 56.7%.

¹H NMR (400 MHz, DMSO-*d⁶*) δ 11.61 (s, 1H), 11.36 (s, 1H), 9.43 (s, 1H), 9.04 (s, 1H), 7.79 (s, 1H), 6.90 (s, 2H), 3.14 (s, 3H), 2.62 (s, 3H), 2.15-2.07 (m, 1H), 0.86 (d, *J =* 6.4 Hz, 4H).

### Example 36

### Preparation of (R)-4-((6-amino-5-chloro-3-(N,S-dimethylsulfoximino)pyridin-2-yl)amino)-6-(cyclopropylcarbonyl)-N-(methyl-d3)pyridazine-3-carboxamide hydrochloride

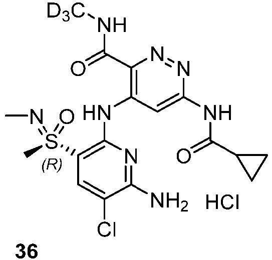

(*R*)-4-((6-amino-5-chloro-3-(*N,S*-dimethylsulfoximino)pyridin-2-yl)amino)-6-(cyclopropylformyl)-*N*-(methyl-*d3*)pyridazine-3-carboxamide (28) (500 mg, 1.10 mmol) was added to MeCN (5 mL) in suspension state. 37% HCl aq. (271 mg, 2.75 mmol) was added dropwise at room temperature, heated to reflux in an oil bath at 85 °C, water (2 mL) was added dropwise, and dissolved to be clear in reflux state. Then the solution was stirred at room temperature for 1 h. The solid was precipitated, suction filtered, the filter cake was washed with MeCN (1 mL*2), and the filter cake was dried under reduced pressure at 50 °C to obtain 336 mg of yellow solid, yield 57.9%.

¹H NMR (400 MHz, DMSO-*d⁶*) δ 12.03 (s, 1H), 11.92 (s, 1H), 9.49 (s, 1H), 9.23 (s, 1H), 7.92 (s, 1H), 7.86 (s, 2H), 3.92 (s, 3H), 2.83 (s, 3H), 2.21-2.14 (m, 1H), 0.90 (d, *J* = 6.7 Hz, 4H).

### Example 37

### Preparation of (R)-4-((6-amino-5-chloro-3-(N,S-dimethylsulfoximino)pyridin-2-yl)amino)-6-(cyclopropylformyl)-N-(methyl-d3)pyridazine-3-carboxamide sulfate

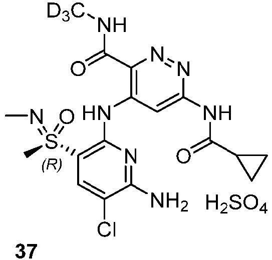

(*R*)-4-((6-amino-5-chloro-3-(*N*,*S*-dimethylsulfoximino)pyridin-2-yl)amino)-6-(cyclopropylformyl)-*N*-(methyl-*d3*)pyridazine-3-carboxamide (28) (500 mg, 1.10 mmol) was added to THF (5 mL) in suspension state. 20% H₂SO₄ aq. (646 mg,1.32 mmol) was added dropwise at room temperature, placed in an oil bath at 70 °C, water (0.5 mL) was added dropwise in the reflux state, dissolved to be clear in the reflux state, and then placed in the environment at room temperature and stirred for 1h, the solid was precipitated, suction filtered, the filter cake was washed with THF (1 mL*2), the filter cake was dried at 50 °C under reduced pressure to obtain 495 mg of yellow solid. Y=81.5%, HPLC:99.0%.

¹H NMR (400 MHz, DMSO-*d6*) δ 11.81 (s, 1H), 11.45 (s, 1H), 9.33 (s, 1H), 9.29 (s, 1H), 7.89 (s, 1H), 7.62 (s, 2H), 3.90 (s, 3H), 2.85 (s, 3H), 2.16-2.08 (m, 1H), 0.87 (d, *J* = 6.7 Hz, 4H).

### Example 38

### Preparation of (R)-4-((6-amino-5-chloro-3-(N,S-dimethylsulfoximino)pyridin-2-yl)amino)-6-(cyclopropylformyl)-N-(methyl-d3)pyridazine-3-carboxamide methanesulfonate

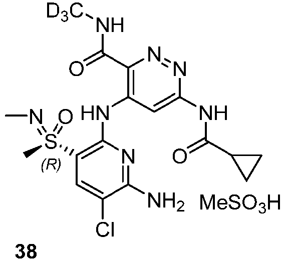

(*R*)-4-((6-Amino-5-chloro-3-(*N,S*-dimethylsulfoximino)pyridin-2-yl)amino)-6-(cyclopropylformyl)-*N*-(methyl-*d3*)pyridazine-3-carboxamide (28) (100 mg, 0.22 mmol) was dissolved in DCM/MeOH (9:1, 6 mL), and methylsulfonic acid (42 mg, 0.44 mmol) was added, stirred at room temperature for 10 min. The solvent was evaporated under reduced pressure and DCM/THF (1:1, 3 mL) was added. The solid was precipitated, suction filtered, washed with DCM/THF (1:1, 1 mL) and washed with PE (3 mL). 110 mg of yellow solid was obtained. Yield: 77.4%.

¹H NMR (400 MHz, DMSO-*d⁶*) δ 11.82 (s, 1H), 11.47 (s, 1H), 9.34 (s, 1H), 9.30 (s, 1H), 7.90 (s, 1H), 7.63 (s, 2H), 3.91 (s, 3H), 2.86 (s, 3H), 2.35 (d, *J=* 1.0 Hz, 6H), 2.12 (d, *J = 5.7* Hz, 1H), 0.88 (d, *J =* 6.7 Hz, 4H).

### Example 39

### Preparation of 6-(cyclopropylcarboxamido)-N-(methyl-d₃)-4-((3-(S-methyl-N-(methyl-d3)sulfoximino)pyridin-2-yl)amino)pyridazine-3-carboxamide

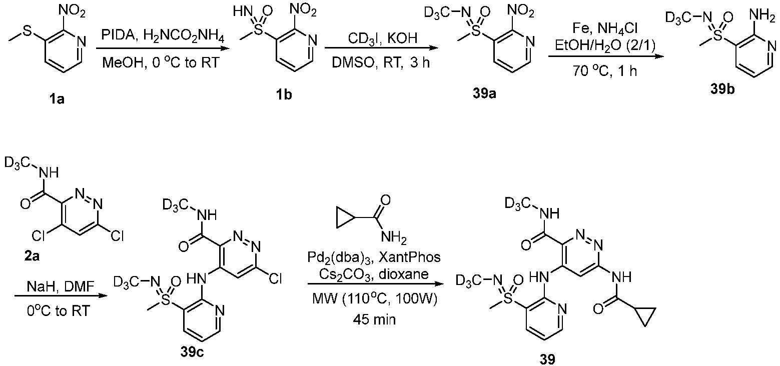

The syntheses were prepared using CD₃I instead of MeI referenced Example 1 and Example 2.

1H NMR (400 MHz, dmso) δ 11.86 (s, 1H), 11.37 (s, 1H), 9.46 (s, 1H), 9.06 (s, 1H), 8.53 (d, J = 4.0 Hz, 1H), 8.22 (d, J = 7.6 Hz, 1H), 7.32-7.24 (m, 1H), 3.21 (s, 3H), 2.09 (dd, J = 13.2, 7.3 Hz, 1H), 0.85 (d, J = 6.7 Hz, 4H).

MS *m*/*z* [M+H]⁺: 410.

### Example 40

### Preparation of (R)-4-((6-amino-5-chloro-3-(S-methyl-N-(methyl-d₃)sulfonylimino)pyridin-2-yl)amino)-6-(cyclopropylcarboxamide)-N-(methyl-d₃)pyridazine-3-carboxamide

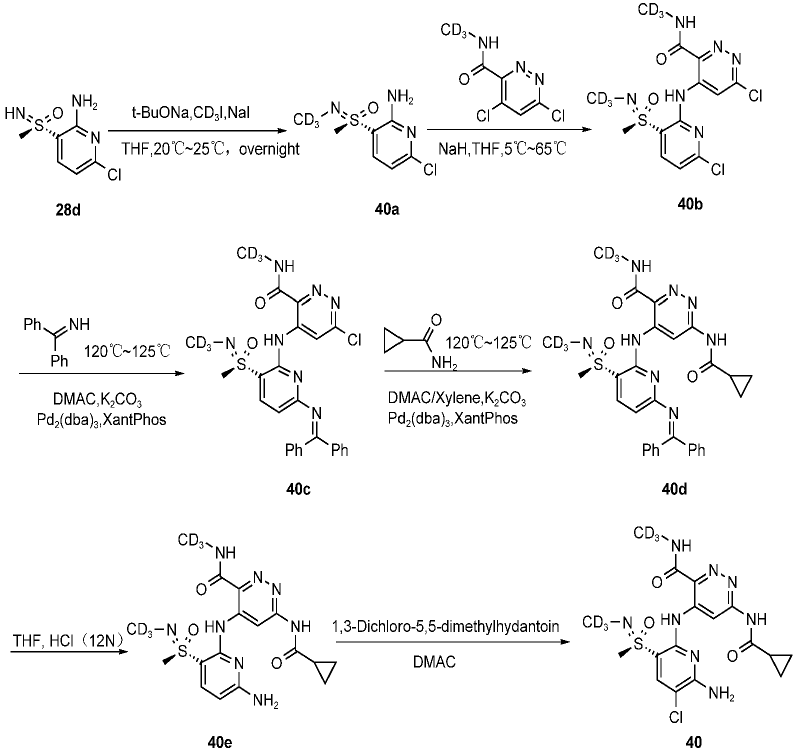

### Step 1: Synthesis of (R)-6-chloro-3-(S-methyl-N-(methyl-d₃) sulfonylimino)pyridin-2-amine (40a)

(*R*)-6-Chloro-3-(*S*-methylsulfoximino)pyridin-2-amine (28d) (29.7 g, 144.88 mmol) was dissolved in 600 mL of tetrahydrofuran, NaI (2.18 g, 14.5 mmol) was added, sodium tert-butanolate (13.91 g, 144.88 mmol) was added in batches n a water bath, and stirred for 20 min. CD₃I (21 g, 144.88 mmol) dissolved in tetrahydrofuran (50 mL) was added dropwise, after the addition, and stirred at room temperature overnight. The reaction solution was quenched with saturated ammonium chloride solution, diluted with water, the liquid was separated, the aqueous layer was extracted with ethyl acetate, the organic phases were combined, concentrated and dissolved with ethyl acetate, the liquid was separated, the aqueous layer was extracted with ethyl acetate, the organic phases were combined and washed with saturated brine, dried over anhydrous sodium sulfate, concentrated and separated by column chromatography (petroleum ether: ethyl acetate=2: 1) to obtain (*R*)-6-chloro-3-(*S*-methyl-*N*-(methyl *-d₃*)sulfonylimino)pyridin-2-amine (22.5 g, off-white solid), yield 70%.

¹H NMR (400 MHz, DMSO-*d₆*) δ 7.84 (d, J = 8.0 Hz, 1H), 7.24 (s, 2H), 6.78 (d,J=8.0 Hz 1H), 3.09 (s,3H).

MS m/z [M+H]⁺: 223.

### Step 2: Synthesis of (R)-6-chloro-4-((6-chloro-3-(S-methyl-N-(methyl-d₃) sulfonylimino)pyridin-2-yl)amino)-N-(methyl-d₃)pyridazine-3-carboxamide (40b)

(R)-6-Chloro-3-(S-methyl-N-(methyl-*d3*)sulfonylimino) pyridin -2-amine (40a) (36.7 g, 0.165 mol) was dissolved in 1 L of tetrahydrofuran, and cooled down to 0~5 °C under N₂ protection, NaH (19.8 g, 60% w/w, 0.496 mol) was added in batches and then stirred for 25 min, and 4,6-dichloro-N-(methyl-*d3*)pyridazine-3-carboxamide (41.3 g, 0.198 mol) was added in batches, stirred for 15 min and then heated to 70 °C and reacted for 2 h. The reaction was quenched with saturated ammonium chloride solution in an ice bath, diluted with water, extracted with ethyl acetate, the liquid was separated, and the organic phases were combined and washed with saturated brine, and dried over anhydrous sodium sulfate. The obtained was concentrated, and ethanol was added for cold beating for 30 min, suction filtered, the filter cake was washed with ethanol, drained to obtain (*R*)-6-chloro-4-((6-chloro-3-(S-methyl-*N*-(methyl-*d₃*)sulfonylimino)pyridin-2-yl)amino)-*N*-(methyl-*d₃*)pyridazine-3-carboxamide (40.25 g, 61.8%).

1H NMR (400 MHz, DMSO-d6) δ 12.23 (s, 1H), 9.33 (s, 1H), 8.79 (d, J = 1.4 Hz, 1H), 8.23 (dd, J = 8.0, 1.4 Hz, 1H), 7.43 (dd, J = 8.1, 1.4 Hz, 1H),3.24 (s, 3H).

MS m/z [M+H]⁺: 395

### Step 3: Synthesis of (R)-6-chloro-4-((6-((diphenylmethylene)amino)-3-(S-methyl-N-(methyl-d₃)sulfonylimino)pyridin-2-yl)amino)-N-(methyl-d₃)pyridazine-3-carboxamide (40c)

Pd₂(dba)₃ (4.6 g, 0.005 mol), XantPhos (5.78 g, 0.01 mol), (*R*)-6-chloro-4-((6-chloro-3-(*S*-methyl-*N*-(methyl-*d₃*)sulfonylimino)pyridin-2-yl)amino)-*N*-(methyl-*d3*)pyridazin- 3-carboxamide (40b) (40 g, 0.1 mol), K₂CO₃ (34.5 g, 0.25 mol) and diphenylimine (27.6 g, 0.15 mol) were added to DMAC (400 mL) under N₂ atmosphere, and reacted at 130 °C for 5 h. After being cooled, the reaction was quenched with saturated ammonium chloride solution, diluted with water, solubilized with ethyl acetate, filtered through diatomaceous earth, and the filter cake was washed with ethyl acetate. The liquid was separated, the aqueous phase was extracted with ethyl acetate, and the organic phases were combined and washed with saturated brine, dried over anhydrous sodium sulfate, concentrated and beated with MTBE for 30 min, suction filtered, and the filter cake was washed with MTBE, and drained to obtain (*R*)-6-chloro-4-((6-((diphenylmethylene)amino)-3-(*S*-methyl-*N*-(methyl-*d₃*)sulfonylimino)pyridin-2-yl)amino)-*N*-(methyl -*d₃*)pyridazine-3-carboxamide (38 g, 69.5%).

¹H NMR (400 MHz, DMSO-*d₆*) δ 11.96 (s, 1H), 9.24 (s, 1H), 8.48 (s, 1H), 8.06 (d, J = 8.2 Hz, 1H), 7.43 (m, 10H), 6.79 (d, J = 8.0 Hz, 1H), 3.16 (s, 3H).

MS m/z [M+H]⁺: 540

### Step 4: Synthesis of (R)-6-(cyclopropylcarboxamide)-4-((6-((diphenylmethylene)amino)-3-(S-methyl-N-(methyl-d₃)sulfonylimino)pyridin-2-yl)amino)-N-(methyl-d₃)pyridazine-3-carboxamide (40d)

Pd₂(dba)₃ (238 mg, 0.26 mmol), XantPhos (300 mg,0.52 mol), (*R*)-6-chloro-4-((6-((dibenzylidene)amino)-3-(*S*-methyl-*N*-(methyl-*d₃*)sulfonylimino)pyridin-2-yl)amino)-*N*-(methyl-*d₃*)pyridazine-3-carboxamide (40c) (2.8 g, 5.19 mmol), K₂CO₃ (1.79 g, 12.98 mmol) and cyclopropylamide (882 mg, 10.38 mmol) were added to xylene: DMAC=10:1 (33 mL) under N₂ atmosphere, and the reaction was carried out at 130 °C for 2 h. After being cooled, the reaction was quenched with saturated ammonium chloride solution, diluted with water, and dissolved with dichloromethane, and filtered through diatomaceous earth, the filter cake was washed with dichloromethane. The liquid was separated, the aqueous phase was extracted with dichloromethane, and the organic phases were combined and washed with saturated brine, dried over anhydrous sodium sulfate, concentrated and beated with petroleum ether for 10 min, suction filtered, and the filter cake was washed with petroleum ether, and drained to obtain (*R*)-6-(cyclopropylcarboxamido)-4-((6-((dibenzylidene)amino)-3-(S-methyl-*N*-(methyl-*d₃*)sulfonylimino)pyridin-2-yl)amino)-*N*-(methyl-*d₃*)pyridazine-3-carboxamide in crude form, which was directly used in the next step of the reaction without separation.

MS m/z [M+H]⁺: 589

### Step 5: Synthesis of (R)-4-((6-amino-3-(S-methyl-N-(methyl-d₃)sulfonylimino)pyridin-2-yl)amino)-6-(cyclopropylcarboxamide)-N-(methyl-d₃)pyridazine-3-carboxamide (40e)

Crude (*R*)-6-(cyclopropylcarboxamide)-4-((6-((dibenzylidene)amino)-3-(S-methyl-*N*-(methyl-*d₃*)sulfonylimino)pyridin-2-yl)amino)-*N*-(methyl-*d₃*)pyridazine-3-carboxamide (40d) was dissolved in tetrahydrofuran (30 mL), and 12N HCl (0.6 mL) was added in an ice bath, and the solution was heated to room temperature and stirred for 1.5 h. The solution was diluted with water, and extracted with ethyl acetate, the organic phase was washed with water, the aqueous layer was combined, 10% potassium carbonate solution was slowly added to adjust the pH to be 9~10, extracted with methanol: dichloromethane = 10:1, the liquid was separated,, the organic phases were combined, and washed with saturated brine, dried over anhydrous sodium sulfate, concentrated, ethanol was added, heated to reflux, beated for 20 min in an ice bath, suction filtered, and the filter cake was washed with ethanol, and drained to obtain (*R*)-4-((6-amino-3-(*S*-methyl-*N*-(methyl-*d₃*)sulfonylimino)pyridin-2-yl)amino)-6-(cyclopropylcarboxamide)-*N*-(methyl-*d₃*)pyridazine-3-carboxamide (1.4 g, 65%).

¹H NMR (400 MHz, DMSO-*d₆*) δ 11.56 (s, 1H), 11.32 (s, 1H), 9.51 (s, 1H), 9.00 (s, 1H), 7.75 (d, J = 8.8 Hz, 1H), 6.55 (s, 2H), 6.24 (d, J = 8.6 Hz, 1H), 3.06 (s, 3H), 2.15 -2.03 (m, 1H), 0.86 (m, 4H).

MS m/z [M+H]⁺: 425

### Step 6: Synthesis of (R)-4-((6-amino-5-chloro-3-(S-methyl-N-(methyl-d₃)sulfonylimino)pyridin-2-yl)amino)-6-(cyclopropylcarboxamide)-N-(methyl-d₃)pyridazine-3-carboxamide (40)

(*R*)-4-((6-Amino-3-(*S*-methyl-*N*-(methyl-*d₃*)sulfonylimino)pyridin-2-yl)amino)-6-(cyclopropylcarboxamido)-*N*-(methyl-*d₃*)pyridazine-3-carboxamide (**40e**) (1.4 g, 3.30 mmol) was dissolved in DMAC (14 mL), cooled down in an ice bath under the protection of N2 (0-5 °C), 1,3-dichloro-5,5-dimethylhydantoin (683 mg, 3.47 mmol) was added in batches, and the reaction was carried out for 15 min. The reaction was quenched with ice water and saturated sodium sulfite solution, suction filtered, the filter cake was washed with water, and dissolved with dichloromethane: methanol = 10:1, and the liquid was separated, and the organic phase was washed with saturated brine, dried over anhydrous sodium sulfate, concentrated and separated by Flash (methanol/dichloromethane = 3%) to obtain (R)-4-((6-amino-5-chloro-3-(*S*-methyl-*N-*(methyl-*d₃*)sulfonylimino)pyridin-2-yl)amino)-6-(cyclopropylcarboxamido)-*N-*(methyl-*d₃*)pyridazine-3-carboxamide (669 mg, off-white solid), yield 44%.

¹H NMR (400 MHz, DMSO-*d₆*) δ 11.62 (s, 1H), 11.37 (s, 1H), 9.43 (s, 1H), 9.04 (s, 1H), 7.79 (s, 1H), 6.89 (s, 2H), 3.13 (s, 3H), 2.16 - 2.07 (m, 1H), 0.87 (m, 4H).

MS m/z [M+H]+: 459

### Example 41

### Preparation of (R)-4-((6-amino-5-bromo-3-(S-methyl-N-(methyl-d₃)sulfonylimino)pyridin-2-yl)amino)-6-(cyclopropylcarboxamide)-N-(methyl-d₃)pyridazine-3-carboxamide

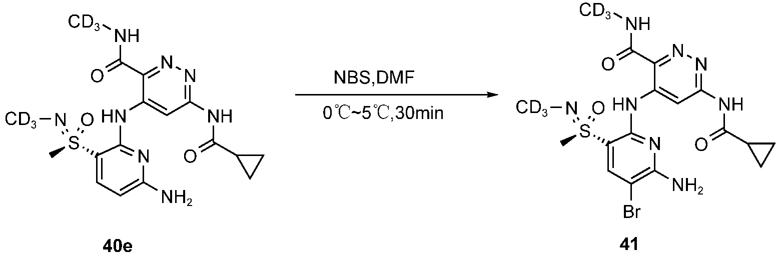

(R)-4-((6-Amino-3-(S-methyl-N-(methyl-*d₃*)sulfonylimino)pyridin-2-yl)amino)-6-(cyclopropylcarboxamido)-N-(methyl-*d₃*)pyridazine-3-carboxamide (**40e**) (113 mg, 0.26 mmol) was dissolved in DMF (1.4 mL), cooled down to 0~5 °C in an ice bath under the protection of N2, and NBS ( 50 mg, 0.28 mmol) was added, reacted for 30 min. The reaction was quenched with saturated sodium sulfite solution, diluted with water, extracted with ethyl acetate, the liquid was separated, the organic phases were combined and washed with saturated brine, dried over anhydrous sodium sulfate, concentrated and Flash separated (methanol/dichloromethane = 3%) to obtain (R)-4-((6-amino-5-bromo-3-(S-methyl-N-(methyl-*d₃*)sulfonylimino)pyridin-2-yl)amino)-6-(cyclopropylcarboxamide)-N-(methyl-*d₃*)pyridazine-3-carboxamide (62 mg, light yellow solid), yield 46%.

¹H NMR (400 MHz, DMSO-*d₆*) δ 11.62 (s, 1H), 11.37 (s, 1H), 9.48 (s, 1H), 9.04 (s, 1H), 7.91 (s, 1H), 6.79 (s, 2H), 3.13 (s, 3H), 2.12 (s, 1H), 0.88 (m, 4H).

MS m/z [M+H]⁺: 503

### Example 42

### Preparation of (R)-4-((6-amino-5-iodo-3-(S-methyl-N-(methyl-d₃)sulfonylimino)pyridin-2-yl)amino)-6-(cyclopropylcarboxamide)-N-(methyl-d₃)pyridazine-3-carboxamide

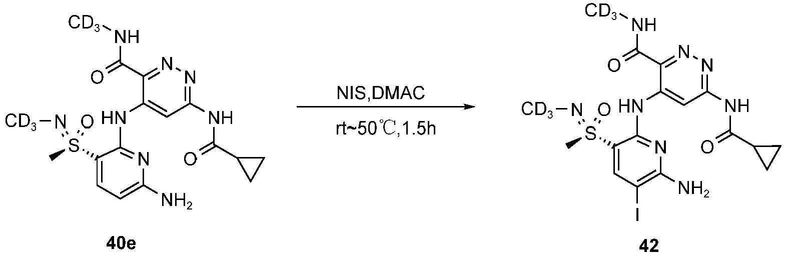

(R)-4-((6-amino-3-(S-methyl-N-(methyl-*d₃*)sulfonylimino)pyridin-2-yl)amino)-6-(cyclopropylcarboxamido)-N-(methyl-*d₃*)pyridazine-3-carboxamide (**40e**) (2.1 g, 2.59 mmol) was dissolved in DMAC (6 mL), and NIS (759 mg, 3.41 mmol) was added in batches, reacted at 50°C for 1 h. The reaction was quenched with saturated sodium sulfite solution in an ice bath, diluted with water, extracted with dichloromethane: methanol=10:1, the liquid was separated, the organic phases were combined and washed with saturated brine, dried over anhydrous sodium sulfate, concentrated and Flash separated (methanol/dichloromethane = 3%) to obtain (R)-4-((6-Amino-5-iodo-3-(S-methyl-N-(methyl-*d₃*)sulfonylimino)pyridin-2-yl)amino)-6-(cyclopropylcarboxamido)-N-(methyl-*d₃*)pyridazin-3-carboxamide (912 mg, pale yellow solid), yield 64%.

¹H NMR (400 MHz, DMSO-*d₆*) δ 11.61 (s, 1H), 11.37 (s, 1H), 9.54 (s, 1H), 9.04 (s, 1H), 8.06 (s, 1H), 6.60 (s, 2H), 3.12 (s, 3H), 2.17 - 2.07 (m, 1H), 0.87 (d, J = 6.0 Hz, 4H).

MS m/z [M+H]⁺: 551

### Example 43

### Preparation of (R)-4-((6-amino-5-cyclopropyl-3-(S-methyl-N-(methyl-d₃)sulfonylimino)pyridin-2-yl)amino)-6-(cyclopropylcarboxamide)-N-(methyl-d₃)pyridazine-3-carboxamide

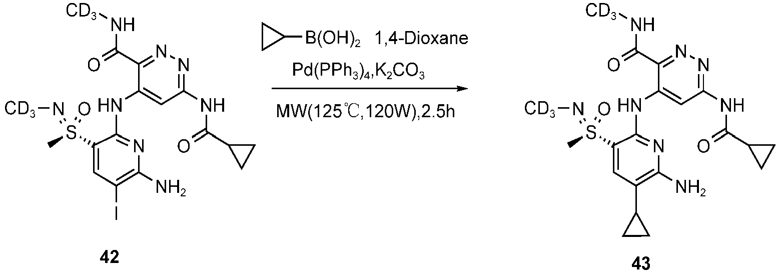

Under N2 atmosphere, (R)-4-((6-amino-5-iodo-3-(S-methyl-N-(methyl-*d₃*)sulfonylimino)pyridin-2-yl)amino)-6-(cyclopropylcarboxamido)-N-(methyl-*d₃*)pyridazine-3-carboxamide (42) (203 mg, 0.367 mmol) was dissolved in anhydrous 1,4-dioxane (4 mL), cyclopropylboronic acid (63 mg, 0.734 mmol), Pd(PPh₃)₄ (81 mg, 0.07 mmol) and K₂CO₃ (127 mg, 0.92 mmol) were added, and the reaction was carried out under microwave (120 °C, 120 W) for 2.5 h. After the reaction solution was cooled to room temperature, the reaction solution was filtered through diatomaceous earth, and the filter cake was washed with dichloromethane: methanol=10:1, and the filtrate was concentrated and Flash separated (methanol/dichloromethane = 7%) to obtain (R)-4-((6-amino-5-cyclopropyl-3-(S-methyl-N-(methyl-*d3*)sulfonylimino)pyridin-2-yl)amino)-6-(cyclopropylcarboxamido)-N-(methyl-*d3*)pyridazine-3-carboxamide (32 mg, light yellow solid), yield 18.7%.

¹H NMR (400 MHz,DMSO-d6) δ 11.55 (s, 1H), 11.32 (s, 1H), 9.57 (s, 1H), 8.99 (s, 1H), 7.42 (s, 1H), 6.49 (s, 2H), 3.05 (s, 3H), 2.12 (s, 1H), 1.66 (s, 1H), 0.88 (m, 6H), 0.51 (d, J = 4.4 Hz, 2H).

MS m/z [M+H]⁺: 465

### Example 44

### Preparation of (R)-4-((6-amino-3-(S-methyl-N-(methyl-d₃)sulfonylimino)-5-vinylpyridin-2-yl)amino)-6-(cyclopropylcarboxamide)-N-(methyl-d₃)pyridazine-3-carboxamide

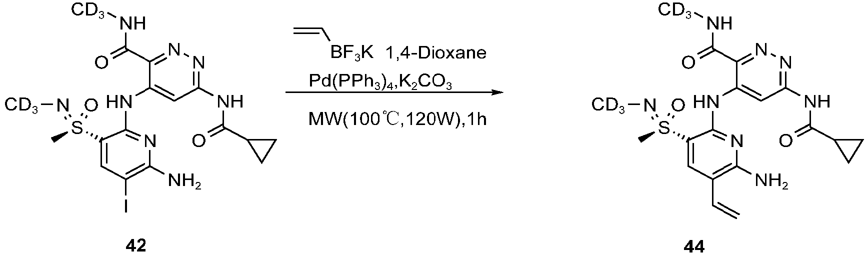

Under N2 atmosphere, (R)-4-((6-amino-5-iodo-3-(S-methyl-N-(methyl-*d₃*)sulfonylimino)pyridin-2-yl)amino)-6-(cyclopropylcarboxamido)-N-(methyl-*d₃*)pyridazine-3-carboxamide (42) (103 mg, 0.187 mmol) was dissolved in anhydrous 1,4-dioxane (2 mL), potassium ethylenetrifluoroborate (50 mg, 0.37 mmol), Pd(PPh₃)₄ (46 mg, 0.04 mmol) and K₂CO₃ (52 mg, 0.37 mmol) were added, and the reaction was carried out under microwave (120 °C, 120 W) for 1 h. After the reaction solution was cooled to room temperature, the reaction solution was filtered through diatomaceous earth, the filter cake was washed with dichloromethane, and the filtrate was concentrated and Flash separated (methanol/dichloromethane = 3%) to obtain (R)-4-((6-amino-3-(S-methyl-N-(methyl-*d₃*)sulfonylimino)-5-vinylpyridin-2-yl)amino)-6-(cyclopropylcarboxamido)-N-(methyl-*d3*)pyridazine-3-carboxamide (40 mg, light yellow solid), yield 47%.

¹H NMR (400 MHz, DMSO-*d₆*) δ 11.68 (s, 1H), 11.36 (s, 1H), 9.67 (s, 1H), 9.02 (s, 1H), 7.92 (s, 1H), 6.81 (dd, J = 17.6, 10.6 Hz, 1H), 6.59 (s, 2H), 5.63 (d, J = 16.9 Hz, 1H), 5.25 (d, J = 10.2 Hz, 1H), 3.12 (s, 3H), 2.12 (s, 1H), 0.87 (m, 4H).

MS m/z [M+H]⁺: 451

### Example 45

### Preparation of (R)-4-((6-amino-3-(S-methyl-N-(methyl-d₃)sulfonylimino)-5-(propynyl -1-yl)pyridin-2-yl)amino)-6-(cyclopropylcarboxamide)-N-(methyl-d₃)pyridazine-3-carboxamide

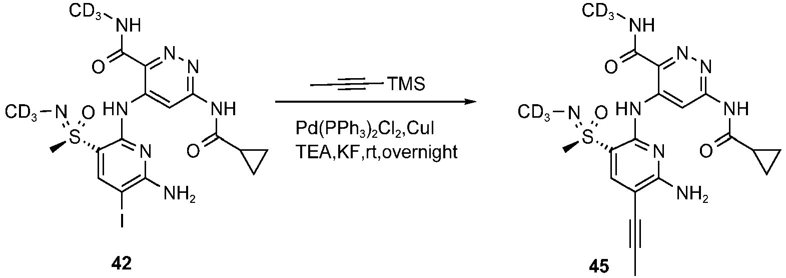

(R)-4-((6-Amino-5-iodo-3-(S-methyl-N-(methyl-*d₃*)sulfonylimino)pyridin-2-yl)amino)-6-(cyclopropylcarboxamido)-N-(methyl-*d3*)pyridazine-3-carboxamide (42) (100 mg, 0.182 mmol) was dissolved in DMF (2 mL), CuI (4 mg, 0.0182 mmol ), Pd(PPh₃)₂Cl₂ (13 mg, 0.0182 mmol), KF (16 mg, 0.273 mmol), and triethylamine (0.7 mL) were added, and 1-(trimethylsilyl)propyne (31 mg, 0.273 mmol) in DMF (0.5 mL) was added under N₂ atmosphere, reacted at 50 °C for 1 h. The reaction solution was cooled to room temperature and then quenched with saturated ammonium chloride solution, diluted with water, extracted with dichloromethane, the organic phases were combined, washed with saturated brine and dried over anhydrous sodium sulfate. After being concentrated, the obtained was Flash separated (methanol/dichloromethane = 3%) to obtain (R)-4-((6-amino-3-(S-methyl-N-(methyl-*d₃*)sulfonylimino)-5-(propynyl -1-yl)pyridin-2-yl)amino)-6-(cyclopropylcarboxamido)-N-(methyl-*d₃*)pyridazine-3-carboxamide (64 mg, yellow solid), yield 76%.

¹H NMR (400 MHz, DMSO-d6) δ 11.67 (s, 1H), 11.36 (s, 1H), 9.55 (s, 1H), 9.02 (s, 1H), 7.71 (s, 1H), 6.67 (s, 2H), 3.10 (s, 3H), 2.10 (m,, 4H), 0.87 (m, 4H).

MS m/z [M+H]⁺: 463

### Example 46

### Preparation of (R)-4-((6-amino-5-(cyclopropylethynyl)-3-(S-methyl-N-(methyl-d₃)sulfonylimino)pyridin-2-yl)amino)-6-(cyclopropylcarboxamide)-N-(methyl-d₃)pyridazine-3-carboxamide

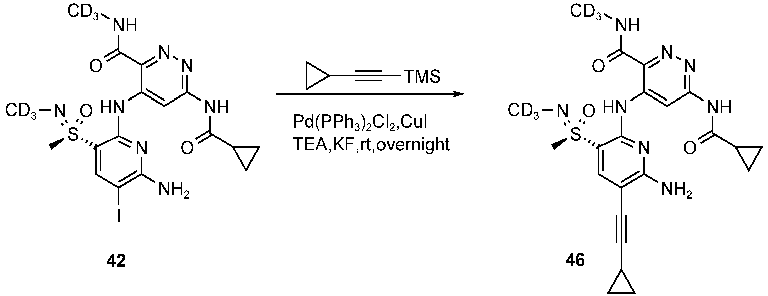

(R)-4-((6-Amino-5-iodo-3-(S-methyl-N-(methyl-*d₃*)sulfonylimino)pyridin-2-yl)amino)-6-(cyclopropylcarboxamido)-N-(methyl-*d3*)pyridazine-3-carboxamide (**42**) (100 mg, 0.182 mmol) was dissolved in DMF (2 mL), CuI (4 mg, 0.0182 mmol ), Pd(PPh₃)₂Cl₂ (13 mg, 0.0182 mmol), KF (16 mg, 0.273 mmol), and triethylamine (0.7 mL) were added. Cyclopropyl (trimethylsilyl) acetylene (38 mg, 0.273 mmol) dissolved in DMF (0.5 mL) was added under N₂ atmosphere, and stirred overnight at room temperature. The reaction solution was quenched with saturated ammonium chloride solution, diluted with water, extracted with dichloromethane, the liquid was separated, and the organic phases were combined, washed with saturated brine and dried over anhydrous sodium sulfate. The obtained was concentrated and Flash separated (methanol/dichloromethane = 3%) to obtain (R)-4-((6-amino-5-(cyclopropylethynyl)-3-(S-methyl-N-(methyl-*d₃*)sulfonylimino)pyridin-2-yl)amino)-6-(cyclopropylcarboxamido)-N-(methyl-*d₃*)pyridazine-3-carboxamide (74 mg, light yellow solid), yield 83%.

¹H NMR (400 MHz, DMSO-d6) δ 11.68 (s, 1H), 11.36 (s, 1H), 9.56 (s, 1H), 9.02 (s, 1H), 7.70 (s, 1H), 6.59 (s, 2H), 3.10 (s, 3H), 2.12 (s, 1H), 1.56 (s, 1H), 0.96 -0.74 (m, 8H).

MS m/z [M+H]⁺: 489

### Example 47

### Synthesis of (R)-4-((6-amino-5-chloro-3-(S-methyl-N-(methyl-d₃)sulfonylimino)pyridin-2-yl)amino)-6-(cyclopropylcarboxamide)-N-(methyl-d₃)pyridazine-3-carboxamide hydrochloride

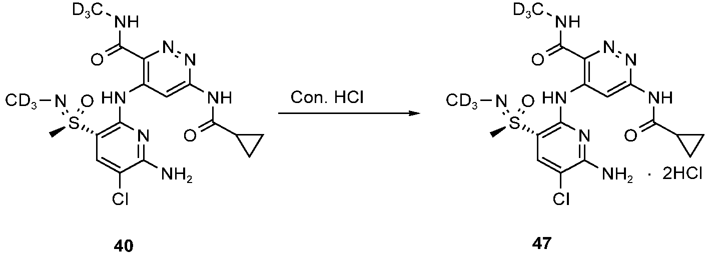

(R)-4-((6-amino-5-chloro-3-(S-methyl-N-(methyl-*d₃*)sulfonylimino)pyridin-2-yl)amino)-6-(cyclopropylcarboxamide)-N-(methyl-*d₃*)pyridazine-3-carboxamide (200 mg, 0.44 mmol) was added into DMF (2 mL) and stirred at room temperature, concentrated HCl (108 mg, 1.09 mmol) was added. The obtained was placed in 90 °C oil bath and stirred for 5 min, became turbid, acetonitrile (10 mL) was added, reflux for 5 min, the suspension was stirred for 1.5 h at room temperature, suction filtered, washed with acetonitrile (0.5 mL * 2), the filter cake was dried at 50 °C under reduced pressure for 1h to obtain the dihydrochloride, light yellow solid: 230 mg, yield: 99.2%.

¹H NMR (400 MHz, DMSO-*d⁶*) δ 11.88 (s, 1H), 11.82 (s, 1H), 9.44 (d, *J* = 2.2 Hz, 1H), 9.24 (s, 1H), 7.91 (d, *J* = 2.6 Hz, 1H), 7.77 (s, 2H), 3.91 (s, 3H), 2.21-2.11 (m, 1H), 0.94-0.86 (m, 4H).

MS m/z [M+H]⁺: 459

### Example 48

### Synthesis of (R)-4-((6-amino-5-chloro-3-(S-methyl-N-(methyl-d₃)sulfonylimino)pyridin-2-yl)amino)-6-(cyclopropylcarboxamide)-N-(methyl-d₃)pyridazine-3-carboxamide sulfate

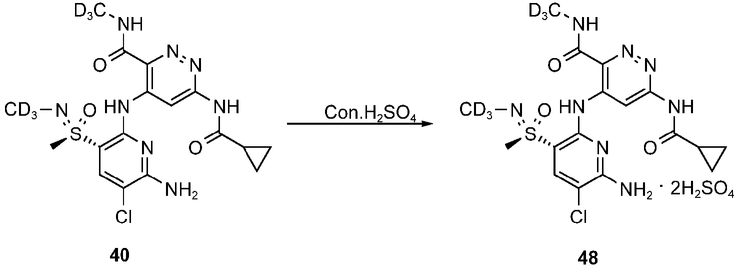

(R)-4-((6-amino-5-chloro-3-(S-methyl-N-(methyl-*d₃*)sulfonylimino)pyridin-2-yl)amino)-6-(cyclopropylcarboxamido)-N-(methyl-*d3*)pyridazine-3-carboxamide (200 mg, 0.44 mmol) was added to DMF (1 mL), stirred at room temperature, became a suspension, concentrated H₂SO₄ (109 mg, 1.09 mmol) was added, and dissolved with acetonitrile (1 mL) with stirring at room temperature. The obtained was placed in 90 °C oil bath and stirred for 5 min, acetonitrile (6 mL) was added, reflux for 5 min, solid was precipitated, stirred for 1.5 h at room temperature, suction filtered, washed with acetonitrile (0.5 mL * 2), the filter cake was dried at 50 °C for 1 h under reduced pressure to obtain disulfate, white solid: 268 mg, yield: 93.8%.

¹H NMR (400 MHz, DMSO-*d⁶*) δ 11.82 (s, 1H), 11.46 (s, 1H), 9.34 (s, 1H), 9.30 (s, 1H), 8.92 (s, 4H), 7.90 (s, 1H), 7.64 (s, 2H), 3.92 (s, 3H), 2.15-2.08 (m, 1H), 0.90-0.82 (m, 4H).

MS m/z [M+H]⁺: 459

### Example 49

### Synthesis of (R)-4-((6-amino-5-chloro-3-(S-methyl-N-(methyl-d₃)sulfonylimino)pyridin-2-yl)amino)-6-(cyclopropylcarboxamido)-N-(methyl-d₃)pyridazine-3-carboxamide methanesulfonate

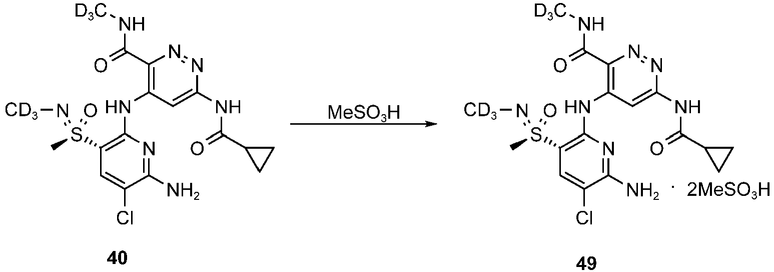

(R)-4-((6-amino-5-chloro-3-(S-methyl-N-(methyl-*d₃*)sulfonylimino)pyridin-2-yl)amino)-6-(cyclopropylcarboxamido)-N-(methyl-*d3*)pyridazine-3-carboxamide (200 mg, 0.44 mmol) was added to DMF (0.5 mL), stirred at room temperature, became a suspension. Methanesulfonic acid (105 mg, 1.09 mmol) was added, dissolved with acetonitrile (1 mL) at room temperature with stirring, placed in 90 °C oil bath and stirred for 5 min, acetonitrile (8 mL) was added, reflux for 5 min, the solid was precipitated, stirred for 1.5 h at room temperature, suction filtered, washed with acetonitrile (0.5 mL * 2), the filter cake was dried at 50°C for 1 h under reduced pressure to obtain dimethylsulfonate, off-white solid: 268 mg, yield: 94.4%.

¹H NMR (400 MHz, DMSO-*d⁶*) δ 11.82 (s, 1H), 11.46 (s, 1H), 9.34 (s, 1H), 9.30 (s, 1H), 7.90 (s, 1H), 7.63 (s, 2H), 3.92 (s, 3H), 2.35 (s, 6H), 2.17-2.09 (m,, 1H), 0.90-0.82 (m, 4H).

MS m/z [M+H]⁺: 459

### Biological Evaluation

### Test Example 1: TYK2 kinase activity inhibition assay

### 1.1 Principle and purpose of the experiment

The TYK2 kinase gene was introduced into wild-type murine-derived B lymphocytes (BaF3) to construct the Ba/F3-FL -TYK2-E957D stable cell line. The growth and proliferation of Ba/F3 -FL -TYK2-E957D were independent of IL-3, and dependent on exogenously transferred TYK2 kinase. If the compound selectively inhibited the activity of TYK2 kinase, it would inhibit the proliferation of this cell line.

In this experiment, the inhibitory effect of the compounds on the proliferation of Ba/F3-FL-TYK2-E957D cells were tested, apoptosis was detected, and the IC50 value was calculated. The inhibitory activity of the compounds against TYK2 kinase was evaluated based on the IC50 value.

### 1.2 Experimental Methods

### 1.2. 1 Preparation of stock solution

The tested compounds, powder and stored at room temperature, were prepared into 10 mM stock solution with DMSO and stored at -20°C away from light for use.

### 1.2. 2 Experimental steps

### Preparation of compounds

Compounds were prepared into dilute solutions which was 1000 times of the final concentration. The compound was prepared into 20-times of the final concentration by diluting the compound with culture medium. 2ul of 1000-times compound was added to per well to 98ul of culture medium.

### Plating

Cells (provided by Sundia Pharmaceutical Technology (Shanghai) Co., Ltd.) were resuspended and counted using an automated cell counter. The cell suspension was diluted to the desired density based on BaF3 seeding density of 2000 cells per well. Each well was plated with 95ul of cells and incubated at 37°C for stable equilibrium. 5ul of 20-times compound was added to each well, with the wells to which the same volume of DMSO was added as a control. The cells were incubated for 72h at 37°C with 5% CO2.

### Detection

The cell plate was equilibrated to room temperature. 40ul CellTiter-Glo^{®} Reagent was added to each well, shaken for 2 minutes and allowed to stand for 10 minutes. Detected with SpectraMax Paradigm.

### Data Analysis

(1) IC50 was calculated using GraphPad Prism 5.
(2) $% Inh = \left(Max signal - Compound signal\right) / \left(Max signal - Min signal\right) \times 100 .$
(3) Max signal was the positive control well with only the same volume of DMSO as the compound.
(4) Min signal was the negative control well with only the culture medium.

The results are shown in Table 1 below.

**Table 1 Inhibitory activity of representative compounds of the present invention against TYK2 kinase**

| Example No. | Ba /F3 -FL -TYK2-E957D cell IC₅₀(nM) |
|---|---|
| BMS-986165 | 2.3 |
| 2 | 2.4 |
| 13 | 3.9 |
| 14 | 3.7 |
| 16 | 3.5 |
| 23 | 2.0 |
| 24 | 1.3 |
| 28 | 0.60 |
| 29 | 1.1 |
| 31 | 1.1 |
| 32 | 0.62 |
| 33 | 1.2 |
| 40 | 0.4 |
| 41 | 0.4 |
| 42 | 0.4 |
| 43 | 1.1 |
| 44 | 0.3 |
| 45 | 0.1 |
| 46 | 0.1 |

Wherein,
the structural formula of compound BMS-986165 is

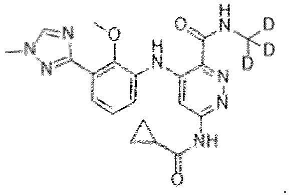

The test results show that the compounds provided in the present invention have excellent TYK2 inhibitory activity, and most of the compounds have TYK2 inhibitory activity superior to that of the positive control BMS-986165, with IC50 values of pM level. In particular, Example 28, Example 32, Example 40, Example 41, Example 42 and Example 44 show a 4- to 7-fold increase in TYK2 inhibitory activity over BMS-986165, and Example 45 and Example 46 show a more than 20-fold increase in TYK2 inhibitory activity over BMS-986165.

### Test Example 2: JAK family kinase selectivity assay

### 2.1 Principle and purpose of the experiment

There are four kinases in the JAK family, respectively TYK2, JAK1, JAK2 and JAK3. JAK family kinase genes were introduced into the wild-type murine B lymphocytes (BaF3), and four BaF3 engineered cell lines were constructed respectively (Ba /F3 -FL -TYK2-E957D, Ba/F3-TEL-JAK1, Ba/F3-TEL-JAK2, Ba/F3-TEL-JAK3). The growth and proliferation of these engineered cell lines were not dependent on IL-3, but on exogenously transferred JAK family kinases. If a compound selectively inhibited the activity of a JAK kinase, it would inhibit the proliferation of the corresponding cell line.

In this experiment, the inhibitory effects of the compounds on the proliferation of four cell types, Ba/F3 -FL -TYK2-E957D, Ba/F3-TEL-JAK1, Ba/F3-TEL-JAK2, and Ba/F3-TEL-JAK3 were tested, and apoptosis was detected, and the IC50 values were calculated. The inhibitory activities of the compounds against TYK2, JAK1, JAK2, and JAK3 kinases were evaluated based on the IC50 value, thus assessing the JAK family kinase selectivity of the compounds.

### 2.2 Experimental Methods

### 2.2. 1 Preparation of stock solution

The tested compounds, powder and stored at room temperature, were prepared into 10 mM stock solution with DMSO and stored at -20°C away from light for use.

### 2.2. 2 Experimental steps

### Preparation of compounds

Compounds were formulated into dilute solutions which was 1000 times of the final concentration. The compound was prepared into 20-times of final concentration by diluting the compound with culture medium. 2ul of 1000-times compound was added per well to 98ul of culture medium.

### Plating

Cells (provided by Sundia Pharmaceutical Technology (Shanghai) Co., Ltd.) were resuspended and counted using an automated cell counter. The cell suspension was diluted to the desired density based on BaF3 seeding density of 2000 cells per well. Each well was plated with 95ul of cells and incubated at 37°C for stable equilibrium. 5ul of 20-times compound was added to each well, with the wells to which the same volume of DMSO was added as a control. The cells were incubated at 37°C with 5% CO2 for 72h.

### Detection

The cell plate was equilibrated to room temperature. 40ul Cell Titer-Glo^{®} Reagent was added to each well, shaken for 2 minutes and allowed to stand for 10 minutes. Detected with SpectraMax Paradigm.

### Data Analysis

(1) IC50 was calculated using GraphPad Prism 5.
(2) $%Inh = \left(Max signal - Compound signal\right) / \left(Max signal - Min signal\right) \times 100 .$
(3) Max signal was the positive control well with only the same volume of DMSO as the compound.
(4) Min signal was the negative control well with only the culture medium.

The results are shown in Table 2 below.

**Table 2 Selectivity of representative compounds of the present invention against TYK2 kinase**

| Compound No. | Ba/F3 Cell IC50 (nM) | | | |
|---|---|---|---|---|
| | TYK2 | JAK1 | JAK2 | JAK3 |
| BMS-986165 | 1.37 | >1000 | >1000 | >1000 |
| 28 | 0.6 | >1000 | >1000 | >1000 |
| 29 | 1.1 | >1000 | >1000 | >1000 |

The test results show that the compounds in the present invention have excellent TYK2 inhibitory activity, as well as no inhibitory activity against JAK1, JAK2, and JAK3 kinases, and the JAK family kinases selectivity is very high.

The basic research and clinical results of existing JAK kinase inhibitors show that non-selective kinase inhibitors bring serious side effects, including thrombosis, anemia and serious infections, thus, poor kinase subtype selectivity is a difficulty to be overcome in the development of next-generation JAK inhibitors. In contrast, the compounds of the present invention have excellent kinase selectivity and exhibit high safety of drug use.

### Test Example 3: IFN-α-induced STAT phosphorylation in human whole blood

### 3.1 Principle and purpose of the experiment

The cytokine receptor attached to TYK2 mediates the signaling of type 1 interferon (IFN-α), which has been identified as the most important inflammatory factor of the autoimmune system. Human whole blood is induced with IFN-α, and TYK2 from lymphocytes in whole blood triggered STAT phosphorylation, which mediated subsequent inflammatory signaling. Therefore, TYK2 inhibitors have an inhibitory effect on IFN-α signaling, and the stronger the TYK2 inhibitory activity, the lower the level of STAT phosphorylation, and the stronger the corresponding anti-inflammatory effect.

In this experiment, the ability of the compounds to inhibit IFN-α-induced STAT phosphorylation in human whole blood were tested, and the IC50 values were calculated. The anti-inflammatory activity of the compounds was evaluated at the tissue level and evaluation of the inhibitory activity of compounds on the IFN-α inflammatory pathway based on the IC50 value.

### 3.2 Experimental process

### 3.2. 1 Preparation of stock solution

The tested compound, powder and stored at room temperature, prepared into 10 mM stock solution with DMSO and stored at -80°C in the refrigerator.

Positive control compound, BMS-986165, powder and stored at room temperature, prepared into 10 mM stock solution with DMSO and stored at -80°C in a refrigerator.

### 3.2. 2 Preparation of solution

1X Fix/Lysis solution preparation: 5X Fix/Lysis and ddH2O were diluted according to 1:4 and preheated in 37°C incubator, waiting for use.

Flow cytometric staining buffer preparation: 1 g of bovine serum albumin (BSA) and 1 mL of EDTA (0.5 M) solution were added to 500 mL of phosphate buffer solution (PBS) and stored at 4°C in the refrigerator.

Compound dilution solution: 0.5 g of bovine serum albumin (BSA) was added to 500 mL of phosphate buffer solution (PBS), and stored at 4°C in the refrigerator.

### 3.2. 3 Experimental steps

### Preparation of compounds and cytokines

10 mM of the test compound and the positive compound BMS-986165 were diluted with DMSO into 5 mM of working solution , which was further diluted with DMSO in a 3-fold gradient to eight concentrations. The eight concentrations gradient of the test compound and each concentration point of BMS-986165 were diluted 25-fold with compound diluent and set aside.

50 µg/mL IFN-alpha was diluted to 400 ng/mL with Compound Diluent and set aside.

### Processing of human whole blood

67.5 µL of human whole blood was added to a 96-well plate using a 300 µL electric volley gun. 3.5 µL of prepared test compound was added, mixed well and placed in the cell culture incubator. After 60 min incubation, 400 ng/mL IFN-α (Biolegend 592702, final concentration 20 ng/ml) was added. The obtained was mixed well and placed in the cell culture incubator to continue incubation for 15 min.

After cytokine stimulation, 5 µl of PE-CD3 (BD 555333) antibody was added to each well, mixed and incubation was continued at 4 °C in the refrigerator. At the end, whole blood was transferred to a 96-well deep-well plate using a volley gun, prewarmed 1X Fix/Lysis solution was added, and cells were lysed and fixed for 12 minutes at 37°C in an incubator. After being washed, 400 µL of Perm III solution was added, vortexed and incubated on ice for 30 minutes. Cells were then stained by 100 µL of APC-pSTAT5 (1:100) antibody for 50 min at room temperature. This was followed by flow-through instrumentation. The expression of pSTAT5 was quantified by median fluorescence intensity after gating the CD3-positive signaling cluster.

The results are shown in Table 3 below.

**Table 3 Inhibitory activity of representative compounds of the present invention on IFN-α inflammatory pathway**

| Example No. | IC₅₀(nM) |
|---|---|
| BMS-986165(Deucravacitinib) | 23.2 |
| 7 | 22.1 |
| 15 | 18.0 |
| 22 | 13.0 |
| 23 | 8.1 |
| 24 | 7.5 |
| 26 | 9.4 |
| 28 | 4.0 |
| 29 | 3.7 |
| 32 | 16.1 |

The test results show that the compounds provided in the present invention have high inhibitory activity against IFN-α-induced STAT phosphorylation in human whole blood, indicating that the compounds of the present invention strongly inhibit the IFN-α inflammatory pathway at the tissue level. Compared to the control BMS-986165, the present invention exhibits better IFN-α inflammatory pathway inhibitory activity, and, in particular, Example 28 and Example 29 have about 6-fold higher anti-inflammatory activity.

### Test Example 4: hERG potassium channel inhibition assay with compounds of the invention

### 4.1 Purpose of the experiment

The blocking effect of the compounds in the present invention on hERG potassium currents on stable cell lines transfected with hERG potassium channels was tested by applying a fully automated membrane clamp.

### 4.2, Experimental method

### 4.2.1 Cell preparation

CHO-hERG cells were cultured in 175 cm² culture flasks, and when the cell density grew to 60-80%, the culture medium was removed and washed with 7 ml of PBS (Phosphate Buffered Saline), and then 3 ml of Detachin was added for digestion.

When digestion was completed, 7 ml of culture medium was added to neutralize the cells, then centrifuged, the supernatant was removed, and 5 ml of culture medium was added to resuspend the cells to ensure that the cell density is 2-5×10⁶/ml.

### 4.2.2 Solution preparation

| Reagent | Extracellular fluid(mM) | Intracellular fluid(mM) |
|---|---|---|
| CaCl₂ | 2 | 5.374 |
| MgCl₂ | 1 | 1.75 |
| KCl | 4 | 120 |
| NaCl | 145 | - |
| Glucose | 10 | - |
| HEPES | 10 | 10 |
| EGTA | - | 5 |
| Na₂ATP | - | 4 |
| pH | 7.4 | 7.25 |

### 4.2.3 Electrophysiological recording process

The single-cell high-impedance sealing and whole-cell pattern formation processes were all automated with the Qpatch instrument. After obtaining the recording pattern, the cells were clamped at -80 mV, and a -50 mV preconditioning voltage was given for 50 ms prior to giving a +40 mV depolarizing stimulus for 5 s. The cell was then repolarized to -50 mV to be maintained for 5 s and then returned to -80 mV. This voltage stimulus was applied every 15 s. After recording for 2 min, extracellular fluid were given, recording for 5 min, and then the drug administration process was started, with compound concentrations starting from the lowest tested concentration, and each tested concentration was given for 2.5 min, and after giving all the concentrations consecutively, the positive control compound 3 µM Cisapride was given. At least 3 cells were tested for each concentration (n ≥ 3).

### 4.2.4 Compound preparation

20 mM compound stock solution was diluted with extracellular fluid, 5 µL of 20 mM compound stock solution was added to 2495 µL of extracellular fluid, 500-fold diluted to 40 µM, and then 3-fold serial dilution was sequentially carried out in extracellular fluid containing 0.2% DMSO to obtain the final concentration to be tested.

The highest tested concentration was 40 µM, and there were six concentrations of 40, 13.33, 4.44, 1.48, 0.49, and 0.16 µM, respectively.

The DMSO content in the final test concentration did not exceed 0.2%, and this concentration of DMSO had no effect on hERG potassium channels.

### 4.3 Data analysis

The experimental data was analyzed by XLFit software.

### 4.4 Experimental results

The results are shown in Table 4 below.

**Table 4 Inhibitory activity of representative compounds of the present invention on hERG potassium channel**

| Example No. | IC₅₀(µM) |
|---|---|
| 28 | >30 |
| 29 | >30 |

The test results show that the compounds of the present invention have no hERG potassium channel inhibitory activity and low risk of cardiotoxicity.

### Test Example 5: In vivo pharmacokinetics assay of the compounds of the present invention

### 5.1 In vivo pharmacokinetic experiment in rats

Experimental method: Healthy male SD rats were randomly grouped into 3 rats in each group and given the test compound orally. The rats were fasted for 12 h before the gavage experiment, and drank water freely, and were fed uniformly for 4 h after the administration of the compounds. Before (0 h) and 0.25, 0.5, 1.0, 2.0, 4.0, 6.0, 8.0 and 24 h after the administration of the test compounds, 0.2 mL of blood was collected from the jugular vein at the above set time points and placed in EDTA-K2 tubes, and the whole blood was collected and stored temporarily in an ice-water bath, and then centrifuged at 11,000 rpm for 5 min within 30 min, the plasma was separated, and frozen at -70° C in a refrigerator for testing.

### Test Example 5.1 Results: see Table 5.1 below.

**Table 5.1 Metabolic parameters of representative compounds of the invention in rats**

| Example No. | Dose | T_{1/2} (h) | Cmax (ng/mL) | AUC (ngh/mL) | MRT (h) | F (%) |
|---|---|---|---|---|---|---|
| BMS-986165 | 5mg/kg | 3.68 | 229 | 2804 | 6.43 | 28.5 |
| 28 | 5mg/kg | 2.48 | 651 | 2644 | 3.54 | 103.8 |
| 40 | 20mg/kg | 2.65 | 2953 | 22029 | 4.7 | 109 |

### 5.2 In vivo pharmacokinetic experiments in Beagle dogs

Experimental Methods: Healthy male Beagle dogs were randomly grouped into 3 rats in each group, and were given the test compounds orally. Before the gavage experiment, the dogs were fasted for 12 h, drank water freely, and were uniformly fed for 4 h after the administration of the drug. 0.083, 0.25, 0.5, 1.0, 2.0, 4.0, 6.0, 8.0, 10 and 24 h after administration; about 600 µL of blood was collected from the forelimb or hindlimb at the above set time points and placed in EDTA-K2 tubes, and the whole blood was collected and stored temporarily in an ice-water bath, and then centrifuged at least 3500 rpm for 10 min within 30 min, and the plasma was separated and frozen in a -70°C refrigerator for testing.

### Test Example 5.2 Results: see Table 5.2 below.

**Table 5.2 dogs metabolic parameters of representative compounds of the inventions**

| Example No. | Dose | T_{1/2} (h) | Cmax (ng/mL) | AUC (ngh/mL) | MRT (h) | F (%) |
|---|---|---|---|---|---|---|
| BMS-986165(literature values) | 2mg/kg | 6.21 | 222 | 1312 | | 35.5 |
| 28 | 2mg/kg | 7.26 | 485 | 6684 | 10.68 | 117 |
| 30 | 2mg/kg | 8.61 | 625 | 8454 | 11.8 | |
| 40 | 10mg/kg | 9.28 | 4853 | 79882 | 12.7 | 136.6 |

The test results show that the compounds in the present invention have good drug metabolizing properties, good drug-forming properties and their plasma exposure (AUC) is high. The oral bioavailability (F%) and plasma exposure of the compounds of the present invention in rats and dogs have significant advantages over BMS-986165, showing excellent oral absorption properties and excellent metabolic profiles.

### Test Example 6: Solubility of the compounds of the invention in water (at pH=7)

### Test Example 6 results: see Table 6 below.

**Table 6 Solubility of representative compounds of the present invention**

| Example No. | Solubility (µg/ml) |
|---|---|
| BMS-986165 | 8.2 |
| 28 | 22 |
| 38 | 47 |

The test results show that the water solubility properties of the compounds provided by the present invention are significantly better than that of BMS-986165.

Water solubility is an important indicator for evaluating the drug ability of small molecule oral drugs. Only when the drug is dissolved can it be absorbed into the body circulation through the stomach and intestines. Drugs with poor water solubility are slowly absorbed and have low bioavailability, affecting the blood drug concentration and pharmacological effects, which directly leads to a high clinical failure rate. The compounds in the present invention have higher solubility compared to BMS-986165, and thus have better oral absorption properties and drug-forming properties.

All literatures mentioned in the present invention are incorporated by reference herein, as though individually incorporated by reference. Additionally, it should be understood that after reading the above teaching, many variations and modifications may be made by the skilled in the art, and these equivalents also fall within the scope as defined by the appended claims.

## Claims

1. A compound of formula (I) or a tautomer, a mesomer, a racemate, an enantiomer, a diastereoisomer, or a mixture thereof, or a pharmaceutically acceptable salt thereof: 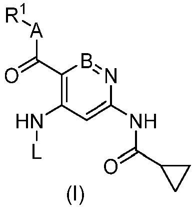 wherein,
R¹ is selected from the group consisting of H, substituted or unsubstituted C1-C6 alkyl and substituted or unsubstituted C3-C6 cycloalkyl, the substitution refers to be substituted by one or more substituents selected from the group consisting of deuterium and halogen;
A is NH or CH₂;
B is CH or N;
L is selected from the group consisting of 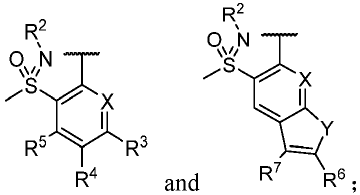
R² is selected from the group consisting of H, -CD₃, C1-C6 alkyl and C3-C6 cycloalkyl;
X is CH or N;
Y is NH, O or S;
R³, R⁴, R⁵ are each independently selected from the group consisting of H, -NH₂, -OH, C1-C6 alkoxy, C1-C6 alkyl, halogenated C1-C6 alkyl, C3-C6 cycloalkyl, - CONH₂, -CN, halogen, -O(CH₂)ₙR⁹, C2-C6 alkenyl, C2-C6 alkynyl, C3-C6 cycloalkyl-substituted C2-C6 alkynyl and -NR⁸(CH₂)ₙR⁹;
n is 1, 2, 3, 4 or 5;
R⁹ is selected from the group consisting of -OH, C1-C6 alkoxy, C4-C6 cycloalkoxy, 4-8-membered heterocyclic group containing 1, 2 or 3 heteroatoms selected from N, O or S and -CN;
R⁸ is H or C1-C6 alkyl;
R⁶, R⁷ are each independently selected from the group consisting of H, C1-C6 alkyl and halogen

2. The compound or the tautomer, the mesomer, the racemate, the enantiomer, the diastereoisomer, or the mixture thereof, or the pharmaceutically acceptable salt thereof according to claim 1, wherein R¹ is a fully deuterated C1-C6 alkyl;
A is NH;
B is CH or N

3. The compound or the tautomer, the mesomer, the racemate, the enantiomer, the diastereoisomer, or the mixture thereof, or the pharmaceutically acceptable salt thereof according to claim 1, wherein L is 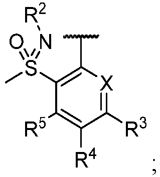
R² is selected from the group consisting of H, -CD₃, and C1-C6 alkyl;
X is N;
R³, R⁴, R⁵ are each independently selected from the group consisting of H, -NH₂, -OH, C1-C6 alkoxy, C1-C6 alkyl, C3-C6 cycloalkyl, -CONH₂, -CN, halogen, - O(CH₂)ₙR⁹, C2-C6 alkenyl, C2-C6 alkynyl, C3-C6 cycloalkyl-substituted C2-C6 alkynyl and -NR⁸(CH₂)ₙR⁹;
n is 1, 2, 3, 4 or 5;
R⁹ is selected from the group consisting of C1-C6 alkoxy, C4-C6 cycloalkoxy, 4-8-membered heterocyclic group containing 1, 2 or 3 heteroatoms selected from N, O or S and -CN;
R⁸ is H or C1-C6 alkyl.

4. The compound or the tautomer, the mesomer, the racemate, the enantiomer, the diastereoisomer, or the mixture thereof, or the pharmaceutically acceptable salt thereof according to claim 1, wherein R¹ is a fully deuterated C1-C6 alkyl;
A is NH;
B is N;
L is 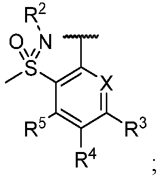
R² is selected from the group consisting of H, -CD₃ and C1-C6 alkyl;
X is N;
R³ is -NH₂;
R⁴ is selected from the group consisting of halogen, C3-C6 cycloalkyl, C2-C6 alkenyl, C2-C6 alkynyl and C3-C6 cycloalkyl-substituted C2-C6 alkynyl;
R⁵ is H.

5. The compound or the tautomer, the mesomer, the racemate, the enantiomer, the diastereoisomer, or the mixture thereof, or the pharmaceutically acceptable salt thereof according to claim 1, wherein R¹ is a fully deuterated C1-C6 alkyl;
A is NH;
B is N;
L is 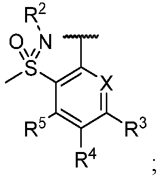
R² is -CD₃;
X is N;
R³ is -NH₂;
R⁴ is selected from the group consisting of halogen, C3-C6 cycloalkyl, C2-C6 alkenyl, C2-C6 alkynyl and C3-C6 cycloalkyl-substituted C2-C6 alkynyl;
R⁵ is H.

6. The compound or the tautomer, the mesomer, the racemate, the enantiomer, the diastereoisomer, or the mixture thereof, or the pharmaceutically acceptable salt thereof according to claim 1, wherein the compound is selected from the group consisting of:

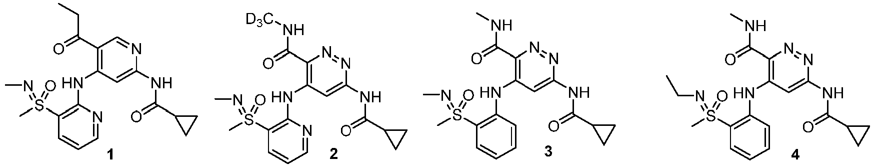

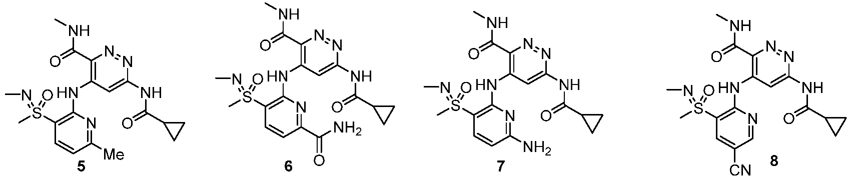

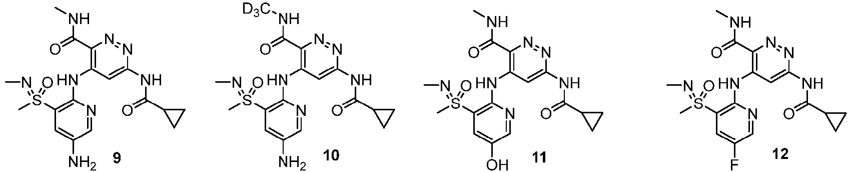

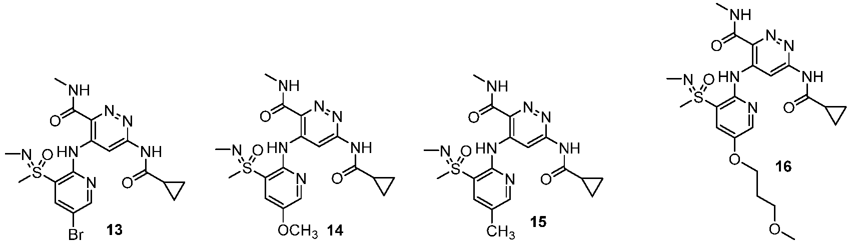

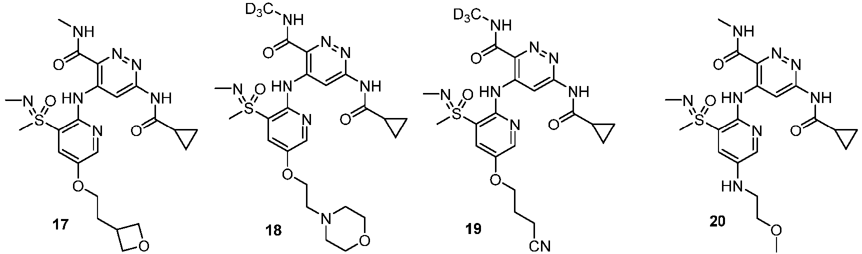

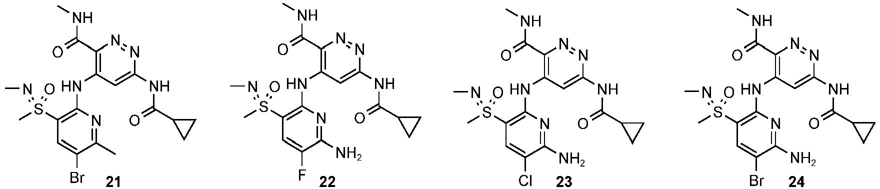

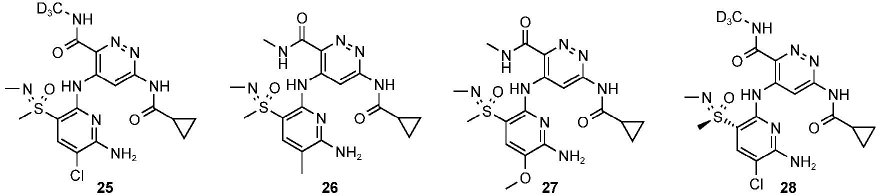

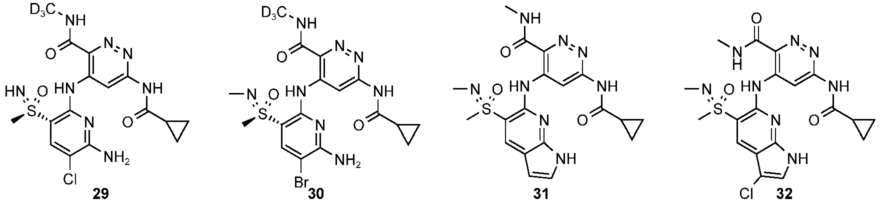

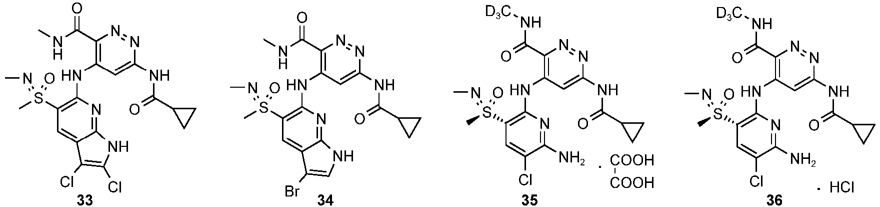

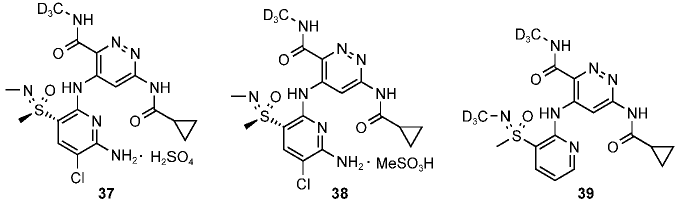

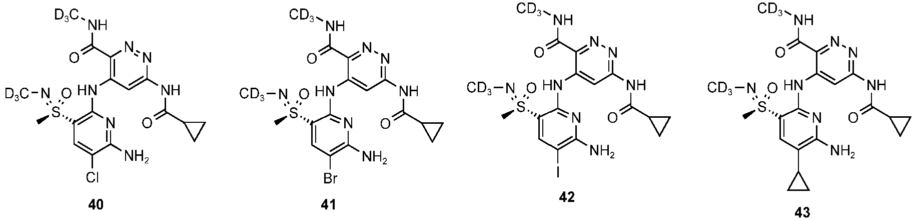

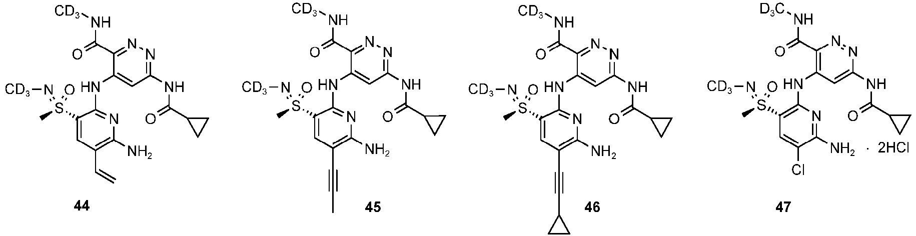

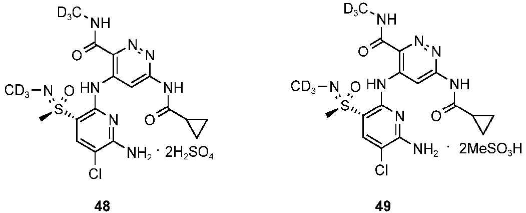

7. A preparation method for the compound or the tautomer, the mesomer, the racemate, the enantiomer, the diastereoisomer, or the mixture thereof, or the pharmaceutically acceptable salt thereof according to claim 1 comprising the following steps: 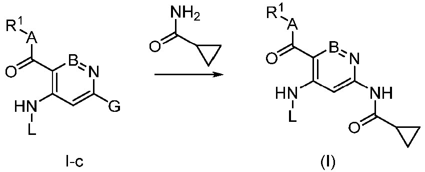
reacting formula I-c with cyclopropyl amide to obtain the compound of formula (I);
wherein,
R¹, A, B, L are as defined in claim 1;
G is halogen.

8. A pharmaceutical composition comprising a pharmaceutically acceptable carrier and one or more safe and effective amounts of the compound or the tautomer, the mesomer, the racemate, the enantiomer, the diastereoisomer, or the mixture thereof, or the pharmaceutically acceptable salt thereof of claim 1.

9. A use of the compound or the tautomer, the mesomer, the racemate, the enantiomer, the diastereoisomer, or the mixture thereof, or the pharmaceutically acceptable salt thereof of claim 1 for the preparation of a medicament, the medicament is used for the prevention and/or treatment of a disease mediated by TYK2.

10. The use according to claim 9, wherein the disease mediated by TYK2 is an inflammatory or autoimmune disease in mammals.
